(19) Europäisches Patentamt
European Patent Office
Office européen des brevets

(11) **EP 2 029 512 B1**

(12) **EUROPÄISCHE PATENTSCHRIFT**

(45) Veröffentlichungstag und Bekanntmachung des Hinweises auf die Patenterteilung:
**11.10.2017 Patentblatt 2017/41**

(21) Anmeldenummer: **07729181.3**

(22) Anmeldetag: **16.05.2007**

(51) Int Cl.:
*C07C 45/33* (2006.01)    *C07C 47/22* (2006.01)
*C07C 45/35* (2006.01)    *C07C 51/215* (2006.01)
*C07C 51/25* (2006.01)    *C07C 57/04* (2006.01)

(86) Internationale Anmeldenummer:
**PCT/EP2007/054733**

(87) Internationale Veröffentlichungsnummer:
**WO 2007/135041 (29.11.2007 Gazette 2007/48)**

(54) **VERFAHREN ZUR HERSTELLUNG VON ACROLEIN, ODER ACRYLSÄURE, ODER DEREN GEMISCH AUS PROPAN**

PROCESS FOR PREPARING ACROLEIN OR ACRYLIC ACID OR A MIXTURE THEREOF FROM PROPANE

PROCÉDÉ DE FABRICATION D'ACROLÉINE, D'ACIDE ACRYLIQUE, OU DE LEUR MÉLANGE À PARTIR DE PROPANE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **24.05.2006 US 802786 P**
**24.05.2006 DE 102006024901**

(43) Veröffentlichungstag der Anmeldung:
**04.03.2009 Patentblatt 2009/10**

(73) Patentinhaber: **BASF SE**
**67056 Ludwigshafen am Rhein (DE)**

(72) Erfinder:
• **MACHHAMMER, Otto**
**68163 Mannheim (DE)**
• **MÜLLER-ENGEL, Klaus, Joachim**
**76297 Stutensee (DE)**
• **DIETERLE, Martin**
**67063 Ludwigshafen (DE)**

(74) Vertreter: **BASF IP Association**
**BASF SE**
**G-FLP-C006**
**67056 Ludwigshafen (DE)**

(56) Entgegenhaltungen:
**DE-A1- 10 028 582**

**Beschreibung**

Vorliegende Erfindung betrifft ein Verfahren zur Herstellung von Acrolein, oder Acrylsäure, oder deren Gemisch aus Propan, bei dem man

A)

- einer ersten Reaktionszone A unter Ausbildung eines Reaktionsgases A wenigstens zwei gasförmige, Propan enthaltende Zufuhrströme zuführt, von denen wenigstens einer Frischpropan enthält;

- in der Reaktionszone A das Reaktionsgas A durch wenigstens ein Katalysatorbett führt, in welchem durch partielle heterogen katalysierte Dehydrierung des Propans molekularer Wasserstoff und Propylen gebildet werden;

- der Reaktionszone A molekularen Sauerstoff zuführt, der in der Reaktionszone A wenigstens eine Teilmenge von in Reaktionsgas A enthaltenem molekularem Wasserstoff zu Wasserdampf oxidiert, und

- der Reaktionszone A Produktgas A entnimmt, das Propylen, Propan und Wasserdampf enthält;

B) gegebenenfalls in einer ersten Trennzone I in Produktgas A enthaltenen Wasserdampf teilweise oder vollständig durch indirekte und/oder direkte Kühlung von Produktgas A unter Verbleib eines Produktgases A* kondensativ abtrennt;

C) in einer Reaktionszone B Produktgas A oder Produktgas A* unter Zufuhr von molekularem Sauerstoff zur Beschickung wenigstens eines Oxidationsreaktors mit einem Propan, Propylen und molekularen Sauerstoff enthaltenden Reaktionsgas B verwendet und das in selbigem enthaltene Propylen einer heterogen katalysierten partiellen Gasphasenoxidation zu einem Acrolein, oder Acrylsäure, oder deren Gemisch als Zielprodukt, sowie nicht umgesetztes Propan enthaltenden Produktgas B unterwirft;

D) aus der Reaktionszone B Produktgas B herausführt und in einer zweiten Trennzone II darin enthaltenes Zielprodukt unter Verbleib eines Propan enthaltenden Restgases abtrennt;

E) gegebenenfalls eine die Zusammensetzung des Restgases aufweisende Teilmenge an Restgas als einen Propan enthaltenden Zufuhrstrom in die Reaktionszone A rückführt;

F) in einer Trennzone III in nicht in die Reaktionszone A rückgeführtem Restgas, aus dem gegebenenfalls zuvor darin gegebenenfalls enthaltener Wasserdampf kondensativ und/oder gegebenenfalls darin enthaltener molekularer Wasserstoff über eine Trennmembran teilweise oder vollständig abgetrennt werden, enthaltenes Propan unter Ausbildung eines Propan enthaltenden Absorbats aus dem Restgas durch Absorption in ein organisches Lösungsmittel (Absorptionsmittel) aufnimmt; und

G) in einer Trennzone IV das Propan aus dem Absorbat abtrennt und als Propan enthaltenden Zufuhrstrom in die Reaktionszone A rückführt.

Acrylsäure ist eine bedeutende Grundchemikalie, die unter anderem als Monomeres zur Herstellung von Polymerisaten Verwendung findet, die beispielsweise in disperser Verteilung in wässrigem Medium befindlich als Bindemittel angewendet werden. Ein weiteres Anwendungsgebiet von Acrylsäurepolymerisaten bilden Superabsorber im Hygienebereich und anderen Anwendungsgebieten. Acrolein ist ein bedeutendes Zwischenprodukt, beispielsweise für die Herstellung von Glutardialdehyd, Methionin, 1,3-Propandiol, 3-Picolin, Folsäure und Acrylsäure (vgl. z. B. US-A 6,166,263 und US-A 6,187,963).

Verfahren zur Herstellung von Acrolein, oder Acrylsäure, oder deren Gemisch aus Propan, bei denen man in einer ersten Reaktionszone Propan heterogen katalysiert partiell zu Propylen dehydriert und nachfolgend das gebildete Propylen im Beisein des nicht umgesetzten Propan (d.h., ohne zuvor das Propylen vom in der Dehydrierung verbliebenen Propan zu trennen, wie es z. B. in der EP-A 1 617 931 empfohlen wird (vgl. auch WO 04/09041)) heterogen katalysiert zu Acrolein, oder zu Acrylsäure, oder zu deren Gemisch partialoxidiert, sind bekannt (vgl. z. B. DE-A 33 13 573, EP-A 117 146, US-A 3,161,670, DE-A 10 2004 032 129, EP-A 731 077, DE-A 10 2005 049 699, DE-A 10 2005 052 923, WO 01/96271, WO 03/011804, WO 03/076370, WO 01/96270, DE-A 10 2005 009 891, DE-A 10 2005 013 039, DE-A 10 2005 022 798, DE-A 10 2005 009 885, DE-A 10 2005 010 111, DE-A 102 455 85, DE-A 103 160 39, Deutsches Aktenzeichen Nr. 10 2005 056377.5, Deutsches Aktenzeichen Nr. 10 2005 049699.7, Deutsches Aktenzeichen Nr. 10 2006 015235.2, Deutsches Aktenzeichen Nr. 10 2005 061626.7 und der in diesen Schriften genannte Stand der Technik).

Gemeinsames Merkmal der dort beschriebenen Verfahren ist, dass man nach der Abtrennung des Zielproduktes Acrolein, oder Acrylsäure, oder Acrolein und Acrylsäure aus dem Produktgasgemisch der Propylenpartialoxidation aus dem dabei verbleibenden, Propan enthaltenden Restgas wenigstens eine Teilmenge des im Restgas enthaltenen Propan als Kreisgas in die heterogen katalysierte partielle Dehydrierung von Propan zu Propylen rückführt.

Im übrigen lassen sich die vorstehenden Verfahren des Standes der Technik im wesentlichen in zwei Gruppen aufteilen. Die Verfahren der einen der beiden Gruppen sind dadurch gekennzeichnet, dass man aus dem Produktgasgemisch der heterogen katalysierten partiellen Dehydrierung die von Propan und Propylen verschiedenen Bestandteile absorptiv abtrennt, bevor das Propylen im Beisein des verbliebenen Propan der Partialoxidation zum gewünschten Zielprodukt unterworfen wird (Deutsches Aktenzeichen Nr. 10 2005 013039.9). Nachteilig an dieser Verfahrensweise ist, dass sie in der Regel wenigstens dreier Zonen bedarf, in denen Gas verdichtet werden muss. Zum einen bedarf es üblicherweise einer Verdichtung des Produktgases der heterogen katalysierten partiellen Dehydrierung des Propans, bevor selbiges der absorptiven Abtrennung des darin enthaltenen Propylen und Propan unterworfen wird, da normalerweise nur eine absorptive Abtrennung unter Druck effizient ausführbar ist. Zusätzlich bedarf es in der Regel einer Verdichtung der für die heterogen katalysierte Partialoxidation eingesetzten Sauerstoffquelle und darüber hinaus bedarf es normalerweise einer Verdichtung des in die heterogen katalysierte partielle Dehydrierung rückgeführten, in der heterogen katalysierten partiellen Oxidation des Propylens verbliebenen, Propans. Die Verfahren der anderen der beiden Gruppen sind dadurch gekennzeichnet, dass sie empfehlen, das Produktgasgemisch der heterogen katalysierten partiellen Dehydrierung als solches zur Beschickung der heterogen katalysierten Propylenpartialoxidation einzusetzen (vgl. z. B. DE-A 103 160 39). Grundlage dieser Verfahrensweise ist u.a. die Annahme, dass der bei der heterogen katalysierten partiellen Dehydrierung des Propans gebildete molekulare Wasserstoff sich in der nachfolgenden heterogen katalysierten partiellen Oxidation des Propylens nicht nachteilig auswirkt (im Unterschied zur exotherm verlaufenden heterogen katalysierten Oxidehydrierung, die durch anwesenden Sauerstoff erzwungen und bei der intermediär kein freier Wasserstoff gebildet wird (der dem zu dehydrierenden Kohlenwasserstoff entrissene Wasserstoff wird unmittelbar als Wasser ($H_2O$) entrissen) bzw. nachweisbar ist, soll in dieser Anmeldung unter der (z. B. in der Reaktionszone A durchzuführenden) heterogen katalysierten Dehydrierung eine ("konventionelle") Dehydrierung verstanden werden, deren Wärmetönung im Unterschied zur Oxidehydrierung endotherm ist (als Folgeschritt ist in der Reaktionszone A eine exotherme Wasserstoffverbrennung einbezogen) und bei der wenigstens intermediär freier molekularer Wasserstoff gebildet wird; dazu bedarf es in der Regel anderer Reaktionsbedingungen und anderer Katalysatoren als zur Oxidehydrierung; d.h., die heterogen katalysierte partielle Dehydrierung des Propans in der Reaktionszone A verläuft beim erfindungsgemäßen Verfahren in notwendiger Weise unter $H_2$-Entwicklung). Eigene Untersuchungen haben jedoch ergeben, dass sich ein Beisein erhöhter Mengen an molekularem Wasserstoff im Reaktionsgasgemisch für die heterogen katalysierte Partialoxidation des Propylens zwar nicht nennenswert auf das Nebenproduktspektrum der Partialoxidation auswirkt, aber (vermutlich auf Grund des ausgeprägten Reduktionspotentials des molekularen Wasserstoffs) doch spürbar die Standzeit der für die heterogen katalysierte partielle Gasphasenoxidation zu verwendenden Katalysatoren beeinträchtigt.

Zusätzlich belastet molekularer Wasserstoff das Reaktionsgas B mit einem knallgasfähigen und ein spezielles Diffusionsverhalten (bestimmte Konstruktionsmaterialien sind für molekularen Wasserstoff durchlässig) aufweisenden Bestandteil. Untrennbar verknüpft mit der vorstehend beschriebenen Problematik ist die für eine erhöhte Katalysatorstandzeit in der Partialoxidation der Reaktionszone B vorteilhafte Anwendung eines Überschusses an molekularem Sauerstoff relativ zur Zielreaktionsstöchiometrie, was in natürlicher Weise erhöhte Explosionsrisiken im Beisein von molekularem Wasserstoff in der Reaktionszone B nach sich zieht (ist Acrylsäure das Zielprodukt und wird dem Reaktionsgas B die insgesamt benötigte Menge an molekularem Sauerstoff vorab zugegeben, ist normalerweise ein molares Verhältnis von im Reaktionsgas B enthaltenem molekularem Sauerstoff zu darin enthaltenem Propylen von wenigstens > 1,5 erforderlich (dabei ist eine geringfügige Propylenvollverbrennung bereits berücksichtigt)).

Angesichts des beschriebenen Standes der Technik bestand die Aufgabe der vorliegenden Erfindung darin, ein der Präambel der vorliegenden Schrift gemäßes, verbessertes Verfahren zur Verfügung zu stellen, das die beschriebenen Nachteile allenfalls noch in verminderter Form aufweist.

Demgemäß wurde ein Verfahren zur Herstellung von Acrolein, oder Acrylsäure, oder deren Gemisch aus Propan, bei dem man

A)

- einer ersten Reaktionszone A unter Ausbildung eines Reaktionsgases A wenigstens zwei gasförmige, Propan enthaltende Zufuhrströme zuführt, von denen wenigstens einer Frischpropan enthält;

- in der Reaktionszone A das Reaktionsgas A durch wenigstens ein Katalysatorfestbett führt, in welchem durch partielle heterogen katalysierte Dehydrierung des Propans molekularer Wasserstoff und Propylen gebildet werden;

- der Reaktionszone A molekularen Sauerstoff zuführt, der in der Reaktionszone A wenigstens eine Teilmenge von in Reaktionsgas A enthaltenem molekularem Wasserstoff zu Wasserdampf oxidiert, und

- der Reaktionszone A Produktgas A entnimmt, das Propylen, Propan und Wasserdampf enthält;

B) gegebenenfalls in einer ersten Trennzone I in Produktgas A enthaltenen Wasserdampf teilweise oder vollständig durch indirekte und/oder direkte Kühlung von Produktgas A unter Verbleib eines Produktgases A* kondensativ abtrennt;

C) in einer Reaktionszone B Produktgas A oder Produktgas A* unter Zufuhr von molekularem Sauerstoff zur Beschickung wenigstens eines Oxidationsreaktors mit einem Propan, Propylen und molekularen Sauerstoff enthaltenden Reaktionsgas B verwendet und das in selbigem enthaltene Propylen einer heterogen katalysierten partiellen Gasphasenoxidation zu einem Acrolein, oder Acrylsäure, oder deren Gemisch als Zielprodukt, sowie nicht umgesetztes Propan enthaltenden Produktgas B unterwirft;

D) aus der Reaktionszone B Produktgas B herausführt und in einer zweiten Trennzone II darin enthaltenes Zielprodukt unter Verbleib eines Propan enthaltenden Restgases abtrennt;

E) gegebenenfalls eine die Zusammensetzung des Restgases aufweisende Teilmenge an Restgas als einen Propan enthaltenden Zufuhrstrom in die Reaktionszone A rückführt (Restgaskreisgas);

F) in einer Trennzone III in nicht in die Reaktionszone A rückgeführtem Restgas, aus dem gegebenenfalls zuvor darin gegebenenfalls enthaltener Wasserdampf kondensativ und/oder gegebenenfalls darin enthaltener molekularer Wasserstoff über eine Trennmembran teilweise oder vollständig abgetrennt werden, enthaltenes Propan unter Ausbildung eines Propan enthaltenden Absorbats aus dem Restgas durch Absorption in ein organisches Lösungsmittel (Absorptionsmittel) aufnimmt; und

G) in einer Trennzone IV das Propan aus dem Absorbat abtrennt und als Propan enthaltenden Zufuhrstrom (Propankreisgas, das vorzugsweise Wasserdampf enthält) in die Reaktionszone A rückführt;

gefunden, das dadurch gekennzeichnet ist, dass in der Reaktionszone A wenigstens soviel molekularer Wasserstoff zu Wasserdampf oxidiert wird, dass die in der Reaktionszone A zu Wasserdampf oxidierte Wasserstoffmenge wenigstens 20 mol-% der in der Reaktionszone A gebildeten Menge an molekularem Wasserstoff beträgt.

Erfindungsgemäß vorteilhaft wird man wenigstens einen Teil der durch das Oxidieren (Verbrennen) von molekularem Wasserstoff in der Reaktionszone A erzeugten Wärmeenergie auch dazu verwenden, durch indirekten Wärmeaustausch mit Reaktionsgas A und/oder Produktgas A als Wärmeträger, (die) Bestandteile des Reaktionsgas A-Beschickungsgasgemischs (der Reaktionszone A zugeführte gasförmige Zufuhrströme) zu erwärmen.

Erfindungsgemäß vorteilhaft beträgt die in der Reaktionszone A zu Wasserdampf oxidierte Wasserstoffmenge wenigstens 25 mol-%, besser wenigstens 30 mol-%, vorzugsweise wenigstens 35 mol-%, besonders bevorzugt wenigstens 40 mol-%, noch besser wenigstens 45 mol-% und ganz besonders bevorzugt wenigstens 50 mol-%, jeweils bezogen auf die in der Reaktionszone A gebildete Menge an molekularem Wasserstoff.

D.h., erfindungsgemäße Verfahren sind auch solche, bei denen in der Reaktionszone A, bezogen auf die in der Reaktionszone A gebildete Menge an molekularem Wasserstoff, bis zu 55 mol-%, oder bis zu 60 mol-%, oder bis zu 65 mol-%, oder bis zu 70 mol-%, oder bis zu 75 mol-%, oder bis zu 80 mol-%, oder bis zu 85 mol-%, oder bis zu 90 mol-%, oder bis zu 95 mol-%, oder bis zu 100 mol-% an molekularem Wasserstoff zu Wasserdampf oxidiert werden.

Berücksichtigt man zusätzlich zur Auswirkung von molekularem Wasserstoff als Bestandteil von Reaktionsgas B auf die Katalysatorstandzeit in der Partialoxidation, dass die Verbrennung (Oxidation) von molekularem Wasserstoff zu Wasser etwa das zweifache derjenigen Wärmemenge liefert, die zu seiner Bildung im Rahmen der Dehydrierung von Propan verbraucht wird, ist es wärmebilanztechnisch vorteilhaft, wenn die in der Reaktionszone A verbrannte Menge an molekularem Wasserstoff beim erfindungsgemäßen Verfahren, bezogen auf die in der Reaktionszone A gebildete Menge an molekularem Wasserstoff, 30 bis 70 mol-%, besser 40 bis 60 mol-%, beträgt.

Ein begrenzter Gehalt an molekularem Wasserstoff im Reaktionsgas B (d. h., ein Reaktionsgas B, das noch molekularen Wasserstoff enthält) weist auch insofern eine gewisse Vorteilhaftigkeit auf, als molekularer Wasserstoff den Vorteil der höchsten thermischen Leitfähigkeit innerhalb der Gase aufweist. Gemäße Walter J. Moore, Physikalische Chemie, WDEG Verlag, Berlin (1973), Seite 171, ist diese bei Normalbedingungen z. B. mehr als zehn mal so groß, wie die thermische Leitfähigkeit von Kohlendioxid und etwa acht mal so groß wie diejenige von molekularem Stickstoff bzw.

von molekularem Sauerstoff.

Es ist diese erhöhte thermische Leitfähigkeit, die gemäß Tabelle I der DE-A 33 13 573 dafür verantwortlich zeichnet, dass in der Reaktionszone B die Selektivität der $CO_x$-Bildung im Beisein von molekularem Wasserstoff signifikant geringer ist, als in Abwesenheit von molekularem Wasserstoff. Sie bedingt infolge eines schnelleren Wärmeabtransports vom Reaktionsort in der Reaktionszone B niedrigere Katalysatoroberflächentemperaturen und demzufolge einen geringeren Anteil an Propylenvollverbrennung. Dies gilt insbesondere dann, wenn, wie beim erfindungsgemäßen Verfahren, thermisch höchst leitfähiger molekularer Wasserstoff und thermisch höchst aufnahmefähiges Propan in synergistischer Weise gemeinsam am Wärmeabtransport beteiligt sind.

Grundsätzlich kann das Reaktionsgas B beim erfindungsgemäßen Verfahren aber auch keinen molekularen Wasserstoff mehr enthalten.

Grundsätzlich kann das der Reaktionszone A entnommene, Propylen, Propan und Wasserdampf enthaltende Produktgas A als solches zur Beschickung des wenigstens einen Oxidationsreaktors in der Reaktionszone B verwendet werden. Erfindungsgemäß vorteilhaft ist es jedoch, in einer ersten Trennzone I wenigstens eine Teilmenge des im Produktgas A enthaltenen Wasserdampfes durch direkte und/oder indirekte Kühlung von Produktgas A auszukondensieren und so vom Produktgas A abzutrennen, wobei ein Produktgas A* verbleibt, dessen Gehalt an Wasserdampf geringer ist, als derjenige des Produktgases A.

Dies liegt zum einen darin begründet, dass die Zielprodukte Acrolein und/oder Acrylsäure eine wesentlich höhere Affinität zu Wasser aufweisen, als die Bestandteile des Produktgases A, weshalb die Zielproduktabtrennung von einem erhöhte Wasserdampfmengen enthaltenden Produktgas B mit einem signifikanten Energiebedarf einhergeht. Zum anderen mindern erhöhte Wasserdampfmengen im Reaktionsgas B üblicherweise die Standzeit der für die Partialoxidation verwendeten, in der Regel Mo in oxidischer Form enthaltenden, Katalysatoren, da $H_2O$ die Sublimation von Mo-Oxiden fördert. Zusätzlich mindert zwischen der Reaktionszone A und der Reaktionszone B abgetrennter Wasserdampf das Gesamtvolumen des Reaktionsgases B und damit den zur Förderung desselben durch die Reaktionszone B aufzuwendenden Energiebedarf.

Mit Vorteil werden beim erfindungsgemäßen Verfahren in der Trennzone I wenigstens 5 mol-%, besser wenigstens 10 mol-%, noch besser wenigstens 15 mol-%, besonders vorteilhaft wenigstens 20 mol-%, vorteilhafter wenigstens 25 mol-% oder wenigstens 30 mol-%, noch vorteilhafter wenigstens 35 mol-% oder wenigstens 40 mol-%, besser wenigsten 45 mol-% oder wenigstens 50 mol-%, mit besonderem Vorteil wenigstens 55 mol-% oder wenigstens 60 mol-%, bevorzugt wenigstens 65 mol-% oder wenigstens 70 mol-%, besonders bevorzugt wenigstens 75 mol-% oder wenigstens 80 mol-%, ganz besonders bevorzugt wenigstens 85 mol-% oder wenigstens 90 mol-%, vielfach wenigstens 95 mol-% oder im Extremfall bis zu 100 mol-% (häufig aber auch nicht mehr als 80 mol-%, oder nicht mehr als 85 mol-%, oder nicht mehr der 90 mol-%, oder nicht mehr als 95 mol-%, oder nicht mehr als 98 mol-%) des im Produktgas A enthaltenen Wasserdampfes kondensativ abgetrennt. Ein beschränkter (aber nicht verschwindender) Wasserdampfgehalt im Reaktionsgas B ist insofern günstig, als er sich normalerweise vorteilhaft auf die Aktivität der für die Reaktionszone B verwendeten Katalysatoren (insbesondere im Fall von Multimetalloxiden) auswirkt.

Für das erfindungsgemäße Verfahren günstig ist es, dass die Abtrennung des Wassers zwischen der Reaktionszone A und der Reaktionszone B durch einfaches Kondensieren geleistet werden kann.

Bei zwischen der Reaktionszone A und der Reaktionszone B abgetrenntem Wasser kann es sich dabei nicht nur um durch die Wasserstoffoxidation in der Reaktionszone A entstandenes Wasser handeln. Vielmehr kann in der Reaktionszone A zusätzlich Wasserdampf auch als inertes Verdünnungsgas mitverwendet worden sein, das gegebenenfalls gleichfalls teilweise oder vollständig in der Trennzone I abgetrennt wird.

Aufgrund der vergleichsweise hohen Siedetemperatur von Wasser, insbesondere bei erhöhten Drücken, ist vorgenannte Kondensation normalerweise durch einfaches direktes und/oder indirektes Abkühlen des Produktgases A zu erreichen. Grundsätzlich kann die Kondensation durch einstufiges oder mehrstufiges Abkühlen bewirkt werden. Erfindungsgemäß zweckmäßig wird man die kondensative Abtrennung des Wassers durch mehrstufiges Abkühlen bewirken. Anwendungstechnisch vorteilhaft wird man dabei direktes und indirektes Abkühlen kombiniert anwenden. Als indirekte Wärmeaustauscher eignen sich für den erfindungsgemäßen Zweck grundsätzlich alle bekannten indirekten Wärmeaustauschertypen. Bevorzugt werden unter diesen Rohrbündelwärmeaustauscher und Plattenwärmeaustauscher angewendet. Beispielsweise kann in einer ersten Abkühlstufe das der Reaktionszone A heiß (typische Temperaturen des Produktgases A betragen z. B. 400 bis 700°C, vorzugsweise 500 bis 600°C; typische Arbeitsdrücke des Produktgases A betragen in der Regel > 1 oder 1,5 bis 5 bar, bzw. 2 bis 5 bar, vorzugsweise 1,5 bzw. 2 bis 3 bar) entnommene Produktgase A in einem ersten (ganz generell kann beim erfindungsgemäßen Verfahren heißes, der Reaktionszone A entnommenes Produktgas A verwendet werden, um durch indirekten Wärmeaustausch, gegebenenfalls in einer Serie von hintereinandergeschalteten Wärmeaustauschern (z. B. zwei hintereinander geschalteten Wärmeaustauschern) wenigstens einen Teil der Bestandteile oder alle Bestandteile des Reaktionsgas A-Beschickungsgasgemischs (auf die jeweils gewünschte Temperatur) zu erwärmen, und das Produktgas A gleichzeitig abzukühlen) indirekten Wärmeaustauscher z. B. im Gleich- oder Gegenstrom zum in der Trennzone IV aus dem Absorbat abgetrennten, Propan enthaltenden Gasstrom (Propankreisgas) geführt werden, um diesen als einen der in die Reaktionszone A

geführten, Propan enthaltenden, Zuführströme auf das für die Reaktionszone A gewünschte Eingangstemperaturniveau zu bringen. Dieses Temperaturniveau kann sich in für das erfindungsgemäße Verfahren typischer Weise z. B. im Bereich von 400 bis 600°C, vorzugsweise 450 bis 550°C bewegen. In einem nachfolgenden zweiten indirekten Wärmeaustauscher wird man das wie beschrieben bereits abgekühlte Produktgas A in zweckmäßiger Weise z. B. im Gleich- oder im Gegenstrom zu Produktgas A führen, aus welchem der in diesem enthaltene Wasserdampf im gewünschten Umfang zuvor bereits abgetrennt worden ist (d.h., man führt im Gleich- oder im Gegenstrom zu Produktgas A*), um dieses wieder auf ein Temperaturniveau zu heben, wie es für die nachfolgende Partialoxidation des darin enthaltenen Propylens angemessen und zweckmäßig ist. Beim Verlassen des zweiten indirekten Wärmeaustauschers wird das Produktgas A in der Regel noch eine oberhalb von 100°C liegende Temperatur aufweisen. In einem dritten indirekten Wärmeaustauscher, der normalerweise als Luftkühler ausgestattet ist (z. B. Gleich- oder Gegenstromführung des Produktgases A zu Luft), wird man dann die Temperatur des Produktgases A auf eine unterhalb von 100°C liegende Temperatur absenken. Im sich daran anschließenden Direktkühler kann dann die Abtrennung des Wasserdampfes vorgenommen werden. Der Direktkühler (Direktkondensator) kann dabei von an sich bekannter Bauart sein (z. B. kann er einer Rektifikationskolonne entsprechen) und die (z. B. für Rektifikationskolonnen) üblichen Einbauten zum Zweck der Oberflächenvergrösserung der Wärmeaustauschfläche aufweisen. In der Regel wird man aber auf solche Einbauten verzichten. Üblicherweise wird der Kolonnenkörper des Direktkondensators nicht isoliert. Bevorzugt sind alle Maßnahmen, die die Wärmeverluste über die Kolonnenwand erhöhen, beispielsweise parallel zur Kolonnennoberfläche angeschweißte Lochbleche oder Kühlrippen. Als Kühlmittel wird man zum Zweck der Direktkühlung des Produktgases A vorteilhaft zuvor aus anderem Produktgas A kondensativ abgetrennte wässrige Phase verwenden, die man zweckmäßig vorab der Verwendung zur Direktkühlung in einem indirekten Wärmeaustauscher mit Hilfe von Kühlwasser (anwendungstechnisch zweckmäßig Oberflächenwasser) auf Temperaturen von $\leq$ 35 °C, vorzugsweise $\leq$ 30 °C abkühlt. Im Direktkühler können das Produktgas A und feinteilig versprühtes wässriges Direktkühlmittel im Gleich- oder im Gegenstrom geführt werden. In der Regel wird eine Gleichstromführung bevorzugt.

Mit Hilfe von an sich bekannten Abscheidern (z. B. über mechanische Tropfenabscheider) können die wässrige Phase und die verbliebene gasförmige Phase voneinander getrennt werden. Nach wie bereits beschrieben durchzuführender Abkühlung der abgetrennten wässrigen Phase kann diese wiederum der Direktkühlung von Produktgas A zugeführt werden.

Die Temperatur der verbliebenen gasförmigen Phase (sie bildet im beschriebenen Fall das Produktgas A*) kann durch nachfolgenden (vorstehend bereits beschriebenen) indirekten Wärmeaustausch mit noch von Wasserdampf zu befreiendem Produktgas A auf eine für die nachfolgende Partialoxidation des Propylens angemessene Temperatur angehoben werden. Prinzipiell kann beim erfindungsgemäßen Verfahren auch nur von einer Teilmenge des Produktgases A darin enthaltener Wasserdampf kondensativ abgetrennt werden. Das dadurch gebildete Produktgas A* kann nachfolgend als solches oder im Gemisch mit der nicht kondensativ behandelten Teilmenge des Produktgas A (dieses Gemisch bildet im erfindungsgemäßen Sinne gleichfalls ein Produktgas A*) zur Beschickung der heterogen katalysierten Partialoxidation des Propylens verwendet werden.

Eine weitere vorteilhafte Folgewirkung einer Direktkühlung von Produktgas A (z. B. mit zuvor aus Produktgas A kondensativ abgetrennter, vorzugsweise durch indirekten Wärmeaustausch abgekühlter, wässriger Phase) besteht generell darin, dass mit der Direktkühlung gleichzeitig eine Abscheidung von im Produktgas A gegebenenfalls enthaltenen Feststoffpartikeln bewirkt wird, bei denen es sich z. B. um aus der heterogen katalysierten partiellen Dehydrierung des Propans herrührende Dehydrierkatalysatorpartikel handeln kann, die sich gemäß der Lehre der DE-A 103 160 39 in der nachfolgenden heterogen katalysierten partiellen Oxidation des Propylens störend auswirken können. Letzteres insbesondere dann, wenn das zur Beschickung der Partialoxidation verwendete Produktgas A bzw. Produktgas A* noch molekularen Wasserstoff enthält. D. h., erfindungsgemäß zweckmäßig ist zwischen die Reaktionszone A und die Reaktionszone B ganz generell eine mechanische Trennoperation gemäß der DE-A 103 160 39 geschaltet, und sei es nur in Form einer wie beschrieben durchzuführenden Direktkühlung des Reaktionsgases A.

Die kondensative Abtrennung von Wasserdampf aus Produktgas A in der Trennzone I ist deshalb dann ganz besonders vorteilhaft, wenn sie eine Direktkühlung von Produktgas A mit, vorzugsweise zuvor aus Produktgas A abgetrennter und abgekühlter (vorzugsweise durch indirekten Wärmeaustausch), wässriger Phase umfasst.

Die wie beschrieben kondensativ abgetrennte wässrige Phase wird in der Regel noch Propan gelöst enthalten, das in einfacher Weise z. B. durch Erwärmen der wässrigen Phase (gegebenenfalls unter vermindertem Druck) rückgewonnen und als weiterer Propan enthaltender Zuführstrom in die Reaktionszone A rückgeführt werden kann. Mit der direkten und/oder indirekten Kühlung des Produktgases A zum Zweck der kondensativen Abtrennung von in Produktgas A enthaltenem Wasser geht in selbigem in der Regel ein Druckverlust von $\leq$ 1 bar, meist $\leq$ 0,5 bar, vorzugsweise $\leq$ 0,3 bar einher.

Die Oxidation von molekularem Wasserstoff zu Wasser in der Reaktionszone A kann im Anschluss an die eigentliche heterogen katalysierte partielle Dehydrierung des Propans und/oder im Verlauf der heterogen katalysierten partiellen Dehydrierung des Propans durchgeführt werden, z. B. dann, wenn, bezogen auf den bei einmaligem Durchgang des Reaktionsgases A durch die Reaktionszone A sich ergebenden Umsatz des Propans, bestimmte Teilumsatzwerte erreicht worden sind. Erfindungsgemäß vorteilhaft wird wenigstens eine Teilmenge des in der Reaktionszone A zu Wasser oxidierten Wasserstoff bereits zu Wasser oxidiert, bevor der in der Reaktionszone A angestrebte Zielumsatz (und erreichte Endumsatz) des Propans (bezogen auf einmaligen Durchgang des Reaktionsgases A durch die Reaktionszone A) erreicht ist.

Diese Vorgehensweise ist zum einen insofern vorteilhaft, als sie durch Entzug von molekularem Wasserstoff aus dem Gleichgewicht der heterogen katalysierten Dehydrierung die Gleichgewichtslage im Sinne der gewünschten Propylenbildung verschiebt. Sie ist zusätzlich aber auch insofern günstig, als die bei der Wasserstoffoxidation anfallende Reaktionswärme die bei der zuvor erfolgten Dehydrierung verbrauchte Reaktionswärme wieder ausgleichen, und für die nachfolgende weitergehende Dehydrierung Reaktionswärme zur Verfügung stellen kann. Erfindungsgemäß vorteilhaft wird man in der Reaktionszone A spätestens dann (wenigstens in einer Teilmenge) molekularen Wasserstoff mit molekularem Sauerstoff verbrennen, nachdem 90 mol-%, besser nachdem 75 mol-% des in der Reaktionszone A insgesamt gebildeten molekularen Wasserstoff gebildet worden sind. Erfindungsgemäß bevorzugt wird man in der Reaktionszone A spätestens dann (wenigstens in einer Teilmenge) molekularen Wasserstoff mit molekularem Sauerstoff verbrennen, nachdem 65 mol-%, oder 55 mol-%, oder 45 mol-% des in der Reaktionszone A insgesamt gebildeten molekularen Wasserstoff gebildet worden sind.

Der Zeitpunkt einer Verbrennung von molekularem Wasserstoff in der Reaktionszone A ist zum einen dadurch beeinflussbar, wo (wann) in die Reaktionszone A molekularer Sauerstoff zugeführt wird.

Führt man der Reaktionszone A eine Teilmenge (deren Zusammensetzung derjenigen des Restgases entspricht) des in der Trennzone II verbleibenden, Propan enthaltenden, Restgases als solches (d. h. ohne die Trennzonen III/IV zu durchlaufen) zu, kann bereits das Beschickungsgasgemisch der Reaktionszone A molekularen Sauerstoff enthalten, da das vorgenannte Restgas in der Regel noch in der Reaktionszone B im Überschuss angewandten molekularen Sauerstoff enthält.

Desweiteren ist er durch die Gestaltung der Beschickung der Reaktionszone A mit Katalysator beeinflussbar. So kann die Reaktionszone A lokal mit Katalysator beschickt werden, der äußerst selektiv nur die Oxidation von molekularem Wasserstoff mit molekularem Sauerstoff zu Wasser katalysiert. Andere Katalysatoren vermögen äußerst selektiv die Dehydrierung zu katalysieren. In der Regel vermögen diejenigen Katalysatoren, die die Dehydrierung zu katalysieren vermögen, aber auch die Wasserstoffverbrennung zu katalysieren, wobei sie im Normalfall die Aktivierungsenergie der letztgenannten Reaktion besonders ausgeprägt zu verringern vermögen.

Unter Frischpropan wird in dieser Schrift Propan verstanden, das noch nicht an einer Dehydrierung in der Reaktionszone A teilgenommen hat. In der Regel wird es als Bestandteil von Roh-Propan (das vorzugsweise die Spezifikation gemäß DE-A 10246119 und DE-A 10245585 erfüllt) zugeführt, das in geringen Mengen auch von Propan verschiedene Komponenten enthält.

Solches Roh-Propan ist beispielsweise nach in der DE-A 102005 022798 beschriebenem Verfahren erhältlich. In der Regel wird der Reaktionszone A beim erfindungsgemäßen Verfahren neben Frischpropan als weiterer Propan enthaltender Zufuhrstrom nur noch das in der Trennzone IV aus dem Absorbat abgetrennte Propan (sowie gegebenenfalls eine Teilmenge an Restgaskreisgas) zugeführt.

Bevorzugt erfolgt beim erfindungsgemäßen Verfahren eine Zufuhr von Frischpropan ausschließlich in die Reaktionszone A hinein, als Bestandteil des Beschickungsgasgemischs für die Reaktionszone A. Prinzipiell können Teilmengen des Frischpropan aus Gründen der Explosionssicherheit aber auch in die Beschickungsgasgemische der ersten und/oder zweiten Oxidationsstufe der Reaktionszone B hinein zugeführt werden bzw. an jeder anderen Stelle im Verfahren.

Unter der Belastung eines einen Reaktionsschritt katalysierenden Katalysatorbetts mit Reaktionsgas, wird in dieser Schrift die Menge an Reaktionsgas in Normlitern (= Nl; das Volumen in Litern, das die entsprechende Reaktionsgasmenge bei Normalbedingungen (0 °C, 1 bar) einnehmen würde) verstanden, die pro Stunde durch einen Liter Katalysatorbett (z. B. Katalysatorfestbett) geführt wird.

Die Belastung kann auch nur auf einen Bestandteil des Reaktionsgases bezogen sein. Dann ist es die Menge dieses Bestandteils in Nl/l·h, die pro Stunde durch einen Liter des Katalysatorbetts geführt wird (reine Inertmaterialschüttungen werden nicht zum Katalysatorfestbett gerechnet). Besteht das Katalysatorbett aus einer Mischung aus Katalysator und inerten Verdünnungsformkörpern, kann die Belastung, soweit dies entsprechend erwähnt wird, auch nur auf die Volumeneinheit an enthaltenem Katalysator bezogen sein.

Als ein Inertgas soll in dieser Schrift generell ein Reaktionsgasbestandteil verstanden werden, der sich unter den Bedingung der entsprechenden Reaktion im wesentlichen als chemisch inert verhält und -jeder inerte Reaktionsgasbestandteil für sich betrachtet - zu mehr als 95 mol-%, vorzugsweise zu mehr als 97 mol% bzw. zu mehr als 99 mol-% chemisch unverändert erhalten bleibt.

Als Quelle für den in der Reaktionszone A benötigten molekularen Sauerstoff kommt neben aus der Trennzone II stammendem Restgaskreisgas molekularer Sauerstoff als solcher, oder ein Gemisch aus moleklarem Sauerstoff und einem (oder einem Gemisch solcher inerter Gase) sich in der Reaktionszone A chemisch inert verhaltenden Gas (z. B. Edelgase wie Argon, molekularer Stickstoff, Wasserdampf, Kohlendioxid etc.) in Betracht. D. h., der molekulare Sauerstoff kann der Reaktionszone A als Gas zugeführt werden, das nicht mehr als 50 Vol.-%, vorzugsweise nicht mehr als 40 Vol.-% oder nicht mehr als 30 Vol.-%, vorteilhaft nicht mehr als 25 Vol.-% oder nicht mehr als 20 Vol-%, besonders vorteilhaft nicht mehr als 15 Vol.-% oder nicht mehr als 10 Vol.-% und besonders bevorzugt nicht mehr als 5 Vol.-% oder nicht mehr als 2 Vol.-% anderer (von molekularem Sauerstoff verschiedener) Gase (Bestandteile) enthält. Da der Sauerstoffbedarf in der Reaktionszone A im Vergleich zu jenem in der Reaktionszone B beim erfindungsgemäßen Verfahren ein vergleichsweise geringerer ist, ist aber auch eine Verwendung von Luft als Sauerstoffquelle für den Sauerstoffbedarf in der Reaktionszone A beim erfindungsgemäßen Verfahren insbesondere aus Gründen der Wirtschaftlichkeit günstig. Unter dem Aspekt möglichst kleiner zu fördernder und zu komprimierender Gasvolumina ist jedoch die Zufuhr als Reinsauerstoff in die Reaktionszone A bevorzugt. In die Reaktionszone A rückgeführtes Propankreisgas (aus der Trennzone IV kommend) weist molekularen Sauerstoff in der Regel nur in Spuren auf.

Das Vorgenannte gilt im wesentlichen in gleicher Weise für die Zufuhr des in der Reaktionszone B benötigten molekularen Sauerstoff. D. h., die Zufuhr von molekularem Sauerstoff als Reinsauerstoff sowohl für den Sauerstoffbedarf der Reaktionszone A als auch der Reaktionszone B ist insbesondere deshalb vorteilhaft, weil sie die Gasströme des erfindungsgemäßen Verfahrens nicht über das Unabdingbare hinausgehend mit Inertgas belastet, das im weiteren Verlauf des Kreisverfahrens wieder ausgelassen werden müsste.

Typische Reaktionsgase B, mit denen die Reaktionszone B beim erfindungsgemäßen Verfahren beschickt werden kann, weisen nachfolgende Gehalte auf:

| | |
|---|---|
| 4 bis 25 Vol.-% | Propylen, |
| 6 bis 70 Vol.-% | Propan, |
| 0 bis 60 Vol.-% | $H_2O$, |
| 5 bis 60 Vol.-% | $O_2$ und |
| 0 bis 20, vorzugsweise bis 10 Vol.-% | $H_2$. |

Erfindungsgemäß bevorzugte Reaktionsgase B weisen nachfolgende Gehalte auf:

| | |
|---|---|
| 7 bis 25 Vol.-% | Propylen, |
| 10 bis 40 Vol.-% | Propan, |
| 1 bis 10 Vol.-% | $H_2O$, |
| 10 bis 30 Vol.-% | $O_2$ und |
| 0 bis 10 Vol.-% | $H_2$. |

Erfindungsgemäß für vorgenannte Beschickung ganz besonders bevorzugte Reaktionsgase B weisen nachfolgende Gehalte auf:

| | |
|---|---|
| 15 bis 25 Vol.-% | Propylen, |
| 20 bis 40 Vol.-% | Propan, |
| 2 bis 8 Vol.-,% | $H_2O$, |
| 15 bis 30 Vol.-% | $O_2$ und |
| 0 bis 5 Vol.-% | $H_2$. |

Die Vorteilhaftigkeit mittlerer Propangehalte im Reaktionsgas B ergibt sich z. B. aus der DE-A 102 45 585.

Innerhalb der vorgenannten Zusammensetzungsraster ist es günstig, wenn das molare Verhältnis $V_1$ von im Reaktionsgas B enthaltenem Propan zu im Reaktionsgas B enthaltenem Propylen 1 bis 9 beträgt. Ferner ist es innerhalb der vorgenannten Zusammensetzungsraster vorteilhaft, wenn das Verhältnis $V_2$ von im Reaktionsgas B enthaltenem molekularem Sauerstoff zu im Reaktionsgas B enthaltenem Propylen 1 bis 2,5 beträgt. Weiterhin ist es im Sinne der vorliegenden Erfindung innerhalb der vorgenannten Zusammensetzungsraster vorteilhaft, wenn das Verhältnis $V_3$ von im Reaktionsgas B enthaltenem molekularem Wasserstoff zu im Reaktionsgas B enthaltenem Propylen 0 bis 0,80 bzw. 0 bis 0,60 oder 0,1 bis 0,50 beträgt. Häufig beträgt das Verhältnis $V_3$ auch 0,4 bis 0,6. Auch ist es innerhalb der vorgenannten Zusammensetzungsraster von Vorteil, wenn das molare Verhältnis $V_4$ von im Reaktionsgas B enthaltenem Wasserdampf zur molaren Gesamtmenge an im Reaktionsgas B enthaltenem Propan und Propylen 0 bzw. 0,001 bis 10 beträgt.

Mit besonderem Vorteil beträgt $V_1$ im zur Beschickung der Reaktionszone B verwendeten Reaktionsgas B (im Reaktionsgas B-Ausgangsgemisch) 1 bis 7 oder bis 4, bzw. 2 bis 6 und besonders günstig 2 bis 5, bzw. 3,5 bis 4,5. Weiterhin ist es für das Reaktionsgas B-Ausgangsgemisch bevorzugt, wenn $V_2$ 1,2 bis 2,0, bzw. 1,4 bzw. 1,8 beträgt.

In entsprechender Weise beträgt $V_4$ im Reaktionsgas B-Ausgangsgemisch bevorzugt 0,015 bis 5, besser 0,01 bis 3, vorteilhaft 0,01 bis 1 und besonders vorteilhaft 0,01 bis 0,3 bzw. bis 0,1. Nicht explosive Reaktionsgas B-Ausgangsgemische sind erfindungsgemäß bevorzugt.

Entscheidend für die Beantwortung der Frage, ob das Reaktionsgas B-Ausgangsgemisch explosiv ist oder nicht, ist, ob sich unter dem unter bestimmten Bedingungen (Druck, Temperatur) befindlichen Gemisch eine durch eine örtliche Zündquelle (z. B. glühender Platindraht) eingeleitete Verbrennung (Entzündung, Explosion) ausbreitet oder nicht (vgl. DIN 51649 und die Untersuchungsbeschreibung in der WO 04/007405). Erfolgt eine Ausbreitung, soll das Gemisch hier als explosiv bezeichnet werden. Erfolgt keine Ausbreitung, wird das Gemisch in dieser Schrift als nicht explosiv eingeordnet. Ist das Reaktionsgasausgangsgemisch einer erfindungsgemäßen Partialoxidation nicht explosiv, gilt dies normalerweise auch für die im Verlauf der Partialoxidation aus selbigem gebildeten Reaktionsgasgemische (vgl. WO 04/007405).

Ganz generell ist es für das erfindungsgemäße Verfahren typisch, dass der Gesamtgehalt des Reaktionsgas B-Ausgangsgemischs an von Propylen, molekularem Wasserstoff, Wasserdampf, Propan, molekularem Stickstoff und molekularem Sauerstoff verschiedenen Bestandteilen meist ≤ 20 Vol.-%, bzw. ≤ 15 Vol.-%, bzw. ≤ 10 Vol.-%, bzw. ≤ 5 Vol.-% beträgt. Von diesen sonstigen Bestandteilen des Reaktionsgas B-Ausgangsgemischs können bis zu 80 Vol.-% Ethan und/oder Methan sein. Im übrigen kann es sich bei solchen Gehalten vor allem um Kohlenoxide ($CO_2$, CO) und Edelgas, aber auch um Spuren von Nebenkomponentenoxygenaten wie Formaldehyd, Benzaldehyd, Methacrolein, Essigsäure, Propionsäure, Methacrylsäure usw. handeln. Selbstverständlich gehören auch Ethylen, iso-Buten, n-Butan und n-Butene zu diesen möglichen sonstigen Bestandteilen des Reaktionsgas B-Ausgangsgemischs.

Der Gehalt des Reaktionsgas B-Ausgangsgemischs an molekularem Stickstoff kann in einem weiten Bereich variieren. Grundsätzlich kann dieser Stickstoffgehalt bis zu 70 Vol.-% betragen. In der Regel wird der Gehalt des Reaktionsgas B-Ausgangsgemischs an molekularem Stickstoff ≤ 65 Vol.-%, bzw. ≤ 60 Vol.-%, bzw. ≤ 50 Vol.-%, oder ≤ 40 Vol.-%, oder ≤ 30 Vol.-%, oder ≤ 20 Vol.-%, oder ≤ 10 Vol.-%, oder ≤ 5 Vol.-% betragen. Prinzipiell kann der $N_2$-Gehalt des Reaktionsgas B-Ausgangsgemischs verschwindend sein. Eine Minimierung des $N_2$-Gehalts im Reaktionsgas B-Ausgangsgemisch minimiert das im erfindungsgemäßen Verfahren zu fördernde und zu komprimierende Gasvolumen. Umgekehrt mindert eine Verdünnung des Reaktionsgases B mit molekularem Stickstoff die Propionsäurenebenproduktbildung in der Reaktionszone B.

Grundsätzlich kommen für die heterogen katalysierte partielle Dehydrierung des Propans in der Reaktionszone A alle im Stand der Technik bekannten Verfahren der heterogen katalysierten partiellen Dehydrierung von Propan zu Propylen in Betracht, wie sie z. B. aus den Schriften WO 03/076370, WO 01/96271, EP-A 1 17 146, WO 03/011804, EP-A 7 310 77, US-A 3,162,60, WO 01/96270, DE-A 331 35 73, DE-A 102 45 585, DE-A 103 16 039, DE-A 10 2005 009891, DE-A 10 2005 013039, DE-A 10 2005 022 798, DE-A 10 2005 009 885, DE-A 10 2005 010 111, DE-A 10 2005 049 699, DE-A 10 2004 032 129, Deutsches Aktenzeichen 10 2005 056377.5, Deutsches Aktenzeichen 10 2006 017623.5, Deutsches Aktenzeichen 10 2006 015235.2, Deutsches Aktenzeichen 10 2005 061626.7 und Deutsches Aktenzeichen 10 2005 057197.2 sowie dem in diesen Schriften gewürdigten Stand der Technik vorbekannt sind (dies ist darauf zurückzuführen, dass sich die Oxidation (Verbrennung) des molekularen Wasserstoff mit molekularem Sauerstoff in der Reaktionszone A grundsätzlich an jedes Verfahren der heterogen katalysierten partiellen Dehydrierung von Propan anschließen kann. Prinzipiell können die heterogen katalysierte Dehydrierung des Propans und die Verbrennung des molekularen Wasserstoff innerhalb der Reaktionszone A auch in voneinander verschiedenen, z. B. räumlich hintereinander geschalteten, Reaktoren durchgeführt werden. Dabei wirkt es sich vorteilhaft aus, dass das Reaktionsgas B-Ausgangsgemisch beim erfindungsgemäßen Verfahren nicht in notwendiger Weise molekularen Sauerstoff enthält. Ganz generell kann das erfindungsgemäße Verfahren in der Reaktionszone A sowohl in nur einem einzigen Reaktor, als auch z. B. in einer Hintereinanderschaltung von mehr als einem Reaktor durchgeführt werden.

Bezogen auf den einmaligen Durchgang des der Reaktionszone A zugeführten Propan durch die Reaktionszone A, kann die Reaktionszone A grundsätzlich durch gezielten Wärmeaustausch mit außerhalb der Reaktionszone A geführten fluiden (d. h., flüssigen oder gasförmigen) Wärmeträgern isotherm gestaltet werden. Sie kann mit gleicher Bezugsbasis aber auch adiabat, d. h., im wesentlichen ohne einen solchen gezielten Wärmeaustausch mit außerhalb der Reaktionszone A geführten Wärmeträgern ausgeführt werden. In letzterem Fall kann die Bruttowärmetönung, bezogen auf den einmaligen Durchgang des der Reaktionszone A zugeführten Propan durch die Reaktionszone A, durch Ergreifen von in den vorstehenden Schriften empfohlenen und im Folgenden noch zu beschreibenden Maßnahmen sowohl endotherm (negativ), oder autotherm (im wesentlichen Null) oder exotherm (positiv) gestaltet werden. Ebenso können alle in den vorgenannten Schriften (einschließlich des in diesen gewürdigten Standes der Technik) empfohlenen Katalysatoren beim erfindungsgemäßen Verfahren angewendet werden. Prinzipiell ist die heterogen katalysierte Propandehydrierung in der Reaktionszone A, unabhängig davon, ob diese adiabat und/oder isotherm (längs der Reaktionszone A kann auch von adiabat nach isotherm gewechselt werden und umgekehrt) betrieben wird, sowohl in einem (oder in mehreren z. B. hintereinandergeschalteten) Festbettreaktor als auch in einem Wanderbett-, Fließbett- oder Wirbelbettreaktor (letzterer eignet sich aufgrund seiner Rückvermischung insbesondere für eine Aufheizung des Reaktionsgas A-Ausgangsgemischs auf die Reaktionstemperatur in der Reaktionszone A durch Wasserstoffverbrennung im Reaktionsgas A, wenn bereits dem Reaktionsgas A-Ausgangsgemisch molekularer Sauerstoff zugeführt worden ist) durchführbar.

In typischer Weise benötigt die heterogen katalysierte partielle Dehydrierung von Propan zu Propylen vergleichsweise hohe Reaktionstemperaturen. Der dabei erzielbare Umsatz an Propan erreicht normalerweise die Grenze des thermodynamischen Gleichgewichts. Typische Reaktionstemperaturen betragen 300 bis 800 °C bzw. 400 bis 700 °C, bzw. 450 bis 650 °C. Pro Molekül an zu Propylen dehydriertem Propan wird dabei ein Molekül Wasserstoff erzeugt. Der Arbeitsdruck in der gesamten Reaktionszone A beträgt erfindungsgemäß zweckmäßig > 1 bis 5 bar, bevorzugt 1,5 bis 4 bar und vorteilhaft 2 bis 3 bar. Grundsätzlich kann der Arbeitsdruck in der Reaktionszone A aber auch bei höheren oder niedrigeren Werten als den vorstehend genannten liegen. Hohe Temperaturen und eine Entfernung des Reaktionsproduktes $H_2$ begünstigen in der Reaktionszone A die Dehydriergleichgewichtslage im Sinne des in der Reaktionszone A zu bildenden Propylens.

Da die heterogen katalysierte Dehydrierreaktion unter Volumenzunahme verläuft, kann der Umsatz durch Erniedrigung des Partialdrucks der Dehydrierprodukte gesteigert werden. Dies lässt sich in einfacher Weise zum Beispiel durch Dehydrierung bei vermindertem Druck (allerdings ist eine Durchführung bei erhöhtem Druck in der Regel vorteilhaft für die Katalysatorstandzeit) und/oder durch Zumischen von im wesentlichen inerten Verdünnungsgasen, wie zum Beispiel Wasserdampf, erreichen, der für die Dehydrierreaktion im Normalfall ein Inertgas darstellt. Eine Verdünnung mit Wasserdampf bedingt als weiteren Vorteil in der Regel ein vermindertes Verkoken des verwendeten Katalysators, da der Wasserdampf mit gebildetem Koks nach dem Prinzip der Kohlevergasung reagiert. Die Wärmekapazität des Wasserdampfs vermag auch einen Teil der Endothermie der Dehydrierung auszugleichen.

Während sich Wasserdampf in begrenzten Mengen in der nachfolgenden Reaktionszone B für die Aktivität der Partialoxidationskatalysatoren normalerweise als förderlich erweist, ist eine darüber hinausgehende Menge aus den bereits erwähnten Gründen in der Reaktionszone B nachteilig. Erfindungsgemäß muss darüber hinaus in der Reaktionszone A wenigstens eine Wasserstoffteilmenge zu Wasserdampf oxidiert werden. Es ist deshalb erfindungsgemäß vorteilhaft, wenn die der Reaktionszone A zugeführte Menge an Wasserdampf, bezogen auf das der Katalysatorbeschickung der Reaktionszone A zugeführte Reaktionsgas A ≤ 20 Vol.-%, bevorzugt ≤ 15 Vol.-% und besonders bevorzugt ≤ 10 Vol.-% beträgt. In der Regel wird die der Reaktionszone A zugeführte Menge an Wasserdampf, in gleicher Weise bezogen, normalerweise jedoch ≥ 1 Vol.-%, vielfach ≥ 2 Vol.-%, oder ≥ 3 Vol.-% und häufig ≥ 5 Vol.-% betragen.

Weitere für die heterogen katalysierte Propandehydrierung geeignete Verdünnungsmittel sind z. B. Stickstoff, Edelgase wie He, Ne und Ar, aber auch Verbindungen wie CO, $CO_2$, Methan und Ethan. Alle genannten Verdünnungsmittel können entweder für sich oder in Form unterschiedlicher Gemische mitverwendet werden. Soweit die vorgenannten Verdünnungsgase beim erfindungsgemäßen Kreisgasverfahren als Nebenprodukt gebildet, oder als Frischgas bzw. Frischgasbestandteil zugeführt werden, bedarf es beim erfindungsgemäßen Verfahren eines Auslasses in entsprechender Menge, weshalb eine entsprechende Frischgaszufuhr erfindungsgemäß weniger bevorzugt ist. Solche mögliche Auslässe befinden sich in den verschiedenen Trennzonen, insbesondere in den Trennzonen III und IV.

Prinzipiell kommen für die heterogen katalysierte Propandehydrierung alle im Stand der Technik bekannten Dehydrierkatalysatoren in Betracht. Sie lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen (z. B. Zirkondioxid, Magnesiumoxid, Aluminiumoxid, Siliziumdioxid, Titandioxid, Lanthanoxid und/oder Ceroxid), Träger abgeschiedenen, in der Regel vergleichsweise edlen, Metall (zum Beispiel Platin) bestehen. Unter anderem können damit alle Dehydrierkatalysatoren eingesetzt werden, die in der WO 01/96270, der EP-A 731077, der DE-A 10211275, der DE-A 10131297, der WO 99/46039, der US-A 4 788 371, der EP-A 705 136, der WO 99/29420, der US-A 4 220 091, der US-A 5 430 220, der US-A 5 877 369, der EP-A 117 146, der DE-A 199 37 196, der DE-A 199 37 105, der US-A 3 670 044, der US-A 6 566 573, der WO 94/29021 sowie der DE-A 199 37 107 empfohlen werden. Im besonderen können sowohl der Katalysator gemäß Beispiel 1, Beispiel 2, Beispiel 3, und Beispiel 4 der DE-A 199 37 107 eingesetzt werden. Schließlich seien auch noch die Katalysatoren der deutschen Anmeldung Nr. 10 2005 044916 als in der Reaktionszone A für die Dehydrierung zu verwendende Katalysatoren empfohlen. Dabei können diese Katalysatoren beim erfindungsgemäßen Verfahren innerhalb der Reaktionszone A die alleinigen verwendeten Katalysatoren sein, da sie in der Regel auch die Verbrennung von molekularem Wasserstoff mit molekularem Sauerstoff zu Wasser katalysieren.

Grundsätzlich kann eine katalytisch aktive Schüttung innerhalb der Reaktionszone A ausschließlich aus katalytisch aktiven Formkörpern bestehen. Selbstredend kann eine katalytisch aktive Schüttung innerhalb der Reaktionszone A aber auch aus mit inerten Formkörpern verdünnten katalytisch aktiven Formkörpern bestehen. Solche inerten Formkörper können z. B. aus gebrannten Tonen (Aluminiumsilicaten) oder Steatit (z. B. C 220 der Fa. CeramTec), oder sonstigen (vorzugsweise im wesentlichen an Poren freien) Hochtemperaturkeramikmaterialien wie Aluminiumoxiden, Siliciumdioxid, Titandioxid, Magnesiumoxid, Lanthanoxid, Ceroxid, Zinkaluminiummischoxid, Thoriumdioxid, Zirkondioxid, Siliciumcarbid oder sonstigen Silicaten wie Magnesiumsilicat und Mischungen aus vorgenannten Materialien gefertigt sein. Vorgenannte Materialien kommen auch für eine inerte Deck- und gegebenenfalls Abschlussschüttung des Katalysatorfestbetts des erfindungsgemäßen Verfahrens in Betracht.

Dabei handelt es sich um Dehydrierkatalysatoren, die 10 bis 99,9 Gew.-% Zirkondioxid, 0 bis 60 Gew.-%

Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

Besonders geeignet ist auch der in den Ausführungsbeispielen dieser Schrift eingesetzte Dehydrierkatalysator. In einer bevorzugten Ausführungsform enthalten vorgenannte Dehydrierkatalysatoren wenigstens ein Element der VIII. Nebengruppe, mindestens ein Element der I. und II. Hauptgruppe, mindestens ein Element der III. und/oder IV. Hauptgruppe und mindestens ein Element der III. Nebengruppe einschließlich Lanthaniden und Actiniden. Als Element der VIII. Nebengruppe enthält die Aktivmasse der Dehydrierkatalysatoren bevorzugt Platin und/oder Palladium, besonders bevorzugt Platin. Als Elemente der I. und II. Hauptgruppe enthält die Aktivmasse der vorgenannten Dehydrierkatalysatoren bevorzugt Kalium und/oder Cäsium. Als Elemente der III. Nebengruppe einschließlich der Lanthaniden und Actiniden enthält die Aktivmasse der vorgenannten Dehydrierkatalysatoren bevorzugt Lanthan und/oder Cer. Als Elemente der III. und/oder IV. Hauptgruppe enthält die Aktivmasse der vorgenannten Dehydrierkatalysatoren bevorzugt ein oder mehrere Elemente aus der Gruppe bestehend aus Bor, Gallium, Silizium, Germanium, Zinn und Blei, besonders bevorzugt Zinn.

Ganz besonders bevorzugt enthält die Aktivmasse der vorgenannten Dehydrierkatalysatoren jeweils wenigstens einen Vertreter der vorgenannten Elementgruppen.

Generell kann es sich bei den Dehydrierkatalysatoren um Katalysatorstränge (Durchmesser typisch 0,1 bzw. 1 bis 10 mm, bevorzugt 1,5 bis 5 mm; Länge typisch 1 bis 20 mm, bevorzugt 3 bis 10 mm), Tabletten (vorzugsweise gleiche Abmessungen wie bei den Strängen) und/oder Katalysatorringe (Außendurchmesser und Länge jeweils typisch 2 bis 30 mm oder bis 10 mm, Wandstärke zweckmäßig 1 bis 10 mm, oder bis 5 mm, oder bis 3 mm) handeln. Zur Durchführung der heterogen katalysierten Dehydrierung im Wirbelbett (bzw. Fließ- oder Wanderbett) wird man entsprechend feinteiligeren Katalysator einsetzen. Erfindungsgemäß bevorzugt ist für die Reaktionszone A die Anwendung von Katalysatorfestbetten.

Grundsätzlich bestehen weder hinsichtlich der in den Katalysatorfestbetten zu verwendenden Katalysatorgeometrie (insbesondere im Fall von Trägerkatalysatoren) noch hinsichtlich der zu verwendenden Geometrie der inerten Formkörper beim erfindungsgemäßen Verfahren Einschränkungen. Besonders häufig verwendete Geometrien sind Vollzylinder, Hohlzylinder (Ringe), Kugeln, Kegel, Pyramiden und Würfel sowie Stränge, Wagenräder, Sterne und Monolithe.

Die Längstausdehnung der Katalysatorformkörper sowie der Inertformkörper (längste direkte Verbindungslinie zweier auf der Formkörperoberfläche befindlicher Punkte) kann dabei 0,5 mm bis 100 mm, oft 1,5 mm bis 80 mm, und vielfach 3 mm bis 50 mm bzw. bis 20 mm betragen.

In der Regel sind die Dehydrierkatalysatoren (insbesondere die in dieser Schrift beispielhaft verwendeten sowie die in der DE-A 19 937 107 empfohlenen (insbesondere die beispielhaften Katalysatoren dieser DE-A) so beschaffen, dass sie sowohl die Dehydrierung von Propan als auch die Verbrennung von molekularem Wasserstoff und von Propan/Propylen zu katalysieren vermögen. Die Wasserstoffverbrennung verläuft dabei sowohl im Vergleich zur Dehydrierung des Propan als auch im Vergleich zu z. B. dessen Verbrennung im Fall einer Konkurrenzsituation an diesen Katalysatoren sehr viel schneller (d. h., sie bedingen für die Verbrennung von molekularem Wasserstoff unter den gegebenen Bedingungen die mit Abstand niedrigste Aktivierungsenergie). Die Verbrennung von Propan/Propylen verläuft in der Regel an diesen Katalysatoren wiederum schneller als die Dehydrierung.

Der vorgenannte Zusammenhang ermöglicht es, beim erfindungsgemäßen Verfahren mit der Verwendung lediglich eines Katalysatortyps in der Reaktionszone A auszukommen.

Mit Vorteil ist dabei in der Reaktionszone A dabei darauf zu achten, dass immer dann, wenn das Reaktionsgas A molekularen Sauerstoff enthält und vorstehend beschriebene Katalysatoren berührt, das Reaktionsgas A relativ zur darin enthaltenen Menge an molekularem Sauerstoff wenigstens die zweifache Menge an molekularem Wasserstoff enthält, da andernfalls das Risiko einer Verbrennung von Propan und/oder Propylen wächst und diese die auf den umgesetzten Rohstoff Propan bezogene Zielproduktausbeute beim erfindungsgemäßen Verfahren mindert.

Selbstredend können in der Reaktionszone A die für die Verbrennung des molekularen Wasserstoff zu Wasser verwendeten Katalysatoren aber auch solche sein, die diese Reaktion spezifisch katalysieren. Derartige Katalysatoren sind z. B. in den Schriften US-A 4788371, US-A 4886928, US-A 5430209, US-A 5530171, US-A 5527979 und US-A 5563314 beschrieben.

Zur Durchführung der heterogen katalysierten Propandehydrierung kommen prinzipiell alle im Stand der Technik bekannten Reaktortypen und Verfahrensvarianten in Betracht.

Beschreibungen solcher Verfahrensvarianten enthalten zum Beispiel alle bezüglich der Dehydrierkatalysatoren zitierten Schriften des Standes der Technik, sowie der eingangs dieser Schrift zitierte Stand der Technik.

Eine vergleichsweise ausführliche Beschreibung von erfindungsgemäß geeigneten Dehydrierverfahren enthält auch Catalytica® Studies Division, Oxidative Deyhdrogenation and Alternative Dehydrogenation Process, Study Number 4192 OD, 1993, 430 Ferguson Drive, Montain View, California, 94043-5272 U.S.A..

Aufgrund der für die heterogen katalysierte Dehydrierung des Propans benötigten vergleichsweise hohen Reaktionstemperaturen werden in der Reaktionszone A beim erfindungsgemäßen Verfahren in der Regel in geringen Mengen schwersiedende hochmolekulare organische Verbindungen, bis hin zum Kohlenstoff, gebildet, die sich auf der Katalysatoroberfläche abscheiden und selbige dadurch deaktivieren. Um diese nachteilige Begleiterscheinung zu minimieren, kann das zur heterogen katalysierten Dehydrierung bei erhöhter Temperatur über die Katalysatoroberfläche zu leitende propanhaltige Reaktionsgas A zweckmäßig mit Wasserdampf verdünnt werden. Dies ist in besonders eleganter Weise beim erfindungsgemäßen Verfahren z. B. in einfacher Weise dadurch möglich, dass man die Abtrennung des Propans aus dem Absorbat in der Trennzone IV durch Strippen mittels Wasserdampf oder mittels eines Wasserdampf enthaltenden Gases (z. B. eines Inertgases) durchführt und das mit Propan beladene Stripgas (den mit Propan beladenen Wasserdampf) als Propankreisgas in die Reaktionszone A rückführt. Durch den Wasserdampf würde sich abscheidender Kohlenstoff unter den ansonsten gegebenen Bedingungen nach dem Prinzip der Kohlevergasung teilweise oder vollständig eliminiert und die Katalysatorstandzeit so verlängert.

Eine andere Möglichkeit, abgeschiedene Kohlenstoffverbindungen zu beseitigen, besteht darin, den Dehydrierkatalysator (sowie einen für die Wasserstoffverbrennung gegebenenfalls separat eingesetzten Katalysator) von Zeit zu Zeit bei erhöhter Temperatur mit einem Sauerstoff enthaltenden Gas (zweckmäßig in Abwesenheit von Kohlenwasserstoffen) zu durchströmen und damit den abgeschiedenen Kohlenstoff quasi abzubrennen. Eine gewisse Unterdrückung der Bildung von Kohlenstoffablagerungen und damit eine Verlängerung der Katalysatorstandzeit ist aber auch dadurch möglich, dass man dem heterogen katalysiert zu dehydrierenden Propan, bevor es bei erhöhter Temperatur über den Dehydrierkatalysator geführt wird, molekularen Wasserstoff zusetzt. Dies ist beim erfindungsgemäßen Verfahren z. B. gegebenenfalls in einfacher Weise dadurch möglich, dass man über eine Trennmembran vor der Trennzone III (oder hinter der Trennzone III) von in der Trennzone II anfallendem Restgas in selbigem gegebenenfalls noch enthaltenen molekularen Wasserstoff abtrennt und in die Reaktionszone A rückführt. Selbstverständlich kann an dieser Stelle aber auch molekularer Wasserstoff aus anderer Quelle eingesetzt werden. Beispielsweise kann der molekulare Wasserstoff auch noch im gegebenenfalls in die Reaktionszone A rückgeführten Restgaskreisgas enthalten sein.

Natürlich besteht auch die Möglichkeit, dem heterogen katalysiert zu dehydrierenden Propan ein Gemisch aus Wasserdampf und molekularem Wasserstoff zuzusetzen.

Ein Zusatz von molekularem Wasserstoff zur heterogen katalysierten Dehydrierung von Propan mindert auch die unerwünschte Bildung von Allen (Propandien), Propin und Acetylen als Nebenprodukten.

Damit können der Reaktionszone A zur Ausbildung eines durch das wenigstens eine Katalysatorbett zu führenden Reaktionsgases A (in dieser Schrift auch Beschickungsgasgemisch der Reaktionszone A oder Reaktionsgas A-Ausgangsgemisch genannt) im einfachsten Fall nur Frischpropan und Propankreisgas zugeführt werden. Letzteres kann dabei bereits die Menge an molekularem Sauerstoff enthalten (z. B. dann, wenn das Propan in der Trennzone IV aus dem Absorbat durch Strippen abgetrennt wird und das Stripgas molekularen Sauerstoff, z. B. in Form von Luft, zugesetzt enthält), die benötigt wird, um in der Reaktionszone A die erfindungsgemäß erforderliche Wasserstoffverbrennung zu bedingen. Das Reaktionsgas A-Ausgangsgemisch kann aber auch nur aus Frischpropan, Propankreisgas und Restgaskreisgas zusammengesetzt sein, wobei letzteres ebenfalls molekularen Sauerstoff enthalten kann.

In vorteilhafter Weise wird das Propankreisgas in den vorgenannten Fällen gerade soviel Wasserdampf enthalten, dass dieser, gemeinsam mit dem im Rahmen der Wasserstoffverbrennung in der Reaktionszone A gebildeten Wasserdampf, seine vorteilhaften Eigenschaften für das Gesamtverfahren entfalten kann. Der Zufuhr weiterer gasförmiger Ströme in die Reaktionszone A bedarf es in den vorbeschriebenen Fällen nicht in notwendiger Weise. Die in der Reaktionszone A erwünschte Umsetzung erfolgt im einfachen Durchgang des Reaktionsgases A durch die selbige.

Selbstverständlich können zur Ausbildung des durch das wenigstens eine Katalysatorfestbett zu führenden Reaktionsgases A zusätzlich zu Frischpropan, Propankreisgas und gegebenenfalls Restgaskreisgas aber auch noch zusätzlicher Wasserdampf und/oder zusätzlicher molekularer Wasserstoff zugeführt werden, um deren in dieser Schrift beschriebene vorteilhafte Wirkung zu entfalten. Das molare Verhältnis von molekularem Wasserstoff zu Propan im Beschickungsgasgemisch der Reaktionszone A ist in der Regel $\leq 5$, meist $\leq 3$, vielfach $\leq 1$ oder $\leq 0,1$.

Das molare Verhältnis von Wasserdampf zu Propan kann im Beschickungsgasgemisch der Reaktionszone A z. B. $\geq 0$ bis 30 betragen. Zweckmäßig wird es 0,05 bis 2 und günstig 0,1 bis 0,5 betragen.

Außerdem kann dem Beschickungsgasgemisch für die Reaktionszone A je nach Bedarf extra molekularer Sauerstoff (in Reinform und/oder als Gemisch mit Inertgas) und/oder extra Inertgas zugeführt werden. Die in der Reaktionszone A erwünschte Umsetzung kann dann wieder im einfachen Durchgang des Reaktionsgases A (des Beschickungsgases der Reaktionszone A) durch die selbige erfolgen, ohne dass längs des Reaktionspfades eine Zufuhr weiterer gasförmiger Ströme erfolgt. Unter dem Reaktionspfad in der Reaktionszone A soll in dieser Schrift der Strömungsweg desjenigen Propans durch die Reaktionszone A in Abhängigkeit vom dehydrierenden Umsatz (der Umsatz in der heterogen katalysierten Dehydrierung) dieses Propans verstanden werden, das dem Reaktionsgas A vor dessen ersten Durchgang durch wenigstens ein Katalysatorbett der Reaktionszone A zugeführt wird.

Eine geeignete Reaktorform für eine solche heterogen katalysierte Propandehydrierung mit einfachem Durchgang des Beschickungsgasgemischs durch die Reaktionszone A und ohne Zwischengaseinspeisung ist z. B. der Festbettrohr- bzw. Rohrbündelreaktor. Dabei befindet sich der Dehydrierkatalysator in einem oder in einem Bündel von Reaktionsrohren als Festbett. Ist die erfindungsgemäß erforderliche Wasserstoffverbrennung in der Reaktionszone A so bemessen, dass die in der Reaktionszone A erfolgende Bruttoreaktion endotherm verläuft, wird man die Reaktionsrohre erfindungsgemäß zweckmäßig von außen beheizen (selbstredend können sie bei Bedarf aber auch gekühlt werden). Dies kann z. B. dadurch erfolgen, dass im die Reaktionsrohre umgebenden Raum ein Gas, z. B. ein Kohlenwasserstoff wie Methan, verbrannt wird. Günstig ist es, diese direkte Form der Kontaktrohrerwärmung lediglich auf den ersten 20 bis 30 % der Festbettschüttung anzuwenden und die verbleibende Schüttungslänge durch die im Rahmen der Verbrennung freigesetzte Strahlungswärme auf die erforderliche Reaktionstemperatur aufzuwärmen. Auf diesem Weg ist eine annähernd isotherme Reaktionsführung erreichbar. Geeignete Reaktionsrohrinnendurchmesser betragen etwa 10 bis 15 cm. Ein typischer Dehydrierrohrbündelreaktor umfasst 300 bis 1000 oder mehr Reaktionsrohre. Die Temperatur im Reaktionsrohrinneren bewegt sich im Bereich von 300 bis 800°C, vorzugsweise im Bereich von 400 bis 700°C. Mit Vorteil wird das Reaktionsgas A-Ausgangsgemisch dem Rohrreaktor auf die Reaktionstemperatur vorerwärmt zugeführt. Es ist möglich, dass das Produktgas(gemisch) A das Reaktionsrohr mit einer 50 bis 100°C tiefergelegenen Temperatur verlässt. Diese Verlasstemperatur kann aber auch höher oder auf gleichem Niveau liegen. Im Rahmen der vorgenannten Verfahrensweise ist die Verwendung von oxidischen Dehydrierkatalysatoren auf der Grundlage von Chrom- und/oder Aluminiumoxid zweckmäßig. Der Dehydrierkatalysator wird meist unverdünnt angewandt. Großtechnisch kann man mehrere solcher Rohrbündelreaktoren parallel betreiben und ihre Produktgase A gemischt zum Beschicken der Reaktionszone B verwenden. Gegebenenfalls können sich auch nur zwei dieser Reaktoren im Dehydrierbetrieb befinden, während im Dritten Reaktor die Katalysatorbeschickung regeneriert wird.

Ein einfacher Durchgang des Beschickungsgases durch die Reaktionszone A kann aber auch in einem Wanderbett- oder Fließbett- oder Wirbelbettreaktor erfolgen, wie es z. B. die DE-A 102 45 585 sowie die in dieser Schrift diesbezüglich zitierte Literatur beschreibt.

Grundsätzlich kann die Reaktionszone A des erfindungsgemäßen Verfahrens auch aus z. B. zwei Abschnitten bestehen. Ein solcher Aufbau der Reaktionszone A ist insbesondere dann empfehlenswert, wenn das Beschickungsgas für die Reaktionszone A keinen molekularen Sauerstoff umfasst.

In diesem Fall kann im ersten Abschnitt die eigentliche heterogen katalysierte Dehydrierung erfolgen und im zweiten Abschnitt, nach einer Zwischenzufuhr von molekularem Sauerstoff und/oder einem Gemisch aus molekularem Sauerstoff und Inertgas, die erfindungsgemäß erforderliche heterogen katalysierte Wasserstoffverbrennung.

Ganz generell wird man beim erfindungsgemäßen Verfahren die Reaktionszone A zweckmäßig so betreiben, dass, bezogen auf einen einmaligen Durchgang durch die Reaktionszone A, $\geq 5$ mol-% bis $\leq 60$ mol-%, bevorzugt $\geq 10$ mol-% bis $\leq 50$ mol-%, besonders bevorzugt $\geq 15$ mol-% bis $\leq 40$ mol-%, und ganz besonders bevorzugt $\geq 20$ mol-% bis $\leq 35$ mol-% des der Reaktionszone A insgesamt zugeführten Propan in der Reaktionszone A dehydrierend umgesetzt werden. Ein solchermaßen beschränkter Umsatz in der Reaktionszone A ist erfindungsgemäß normalerweise deshalb ausreichend, weil die verbliebene Menge an nicht umgesetztem Propan in der nachfolgenden Reaktionszone B im wesentlichen als Verdünnungsgas fungiert und im weiteren Verlauf der erfindungsgemäßen Verfahrensweise weitgehend verlustfrei in die Reaktionszone A rückgeführt werden kann. Der Vorteil einer Verfahrensweise mit niederem Propanumsatz ist, dass bei einmaligem Durchgang des Reaktionsgases A durch die Reaktionszone A die für die endotherme Dehydrierung benötigte Wärmemenge vergleichsweise niedrig ist und zur Umsatzerzielung vergleichsweise niedrige Reaktionstemperaturen ausreichend sind.

Erfindungsgemäß vorteilhaft kann es, wie bereits angesprochen, zweckmäßig sein, die Propandehydrierung in der Reaktionszone A (z. B. mit vergleichsweise geringem Propanumsatz) (quasi) adiabat durchzuführen. Das heißt, man wird das Beschickungsgasgemisch für die Reaktionszone A in der Regel zunächst auf eine Temperatur von 500 bis 700°C (beziehungsweise von 550 bis 650°C) erhitzen (zum Beispiel durch Direktbefeuerung der es umgebenden Wandung). Im Normalfall wird dann ein einziger adiabater Durchgang durch ein Katalysatorbett ausreichend sein, um sowohl den gewünschten dehydrierenden Umsatz als auch die erfindungsgemäß erforderliche Wasserstoffverbrennung zu erzielen, wobei sich das Reaktionsgas, je nach mengenmäßigem Verhältnis von endothermer Dehydrierung und exothermer Wasserstoffverbrennung in der Bruttobetrachtung erwärmen, abkühlen oder thermisch neutral verhalten wird. Erfindungsgemäß bevorzugt ist eine adiabate Betriebsweise, bei der sich das Reaktionsgas beim einfachen Durchgang um etwa 30 bis 200°C abkühlt. Bei Bedarf kann in einem zweiten Abschnitt der Reaktionszone A im Rahmen der Dehydrierung gebildeter Wasserstoff mit zwischeneingespeistem molekularem Sauerstoff heterogen katalysiert nachverbrannt werden. Diese Verbrennung kann gleichfalls adiabat durchgeführt werden.

Bemerkenswerterweise ist dabei insbesondere im adiabaten Betrieb ein einzelner Schachtofenreaktor als Festbettreaktor ausreichend, der vom Reaktionsgas A axial und/oder radial durchströmt wird.

Im einfachsten Fall handelt es sich dabei um ein einziges geschlossenes Reaktionsvolumen, zum Beispiel einen Behälter, dessen Innendurchmesser 0,1 bis 10 m, eventuell auch 0,5 bis 5 m beträgt und in welchem das Katalysatorfestbett auf einer Trägervorrichtung (zum Beispiel ein Gitterrost) aufgebracht ist. Das mit Katalysator beschickte Reaktionsvolumen, das im adiabaten Betrieb im wesentlichen wärmeisoliert ist, wird dabei mit dem heißen, Propan enthaltenden, Reaktionsgas A axial durchströmt. Die Katalysatorgeometrie kann dabei sowohl kugelförmig als auch ringförmig oder strangförmig sein. Da in diesem Fall das Reaktionsvolumen durch einen sehr kostengünstigen Apparat zu realisieren ist, sind alle Katalysatorgeometrien, die einen besonders niedrigen Druckverlust aufweisen, zu bevorzugen. Das sind vor allem Katalysatorgeometrien, die zu einem großen Hohlraumvolumen führen oder strukturiert aufgebaut sind, wie zum Beispiel Monolithe bzw. Wabenkörper. Zur Verwirklichung einer radialen Strömung des Propan enthaltenden Reaktionsgases A kann der Reaktor zum Beispiel aus zwei in einer Mantelhülle befindlichen, konzentrisch ineinander gestellten, zylindrischen Gitterrosten bestehen und die Katalysatorschüttung in deren Ringspalt angeordnet sein. Im adiabaten Fall wäre die Metallhülle gegebenenfalls wiederum thermisch isoliert.

Als erfindungsgemäße Katalysatorbeschickung für eine heterogen katalysierte Propandehydrierung eignen sich insbesondere auch die in der DE-A 199 37 107 offenbarten, vor allem alle beispielhaft offenbarten, Katalysatoren sowie deren Gemische mit bezüglich der heterogen katalysierten Dehydrierung inerten geometrischen Formkörpern.

Nach längerer Betriebsdauer sind vorgenannte Katalysatoren zum Beispiel in einfacher Weise dadurch regenerierbar, dass man bei einer Eintrittstemperatur von 300 bis 600°C, häufig bei 400 bis 550°C, zunächst in ersten Regenerierungsstufen mit Stickstoff und/oder Wasserdampf (bevorzugt) verdünnte Luft über das Katalysatorbett leitet. Die Katalysator(bett)belastung mit Regeneriergas (z. B. Luft) kann dabei z. B. 50 bis 10000 $h^{-1}$ und der Sauerstoffgehalt des Regeneriergases 0,1 oder 0,5 bis 20 Vol.-% betragen.

In nachfolgenden weiteren Regenerierungsstufen kann unter ansonsten gleichen Regenerierbedingungen als Regeneriergas Luft verwendet werden. Anwendungstechnisch zweckmäßig empfiehlt es sich, den Katalysator vor seiner Regenerierung mit Inertgas (zum Beispiel $N_2$) zu spülen.

Anschließend ist es in der Regel empfehlenswert, noch mit reinem molekularem Wasserstoff oder mit durch Inertgas (vorzugsweise Wasserdampf und/oder Stickstoff) verdünntem molekularem Wasserstoff (der Wasserstoffgehalt sollte $\geq$ 1 Vol.-% betragen) im ansonsten gleichen Bedingungsraster zu regenerieren.

Die heterogen katalysierte Propandehydrierung in der Reaktionszone A des erfindungsgemäßen Verfahrens kann mit vergleichsweise geringem Propanumsatz ($\leq$ 30 mol-%) in allen Fällen bei den gleichen Katalysator(bett)belastungen (sowohl das Reaktionsgas insgesamt, als auch das in selbigem enthaltene Propan betreffend) betrieben werden, wie die Varianten mit hohem Propanumsatz (> 30 mol-%). Diese Belastung mit Reaktionsgas A kann zum Beispiel 100 bis 40000 oder bis 10000 $h^{-1}$, häufig 300 bis 7000 $h^{-1}$, das heißt vielfach etwa 500 bis 4000 $h^{-1}$ betragen. Vorgenannte Belastungen sind auch auf in der Reaktionszone A gegebenenfalls spezifisch für die Wasserstoffverbrennung eingesetzte Katalysatorbetten anwendbar.

In besonders eleganter Weise lässt sich die heterogen katalysierte Propandehydrierung in der Reaktionszone A (insbesondere bei auf einmaligen Durchgang bezogenen Propanumsätzen von 15 bis 35 mol-%) in einem Hordenreaktor verwirklichen, weshalb die Reaktionszone A bevorzugt wenigstens einen solchen umfasst.

Dieser enthält räumlich aufeinanderfolgend mehr als ein die Dehydrierung katalysierendes Katalysatorbett. Die Katalysatorbettanzahl kann 1 bis 20, zweckmäßig 2 bis 8, aber auch 3 bis 6 betragen. Mit zunehmender Hordenzahl lassen sich zunehmend leichter erhöhte Propanumsätze erreichen. Die Katalysatorbetten sind vorzugsweise radial oder axial hintereinander angeordnet. Anwendungstechnisch zweckmäßig wird in einem solchen Hordenreaktor der Katalysatorfestbetttyp angewendet.

Im einfachsten Fall sind die Katalysatorfestbetten in einem Schachtofenreaktor axial oder in den Ringspalten von zentrisch ineinandergestellten zylindrischen Gitterrosten angeordnet. Es ist jedoch auch möglich, die Ringspalte in Segmenten übereinander anzuordnen und das Gas nach radialem Durchtritt in einem Segment in das nächste darüber- oder darunterliegende Segment zu führen.

In zweckmäßiger Weise wird das Reaktionsgas A auf seinem Weg von einem Katalysatorbett zum nächsten Katalysatorbett, zum Beispiel durch Überleiten über mit heißen Gasen erhitzte Wärmetauscheroberflächen (z. B. Rippen) oder durch Leiten durch mit heißen Brenngasen erhitzte Rohre, im Hordenreaktor einer Zwischenerhitzung unterworfen (bei Bedarf kann in entsprechender Weise auch eine Zwischenkühlung erfolgen).

Wird der Hordenreaktor im übrigen adiabat betrieben, ist es insbesondere für Propanumsätze $\leq$ 30 mol-%, vor allem bei Verwendung der in der DE-A 199 37 107 beschriebenen Katalysatoren, insbesondere der beispielhaften Ausführungsformen, ausreichend, das Reaktionsgasgemisch auf eine Temperatur von 450 bis 550°C (vorzugsweise 450 bis 500°C) vorerhitzt in den Dehydrierreaktor zu führen und innerhalb des Hordenreaktors in diesem Temperaturbereich zu halten. Das heißt, die gesamte Propandehydrierung ist so bei äußerst niederen Temperaturen zu verwirklichen, was sich für die Standzeit der Katalysatorfestbetten zwischen zwei Regenerierungen als besonders günstig erweist. Für höhere Propanumsätze wird das Reaktionsgasgemisch zweckmäßig auf höhere Temperaturen vorerhitzt in den Dehydrierreaktor geführt (diese können bis zu 700°C betragen) und innerhalb des Hordenreaktors in diesem erhöhten Temperaturbereich gehalten.

Noch geschickter ist es, die vorstehend geschilderte Zwischenerhitzung auf direktem Weg durchzuführen (ermöglicht autotherme Fahrweise). Dazu wird dem Reaktionsgas A entweder bereits vor (z. B. als Bestandteil von Restgaskreisgas und/oder von Propankreisgas) Durchströmung des ersten Katalysatorbettes (dann sollte das Reaktionsgas A-Ausgangsgemisch vorteilhaft molekularen Wasserstoff zugesetzt enthalten) und/oder zwischen den nachfolgenden Katalysatorbetten in begrenztem Umfang molekularer Sauerstoff zugesetzt. So kann (in der Regel durch die Dehydrierkatalysatoren selbst katalysiert) die erfindungsgemäß erforderliche Verbrennung von im Reaktionsgas A enthaltenem, im Verlauf der heterogen katalysierten Propandehydrierung gebildetem und/oder dem Reaktionsgas A zugesetztem molekularem Wasserstoff auf besonders gezielte und kontrollierte Art und Weise bewirkt werden (es kann auch anwendungstechnisch zweckmäßig sein, im Hordenreaktor Katalysatorbetten einzufügen, die mit Katalysator beschickt sind, der besonders spezifisch (selektiv) die Verbrennung von Wasserstoff katalysiert (als solche Katalysatoren kommen zum Beispiel jene der Schriften US-A 4 788 371, US-A 4 886 928, US-A 5 430 209, US-A 5 530 171, US-A 5 527 979 und US-A 5 563 314 in Betracht; beispielsweise können solche Katalysatorbetten in alternierender Weise zu den Dehydrierkatalysator enthaltenden Betten im Hordenreaktor untergebracht sein; diese Katalysatoren eignen sich auch für die bereits beschriebene Wasserstoffverbrennung in einem zweiten Abschnitt der Reaktionszone A). Die dabei freigesetzte Reaktionswärme ermöglicht so in Abhängigkeit von der verbrannten Menge an molekularem Wasserstoff eine insgesamt exotherme, oder eine insgesamt autotherme (die Bruttowärmetönung ist im wesentlichen Null), oder eine insgesamt endotherme Betriebsweise der heterogen katalysierten Propandehydrierung. Mit zunehmender gewählter Verweilzeit des Reaktionsgases im Katalysatorbett ist so eine Propandehydrierung bei abnehmender oder im wesentlichen konstanter Temperatur möglich, was besonders lange Standzeiten zwischen zwei Regenerierungen ermöglicht und erfindungsgemäß bevorzugt ist.

Insbesondere dann, wenn man sowohl im Verlauf der heterogen katalysierten Dehydrierung des Propans (Phase 1) als auch im Anschluss an die heterogen katalysierte Dehydrierung des Propans (Phase 2) molekularen Wasserstoff verbrennt, ist es erfindungsgemäß zweckmäßig, das nach Abschluss der heterogen katalysierten Dehydrierung vorliegende Reaktionsgas der Reaktionszone A in der Menge, in der es der abschließenden Wasserstoffverbrennung in der Reaktionszone A zugeführt wird (eine Teilmenge kann als Dehydrierkreisgas in die Reaktionszone A als einer der Propan enthaltenden Zufuhrströme rückgeführt werden), zunächst durch indirekten Wärmeaustausch mit den von Propankreisgas und Dehydrierkreisgas verschiedenen Bestandteilen des Reaktionsgas A-Beschickungsgemischs abzukühlen und nach der abschließenden Verbrennung von molekularem Wasserstoff in der Reaktionszone A das Produktgas A ebenfalls durch indirekten Wärmeaustausch, jedoch mit Propankreisgas, abzukühlen, bevor es weiterbehandelt wird. Dies gilt insbesondere dann, wenn die Reaktionszone A insgesamt exotherm gestaltet wird.

Generell sollte erfindungsgemäß eine wie vorstehend beschriebene Sauerstoffeinspeisung so vorgenommen werden, dass der Sauerstoffgehalt des Reaktionsgases A, bezogen auf die darin enthaltene Menge an molekularem Wasserstoff, 0,5 bis 50, bzw. bis 40, bzw. bis 30, vorzugsweise 10 bis 25 Vol.-% beträgt. Als Sauerstoffquelle kommen dabei, wie bereits früher ausgeführt, reiner molekularer Sauerstoff (erfindungsgemäß bevorzugt) oder mit Inertgas, zum Beispiel CO, $CO_2$, $N_2$ und/oder Edelgase, verdünnter molekularer Sauerstoff, insbesondere aber auch Luft in Betracht. Erfindungsgemäß bevorzugt wird der molekulare Sauerstoff als Gas zugeführt, das nicht mehr als 30 Vol.-%, vorzugsweise nicht mehr als 25 Vol.-%, vorteilhaft nicht mehr als 20 Vol.-%, besonders vorteilhaft nicht mehr als 15 Vol.-%, besser nicht mehr als 10 Vol.-% und besonders bevorzugt nicht mehr als 5 Vol.-% anderer (von molekularem Sauerstoff verschiedener) Gase enthält. Besonders vorteilhaft erfolgt die beschriebene Sauerstoffeinspeisung in reiner Form.

Da die Verbrennung von 1 mol molekularem Wasserstoff zu $H_2O$ etwa doppelt soviel (ca. 240 kJ/mol) Energie liefert, wie die Dehydrierung von 1 mol Propan zu Propylen und $H_2$ verbraucht (ca. 120 kJ/mol), ist eine wie beschrieben autotherme Gestaltung der Reaktionszone A im adiabaten Hordenreaktor mit der ins Auge gefassten Vorteilhaftigkeit der erfindungsgemäßen Verfahrensweise gut vereinbar, erfordert sie doch gerade die Verbrennung einer Wasserstoffmenge von etwa 50 mol-% der im Rahmen der Dehydrierung in der Reaktionszone A gebildeten molekularen Wasserstoffmenge.

Die Vorteilhaftigkeit des erfindungsgemäßen Verfahrens kommt jedoch nicht nur dann zum Tragen, wenn man in der Reaktionszone A eine Wasserstoffmenge von etwa 50 mol-% der in der Reaktionszone A gebildeten molekularen Wasserstoffmenge verbrennt. Diese Vorteilhaftigkeit kommt vielmehr auch dann bereits zur Geltung, wenn man in der Reaktionszone A eine Wasserstoffmenge von 25 bis 95 mol-%, bzw. bis 100 mol-%, oder von 30 bis 90 mol-%, oder 35 bis 85 mol-%, oder 40 bis 80 mol-%, oder 45 bis 75 mol-%, oder 50 bis 70 mol-%, oder 35 bis 65 mol-%, oder 40 bis 60 mol-%, oder 45 bis 55 mol-% der in der Reaktionszone A gebildeten molekularen Wasserstoffmenge zu Wasser verbrennt (vorzugsweise in der vorbeschriebenen Betriebsweise eines adiabaten Hordenreaktors).

In der Regel sollte eine wie vorstehend beschriebene Sauerstoffeinspeisung so vorgenommen werden, dass der Sauerstoffgehalt des Reaktionsgases A, bezogen auf die darin enthaltene Menge an Propan und Propylen 0,01 bzw. 0,5 bis 3 Vol.-% beträgt.

Die Isothermie der heterogen katalysierten Propandehydrierung lässt sich dadurch weiter verbessern, dass man im Hordenreaktor in den Räumen zwischen den Katalysatorbetten geschlossene, vor ihrer Füllung günstigerweise aber nicht notwendigerweise evakuierte Einbauten (z. B. rohrförmig) anbringt. Derartige Einbauten können auch ins jeweilige Katalysatorbett gestellt werden. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Eine Möglichkeit, das Beschickungsgasgemisch für die heterogen katalysierte Propandehydrierung in der Reaktionszone A auf die benötigte Reaktionstemperatur zu erwärmen, besteht auch darin, einen Teil des darin enthaltenen

Propans und/oder $H_2$ mittels im Beschickungsgasgemisch enthaltenem molekularem Sauerstoff beim Eintritt in die Reaktionszone A zu verbrennen (zum Beispiel an geeigneten spezifisch wirkenden Verbrennungskatalysatoren, zum Beispiel durch einfaches Überleiten und/oder Durchleiten) und mittels der so freigesetzten Verbrennungswärme die Erwärmung auf die für die Dehydrierung gewünschte Reaktionstemperatur zu bewirken (eine solche Verfahrensweise ist (wie bereits erwähnt) insbesondere in einem Wirbelbettreaktor vorteilhaft).

Erfindungsgemäß bevorzugt wird man die Reaktionszone A adiabat (nach außen wärmeisoliert) gestalten.

Dem Vorgenannten entsprechend, kann man die Reaktionszone A des erfindungsgemäßen Verfahrens wie in den Schriften DE-A 10 2004 032 129 und DE-A 10 2005 013 39 beschrieben gestalten, jedoch mit dem Unterschied, dass als Beschickungsgasgemisch der Reaktionszone A ein Gemisch aus Frischpropan, Wasserdampf enthaltendem Propankreisgas und (gegebenenfalls molekularem Sauerstoff enthaltendem) Restgaskreisgas verwendet wird. Dabei ist die Reaktionszone A als (vorzugsweise adiabater) Hordenreaktor verwirklicht, in welchem Katalysatorbetten (vorzugsweise Festbetten) radial oder axial hintereinander angeordnet sind. Mit Vorteil beträgt die Anzahl der Katalysatorbetthorden in einem solchen Hordenreaktor drei. Bevorzugt wird die heterogen katalysierte partielle Propandehydrierung dabei autotherm durchgeführt. Dazu wird dem Beschickungsgasgemisch der Reaktionszone A hinter dem ersten durchlaufenen Katalysator(fest)bett und zwischen den auf das in Strömungsrichtung erste Katalysator(fest)bett nachfolgenden Katalysator(fest)betten in begrenztem Umfang molekularer Sauerstoff oder ein solchen enthaltendes Gemisch mit Inertgas zugesetzt (z. B. so wie im Deutschen Aktenzeichen Nr. 10 2006 017623.5 beschrieben). So wird, in der Regel durch die Dehydrierkatalysatoren selbst katalysiert, eine begrenzte Verbrennung von im Verlauf der heterogen katalysierten Propandehydrierung gebildetem Wasserstoff (sowie gegebenenfalls in höchstens geringem Umfang von Propan und/oder Propylen) bewirkt werden, deren exotherme Wärmetönung die Dehydriertemperatur im wesentlichen erhält.

Zweckmäßig wird die partielle heterogen katalysierte Dehydrierung des Propans dabei im wesentlichen auf die drei Katalysatorhorden verteilt so betrieben, dass der Umsatz des in den Reaktor geführten Propans, bezogen auf einmaligen Reaktordurchgang, ca. 20 mol.-% beträgt (selbstredend kann er beim erfindungsgemäßen Verfahren aber auch 30 mol.-%, oder 40 mol.-%, oder 50 mol.-% oder mehr betragen). Die erzielte Selektivität der Propylenbildung liegt dabei regelmäßig bei 90 mol.-%. Der maximale Umsatzbeitrag einer einzelnen Horde wandert mit zunehmender Betriebsdauer in Strömungsrichtung von vorne nach hinten. In der Regel wird die Katalysatorbeschickung regeneriert, bevor die in Strömungsrichtung dritte Horde den maximalen Umsatzbeitrag erbringt. Mit Vorteil erfolgt die Regenerierung dann, wenn die Verkokung aller Horden ein identisches Ausmaß erreicht hat.

Zum Abschluss kann in einem der Dehydrierung nachgeschalteten Katalysator(fest)bett, nach zuvor erfolgter Sauerstoffzwischeneinspeisung, noch im wesentlichen alleinige Verbrennung von molekularem Wasserstoff erfolgen. Diese kann grundsätzlich soweit gehen, dass das die Reaktionszone A dann verlassende Produktgas A im wesentlichen keinen molekularen Wasserstoff mehr enthält.

Günstig ist es für die vorbeschriebene heterogen katalysierte partielle Dehydrierung des Propan ganz generell, wenn die Belastung der Katalysatorgesamtmenge (Summe über alle Betten) mit der Gesamtmenge aus Propan und Propylen $\geq$ 500 Nl/l·h und $\leq$ 20000 Nl/l·h beträgt (typische Werte sind 1500 Nl/l·h bis 2500 Nl/l·h). Die maximale Reaktionstemperatur innerhalb eines individuellen Katalysatorfestbetts wird dabei mit Vorteil bei 500°C bis 600°C (bzw. bis 650°C) gehalten.

Nachteilig am vorstehend beschriebenen Verfahren mit einem bereits molekularen Sauerstoff enthaltenden Reaktionsgas A-Beschickungsgemisch ist, dass nahezu alle Katalysatoren, die die Dehydrierung des Propans katalysieren, auch die Verbrennung (vollständige Oxidation von Propan und Propylen zu Kohlenoxiden und Wasserdampf) von Propan und Propylen mit molekularem Sauerstoff katalysieren.

Dem kann man neben der Maßnahme einer wie bereits beschriebenen Zugabe eines molekularen Wasserstoff enthaltenden Gases ins Reaktionsgas A-Beschickungsgasgemisch gemäß der DE-A 102 11 275 auch dadurch begegnen, dass man das der Dehydrierzone (auf die in der Reaktionszone A noch eine Zone zur Verbrennung von molekularem Wasserstoff folgen kann) entnommene Produktgas in zwei Teilmengen identischer Zusammensetzung aufteilt, um nur eine der beiden Teilmengen (gegebenenfalls nach zuvor erfolgter teilweiser oder vollständiger Verbrennung von darin enthaltenem molekularem Wasserstoff) als Produktgas A bzw. Produktgas A* der Partialoxidation zuzuführen, während die andere Teilmenge als Bestandteil des Reaktionsgases A (in der Regel des Reaktionsgas A-Beschickungsgasgemischs) in die Dehydrierung rückgeführt wird. Der in diesem aus der Dehydrierung selbst kommenden Dehydrierkreisgas enthaltene molekulare Wasserstoff, soll dann das im Beschickungsgasgemisch für die Reaktionszone A enthaltene Propan und gegebenenfalls Propylen vor dem in selbigem gleichfalls enthaltenen molekularen Sauerstoff schützen. Dieser Schutz fußt darauf, dass, wie bereits gesagt, die normalerweise durch die selben Katalysatoren heterogen katalysierte Verbrennung von molekularem Wasserstoff zu Wasser gegenüber der Vollbrennung von Propan und/oder Propylen kinetisch bevorzugt ist.

Der Lehre der DE-A 102 11 275 folgend wird man die Dehydrierkreisgasführung zweckmäßig nach dem Strahlpumpenprinzip realisieren (sie wird auch als Schlaufenfahrweise bezeichnet; als Treibstrahl kann Propankreisgas fungieren). Ferner wird in vorgenannter Schrift die Möglichkeit angesprochen, dem Beschickungsgasgemisch für die Reaktionszone A als weiteren Oxidationsschutz zusätzlich molekularen Wasserstoff zuzusetzen (dies kann z. B. auch über eine Trennmembran aus aus der Trennzone II stammendem, noch molekularen Wasserstoff enthaltendem, Restgas abgetrennter molekularer Wasserstoff sein).

Typische Reaktionsgas A-Beschickungsgasgemische (sich in der Reaktionszone A ausbildende Reaktionsgase A) weisen bei erfindungsgemäßen Verfahren folgende Gehalte auf:

| | |
|---|---|
| 55 bis 80 Vol.-% | Propan, |
| 0,1 bis 20 Vol.-% | Propylen, |
| 0 bis 10 Vol.-% | $H_2$, |
| 0 bis 5 Vol.-% | $O_2$, |
| 0 bis 20 Vol.-% | $N_2$ und |
| 5 bis 15 Vol.-% | $H_2O$. |

Für das erfindungsgemäße Verfahren bevorzugte Reaktionsgas A-Beschickungsgasgemische (das Gasgemisch, das in das in Strömungsrichtung des Reaktionsgases A in der Reaktionszone A befindliche erste Katalysatorfestbett eintritt) weisen folgende Gehalte auf:

| | |
|---|---|
| 55 bis 80 Vol.-% | Propan, |
| 0,1 bis 20 Vol.-% | Propylen, |
| 2 bis 10 Vol.-% | $H_2$, |
| 1 bis 5 Vol.-% | $O_2$, |
| 0 bis 20 Vol.-% | $N_2$ und |
| 5 bis 15 Vol.-% | $H_2O$. |

Der Anteil der von den aufgelisteten Bestandteilen verschiedenen Bestandteile der Reaktionsgas A-Beschickungsgasgemische beträgt in der Regel insgesamt $\leq$ 10 Vol.-% und vorzugsweise $\leq$ 6 Vol.-%.
Typische Produktgase A weisen folgende Gehalte auf:

| | |
|---|---|
| 30 bis 50 Vol.-% | Propan, |
| 15 bis 30 Vol.-% | Propylen, |
| 0 bis 10 Vol.-% | $H_2$, vorzugsweise 0 bis 6 Vol.-$H_2$, |
| 10 bis 25 Vol.-% | $H_2O$, |
| 0 bis 1 Vol.-% | $O_2$ und |
| 0 bis 35 Vol.-% | $N_2$. |

Der Anteil der von den aufgelisteten Bestandteilen verschiedenen Bestandteile der Produktgase A beträgt in der Regel $\leq$ 10 Vol.-%.
Die Temperatur des der Reaktionszone A entnommenen Produktgases A beträgt typisch 300 bis 800 °C, vorzugsweise 400 bis 700 °C und besonders bevorzugt 450 bis 650 °C.

Der Druck des die Reaktionszone A verlassenden Produktgases A beträgt in der Regel > 1 bis 5 bar, bevorzugt 1,5 bis 4 bar und vorteilhaft 2 bis 3 bar.

Erfindungsgemäß wird nun Produktgas A entweder ohne weitere Nebenkomponentenabtrennung (vorzugsweise jedoch nach mechanischer Abtrennung von darin enthaltenen Feststoffpartikeln gemäß der Lehre der DE-A 10 316 039) oder nach kondensativer (durch direkte und/oder indirekter Abkühlung bewirkte) Abtrennung einer Teil- oder der Gesamtmenge des im Produktgas A enthaltenen Wasserdampf (d. h., als Produktgas A*) zur Beschickung des wenigstens einen Oxidationsreaktors in der Reaktionszone B mit Reaktionsgas B verwendet.

Unabhängig davon, ob eine kondensative Abtrennung von Wasser aus dem Produktgas A vorgenommen wird, ist es erfindungsgemäß zweckmäßig, das heiße Produktgas A vorab seiner Weiterverwendung in der Reaktionszone B wenigstens durch indirekten Wärmeaustausch mit Propankreisgas (sowie gegebenenfalls anderen Bestandteilen des Reaktionsgas A-Beschickungsgasgemischs (Dehydrierkreisgas normalerweise ausgenommen) abzukühlen und Propankreisgas (sowie gegebenenfalls die anderen Bestandteile des Reaktionsgas A-Beschickungsgasgemischs) so zu erwärmen.

Im übrigen ist es zur Verwendung von Produktgas A bzw. von Produktgas A* zur Beschickung des wenigstens einen Oxidationsreaktors in der Reaktionszone B ausreichend, dem Produktgas A bzw. dem Produktgas A* diejenige Menge an molekularem Sauerstoff zuzusetzten, die für die Erlangung der Zielsetzung in der Reaktionszone B erforderlich ist.

Dieser Zusatz kann, wie bereits gesagt, grundsätzlich als Reinsauerstoff oder als ein Gemisch (z. B. Luft) aus molekularem Sauerstoff und einem oder mehreren sich in der Reaktionszone B chemisch inert verhaltenden Gasen (z. B. $N_2$, $H_2O$, Edelgase, $CO_2$) erfolgen (in dieser Schrift auch als Primärsauerstoffquellen bezeichnet). Erfindungsgemäß bevorzugt erfolgt sie als Gas, das nicht mehr als 30 Vol.-%, vorzugsweise nicht mehr als 25 Vol.-%, vorteilhaft nicht mehr als 20 Vol.-%, besonders vorteilhaft nicht mehr als 15 Vol.-%, besser nicht mehr als 10 Vol.-% und besonderes bevorzugt nicht mehr als 5 Vol-% bzw. nicht mehr als 2 Vol.-% anderer, von molekularem Sauerstoff verschiedener Gase enthält. Ganz besonders vorteilhaft ist an dieser Stelle die Verwendung von Reinsauerstoff.

Normalerweise wird die zugeführte Menge an molekularem Sauerstoff so bemessen, dass im Beschickungsgas für die Reaktionszone B (Reaktionsgas B-Ausgangsgemisch) das molare Verhältnis von enthaltenem molekularem Sauerstoff zu enthaltenem Propylen $\geq 1$ und $\leq 3$ beträgt. Vor der Zuführung des molekularen Sauerstoff enthaltenden Gases zum Produktgas A bzw. Produktgas A* wurde deren Temperatur vorteilhaft auf einen Wert von 250 bis 370 °C, besonders vorteilhaft von 270 bis 320 °C eingestellt. Das den molekularen Sauerstoff enthaltende Gas befindet sich bei der Zufuhr zum Reaktionsgas A bzw. Reaktionsgas A* auf einen Druck komprimiert, der sich normalerweise oberhalb des Druckes des Reaktionsgases A bzw. A* (gegebenenfalls bis zu 1 bar) befindet, so dass das den molekularen Sauerstoff enthaltende Gas in einfacher Weise in das Reaktionsgas A bzw. A* eingedrosselt werden kann (wird als Sauerstoffquelle Luft verwendet, wird zur Verdichtung derselben normalerweise ein Radialverdichter eingesetzt, wie ihn z. B. die DE-A 103 53 014 beschreibt). Die Temperatur des den molekularen Sauerstoff enthaltenden Gases ist dabei normalerweise so bemessen, dass sich die gewünschte Temperatur (häufig 240 bis 300 °C) für das Reaktionsgas B-Beschickungsgemisch einstellt. In der Regel beträgt diese Temperatur des molekularen Sauerstoff enthaltenden Gases 100 bis 200 °C.

Typische Reaktionsgas B-Beschickungsgasgemische enthalten:

| | |
|---|---|
| 10 bis 40 Vol.-% | Propan, |
| 5 bis 25 Vol.-% | Propylen, |
| 0 bis 10 Vol.-% | $H_2$, |
| 10 bis 30 Vol.-% | $O_2$, und |
| 1 bis 10 Vol.-% | $H_2O$. |

Bevorzugte Reaktionsgas B-Beschickungsgasgemische enthalten:

| | |
|---|---|
| 15 bis 40 Vol.-% | Propan, |
| 7 bis 20 Vol.-% | Propylen, |
| 0 bis 6 Vol.-% | $H_2$, |
| 15 bis 30 Vol.-% | $O_2$, und |
| 1 bis 7 Vol.-% | $H_2O$. |

Der Eingangsdruck des Reaktionsgases B in den wenigstens einen Oxidationsreaktor der Reaktionszone B wird beim erfindungsgemäßen Verfahren normalerweise zweckmäßig > 1 bar bis 4 bar, meist 1,3 bar bis 3 bar, vielfach 1,5 bis 2,5 bar betragen.

In an sich bekannter Weise läuft die heterogen katalysierte Gasphasen-Partialoxidation von Propylen zu Acrylsäure mit molekularem Sauerstoff grundsätzlich in zwei längs der Reaktionskoordinate aufeinanderfolgenden Schritten ab, von denen der erste zum Acrolein und der zweite von Acrolein zu Acrylsäure führt.

Dieser Reaktionsablauf in zwei zeitlich aufeinanderfolgenden Schritten eröffnet in an sich bekannter Weise die Möglichkeit, das erfindungsgemäße Verfahren in der Reaktionszone B auf der Stufe des Acrolein (der Stufe überwiegender Acroleinbildung) abzubrechen und die Zielproduktabtrennung auf dieser Stufe vorzunehmen, oder das erfindungsgemäße Verfahren bis zur überwiegenden Acrylsäurebildung fortzuführen und die Zielproduktabtrennung erst dann vorzunehmen.

Führt man das erfindungsgemäße Verfahren bis zur überwiegenden Acrylsäurebildung, ist es erfindungsgemäß vorteilhaft, das Verfahren zweistufig, d. h. in zwei hintereinander angeordneten Oxidationsstufen auszuführen, wobei zweckmäßigerweise in jeder der beiden Oxidationsstufen das zu verwendende Katalysatorfestbett und vorzugsweise auch die sonstigen Reaktionsbedingungen, wie z. B. die Temperatur des Katalysatorfestbetts, in optimierender Weise angepasst werden.

Zwar vermögen die für die Katalysatoren der ersten Oxidationsstufe (Propylen → Acrolein) als Aktivmassen besonders geeigneten, die Elemente Mo, Fe, Bi enthaltenden Multimetalloxide in gewissem Umfang auch die zweite Oxidationsstufe (Acrolein → Acrylsäure) zu katalysieren, doch werden für die zweite Oxidationsstufe normalerweise Katalysatoren bevorzugt, deren Aktivmasse wenigstens ein die Elemente Mo und V enthaltendes Multimetalloxid ist.

Damit eignet sich das erfindungsgemäß in der Reaktionszone B durchzuführende Verfahren der heterogen katalysierten Partialoxidation von Propylen an Katalysatorfestbetten, deren Katalysatoren als Aktivmasse wenigstens ein die Elemente Mo, Fe und Bi enthaltendes Multimetalloxid aufweisen, insbesondere als einstufiges Verfahren zur Herstellung von Acrolein (sowie gegebenenfalls Acrylsäure) oder als erste Reaktionsstufe zur zweistufigen Herstellung von Acrylsäure.

Die Realisierung der einstufigen heterogen katalysierten Partialoxidation von Propylen zu Acrolein sowie gegebenenfalls Acrylsäure bzw. der zweistufigen heterogen katalysierten Partialoxidation von Propylen zu Acrylsäure unter Verwendung eines erfindungsgemäß erzeugten Reaktionsgases B kann dabei im einzelnen wie in den Schriften EP-A 70 07 14 (erste Reaktionsstufe; wie dort beschrieben, aber auch in entsprechender Gegenstromfahrweise von Salzbad und Reaktionsgasausgangsgemisch über den Rohrbündelreaktor), EP-A 70 08 93 (zweite Reaktionsstufe; wie dort beschrieben, aber auch in entsprechender Gegenstromfahrweise), WO 04/085369 (als zweistufiges Verfahren), WO 04/85363, DE-A 103 13 212 (erste Reaktionsstufe), EP-A 1 159 248 (als zweistufiges Verfahren), EP-A 1 159 246 (zweite Reaktionsstufe), EP-A 1 159 247 (als zweistufiges Verfahren), DE-A 199 48 248 (als zweistufiges Verfahren), DE-A 101 01 695 (einstufig oder zweistufig), WO 04/085368 (als zweistufiges Verfahren), DE 10 2004 021 764 (zweistufig), WO 04/085362 (erste Reaktionsstufe), WO 04/085370 (zweite Reaktionsstufe), WO 04/085365 (zweite Reaktionsstufe), WO 04/085367 (zweistufig), EP-A 990 636, EP-A 1 007 007 und EP-A 1 106 598 beschrieben durchgeführt werden.

Dies gilt insbesondere für alle in diesen Schriften enthaltenen Ausführungsbeispiele. Sie können wie in diesen Schriften beschrieben durchgeführt werden, jedoch mit dem Unterschied, dass als Reaktionsgasausgangsgemisch für die erste Reaktionsstufe (Propylen zu Acrolein) ein erfindungsgemäß erzeugtes Reaktionsgas B eingesetzt wird. Die übrigen Parameter betreffend wird wie in den Ausführungsbeispielen der genannten Schriften verfahren (insbesondere die Katalysatorfestbetten und Reaktanden belastung der Katalysatorfestbetten betreffend). Wird in den vorgenannten Ausführungsbeispielen des Standes der Technik zweistufig verfahren und erfolgt zwischen den beiden Reaktionsstufen eine Sekundärsauerstoff(erfindungsgemäß weniger bevorzugt Sekundärluft)einspeisung, so wird diese in entsprechender Weise vorgenommen, in ihrer Menge jedoch dahingehend angepasst, dass das molare Verhältnis von molekularem Sauerstoff zu Acrolein im Beschickungsgasgemisch der zweiten Reaktionsstufe demjenigen in den Ausführungsbeispielen der genannten Schriften entspricht.

Erfindungsgemäß vorteilhaft werden die Sauerstoffmengen in der Reaktionszone B so bemessen, dass das Produktgas B noch nicht umgesetzten molekularen Sauerstoff enthält (zweckmäßig $\geq$ 0,5 bis 6 Vol.-%, vorteilhaft 1 bis 5 Vol.-%, bevorzugt 2 bis 4 Vol.-%). Im Fall einer zweistufigen Verfahrensweise gilt das Vorgenannte für jede der beiden Oxidationsstufen.

Für die jeweilige Oxidationsstufe besonders geeignete Multimetalloxidkatalysatoren sind vielfach vorbeschrieben und dem Fachmann wohlbekannt. Beispielsweise verweist die EP-A 253 409 auf Seite 5 auf entsprechende US-Patente.

Günstige Katalysatoren für die jeweilige Oxidationsstufe offenbaren auch die DE-A 44 31 957, die DE-A 10 2004 025 445 und die DE-A 44 31 949. Dieses gilt insbesondere für jene der allgemeinen Formel I in den beiden vorgenannten älteren Schriften. Besonders vorteilhafte Katalysatoren für die jeweilige Oxidationsstufe offenbaren die Schriften DE-A 103 25 488, DE-A 103 25 487, DE-A 103 53 954, DE-A 103 44 149, DE-A 103 51 269, DE-A 103 50 812 und DE-A 103 50 822.

Für die erfindungsgemäße Reaktionsstufe der heterogen katalysierten Gasphasen-Partialoxidation von Propylen zu Acrolein, oder Acrylsäure, oder deren Gemisch, kommen prinzipiell alle Mo, Bi und Fe enthaltenden Multimetalloxidmassen als Aktivmasse in Betracht.

Dies sind insbesondere die Multimetalloxidaktivmassen der allgemeinen Formel I der DE-A 199 55 176, die Multimetalloxidaktivmassen der allgemeinen Formel I der DE-A 199 48 523, die Multimetalloxidaktivmassen der allgemeinen Formeln I, und III der DE-A 101 01 695, die Multimetalloxidaktivmassen der allgemeinen Formeln I, II und III der DE-A 199 48 248 und die Multimetalloxidaktivmassen der allgemeinen Formeln I, II und III der DE-A 199 55 168 sowie die in der EP-A 7 00 714 genannten Multimetalloxidaktivmassen.

Ferner eignen sich für diese Reaktionsstufe die Mo, Bi und Fe enthaltenden Multimetalloxidkatalysatoren, die in den Schriften Research Disclosure Nr. 497012 vom 29.08.2005 (versehentlich wurde in den dortigen Ausführungsbeispielen die spezifische Oberfläche fehlerhaft in $cm^2/g$ anstelle korrekt bei gleichem Zahlenwert in $m^2/g$ angegeben), DE-A 100 46 957, DE-A 100 63 162, DE-C 3 338 380, DE-A 199 02 562, EP-A 15 565, DE-C 2 380 765, EP-A 8 074 65, EP-A 27 93 74, DE-A 330 00 44, EP-A 575897, US-A 4438217, DE-A 19855913, WO 98/24746, DE-A 197 46 210 (diejenigen der allgemeinen Formel II), JP-A 91/294239, EP-A 293 224 und EP-A 700 714 offenbart werden. Dies gilt insbesondere für die beispielhaften Ausführungsformen in diesen Schriften, unter denen jene der EP-A 15 565, der EP-A 575 897, der DE-A 197 46 210 und der DE-A 198 55 913 besonders bevorzugt werden. Besonders hervorzuheben sind in diesem Zusammenhang ein Katalysator gemäß Beispiel 1c aus der EP-A 15 565 sowie ein in entsprechender Weise herzustellender Katalysator, dessen Aktivmasse jedoch die Zusammensetzung $Mo_{12}Ni_{6,5}Zn_2Fe_2Bi_1P_{0,0065}K_{0,06}O_x \cdot 10SiO_2$ aufweist. Ferner sind hervorzuheben das Beispiel mit der laufenden Nr. 3 aus der DE-A 198 55 913 (Stöchiometrie: $Mo_{12}Co_7Fe_3Bi_{0,6}K_{0,08}Si_{1,6}O_x$) als Hohlzylindervollkatalysator der Geometrie 5 mm x 3 mm x 2 mm (Außendurchmesser x Höhe x Innendurchmesser) sowie der Multimetalloxid II - Vollkatalysator gemäß Beispiel 1 der DE-A 197 46 210. Ferner wären die Multimetalloxid-Katalysatoren der US-A 4,438,217 zu nennen. Letzteres gilt insbesondere dann, wenn diese Hohlzylinder eine Geometrie 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Weitere mögliche Katalysatorgeometrien sind in diesem Zusammenhang Stränge (z. B. 7,7 mm Länge und 7 mm Durchmesser; oder 6,4 mm Länge und 5,7 mm Durchmesser).

Eine Vielzahl der für den Schritt von Propylen zu Acrolein und gegebenenfalls Acrylsäure geeigneten Multimetalloxidaktivmassen lässt sich unter der allgemeinen Formel IV,

$$Mo_{12}Bi_aFe_bX^1_cX^2_dX^3_dX^4_fO_n, \qquad (IV),$$

in der die Variablen nachfolgende Bedeutung aufweisen:

> $X^1$ = Nickel und/oder Kobalt,
> $X^2$ = Thallium, ein Alkalimetall und/oder ein Erdalkalimetall,
> $X^3$ = Zink, Phosphor, Arsen, Bor, Antimon, Zinn, Cer, Blei und/oder Wolfram,
> $X^4$ = Silicium, Aluminium, Titan und/oder Zirkonium,
> a= 0,5 bis 5,
> b= 0,01 bis 5, vorzugsweise 2 bis 4,
> c= 0 bis 10, vorzugsweise 3 bis 10,
> d= 0 bis 2, vorzugsweise 0,02 bis 2,
> e= 0 bis 8, vorzugsweise 0 bis 5,
> f= 0 bis 10 und
> n= eine Zahl, die durch die Wertigkeit und Häufigkeit der von Sauerstoff ver-schiedenen Elemente in IV bestimmt wird,

subsumieren.

Sie sind in an sich bekannter Weise erhältlich (siehe z. B. die DE-A 4 023 239) und werden üblicherweise in Substanz zu Kugeln, Ringen oder Zylindern geformt oder auch in Gestalt von Schalenkatalysatoren, d. h., mit der Aktivmasse beschichteten vorgeformten, inerten Trägerkörpern, eingesetzt. Selbstverständlich können sie aber auch in Pulverform als Katalysatoren angewendet werden (z. B. in Wirbelbettreaktoren).

Prinzipiell können Aktivmassen der allgemeinen Formel IV in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemisch aus Inertgas, $NH_3$, CO und/oder $H_2$) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die Multimetalloxidaktivmassen der allgemeinen Formel IV können für den Schritt "Propylen → Acrolein (und gegebenenfalls Acrylsäure)" sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Anstelle von Graphit kann aber auch hexagonales Bornitrid als Hilfsmittel bei der Formgebung verwendet werden, wie es die DE-A 10 2005 037 678 empfiehlt. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 29 09 671, der EP-A 293 859 oder aus der EP-A 714 700 bekannt ist. Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 $\mu$m, bevorzugt im Bereich 50 bis 500 $\mu$m und besonders bevorzugt im Bereich 150 bis 250 $\mu$m liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z. B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für den Schritt vom Propylen zum Acrolein (sowie gegebenenfalls Acrylsäure) zu verwendende Multimetalloxidaktivmassen sind ferner Massen der allgemeinen Formel V,

$$[Y^1_{a'}Y^2_{b'}O_{x'}]_p[Y^3_{c'}Y^4_{d'}Y^5_{e'}Y^6_{f'}Y^7_{g'}Y^2_{h'}O_{y'}]_q \qquad (V),$$

in der die Variablen folgende Bedeutung haben:

$Y^1$ = nur Wismut oder Wismut und wenigstens eines der Elemente Tellur, Antimon, Zinn und Kupfer,
$Y^2$ = Molybdän, oder Wolfram, oder Molybdän und Wolfram,
$Y^3$ = ein Alkalimetall, Thallium und/oder Samarium,
$Y^4$ = ein Erdalkalimetall, Nickel, Kobalt, Kupfer, Mangan, Zink, Zinn, Cadmium und/oder Quecksilber,
$Y^5$ = Eisen oder Eisen und wenigstens eines der Elemente Chrom und Cer,
$Y^6$ = Phosphor, Arsen, Bor und/oder Antimon,
$Y^7$ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,
a' = 0,01 bis 8,
b' = 0,1 bis 30,
c' = 0 bis 4,
d' = 0 bis 20,
e' > 0 bis 20,
f' = 0 bis 6,
g' = 0 bis 15,
h' = 8 bis 16,
x',y'= Zahlen, die durch die Wertigkeit und Häufigkeit der von Sauerstoff verschiedenen Elemente in V bestimmt werden und
p,q = Zahlen, deren Verhältnis p/q 0,1 bis 10 beträgt,

enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}Y^2_{b'}O_{x'}$, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Besonders vorteilhafte erfindungsgemäß geeignete Multimetalloxidmassen V sind solche, in denen $Y^1$ nur Wismut ist.

Unter diesen werden wiederum jene bevorzugt, die der allgemeinen Formel VI,

$$[Bi_{a''}Z^2_{b''}O_{x''}]_{p''} [Z^2_{12}Z^3_{c''}Z^4_{d''}Fe_{e''}Z^5_{f''}Z^6_{g''}Z^7_{h''}O_{y''}]_{q''} \qquad (VI),$$

in der die Variablen folgende Bedeutung haben:

$Z^2$ = Molybdän, oder Wolfram, oder Molybdän und Wolfram,
$Z^3$ = Nickel und/oder Kobalt,
$Z^4$ = Thallium, ein Alkalimetall und/oder ein Erdalkalimetall,
$Z^5$ = Phosphor, Arsen, Bor, Antimon, Zinn, Cer und/oder Blei,
$Z^6$ = Silicium, Aluminium, Titan und/oder Zirkonium,
$Z^7$ = Kupfer, Silber und/oder Gold,
a" = 0,1 bis 1,
b" = 0,2 bis 2,
c" = 3 bis 10,
d" = 0,02 bis 2,
e" = 0,01 bis 5, vorzugsweise 0,1 bis 3,
f" = 0 bis 5,
g" = 0 bis 10,
h" = 0 bis 1,
x",y"= Zahlen, die durch die Wertigkeit und Häufigkeit der von Sauerstoff verschiede nen Elemente in VI bestimmt werden,
p",q"= Zahlen, deren Verhältnis p"/q" 0,1 bis 5, vorzugsweise 0,5 bis 2 beträgt,

entsprechen, wobei diejenigen Massen VI ganz besonders bevorzugt werden, in denen $Z^2_{b''}$ = (Wolfram)$_{b''}$ und $Z^2_{12}$ = (Molybdän)$_{12}$ ist.

Ferner ist es von Vorteil, wenn wenigstens 25 mol-% (bevorzugt wenigstens 50 mol-% und besonders bevorzugt 100 mol-%) des gesamten Anteils $[Y^1_{a'}Y^2_{b'}O_{x'}]_p$ ($[Bi_{a''}Z^2_{b''}O_{x''}]_{p''}$) der erfindungsgemäß geeigneten Multimetalloxidmassen V (Multimetalloxidmassen VI) in den erfindungsgemäß geeigneten Multimetalloxidmassen V (Multimetalloxidmassen VI) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $Y^1_{a'}Y^2_{b'}O_{x'}$ [$Bi_{a''}Z^2_{b''}O_{x''}$] vorliegen, deren Größtdurchmesser im Bereich 1 nm bis 100 $\mu$m liegt.

Hinsichtlich der Formgebung gilt bezüglich Multimetalloxidmassen V-Katalysatoren das bei den Multimetalloxidmassen IV-Katalysatoren Gesagte.

Die Herstellung von Multimetalloxidmassen V-Aktivmassen ist z. B. in der EP-A 575 897 sowie in der DE-A 198 55 913 beschrieben.

Die vorstehend empfohlenen inerten Trägermaterialien kommen u.a. auch als inerte Materialien zur Verdünnung und/oder Abgrenzung der entsprechenden Katalysatorfestbetten, bzw. als deren schützende und/oder das Gasgemisch aufheizende Vorschüttung in Betracht.

Für den zweiten Schritt (die zweite Reaktionsstufe), die heterogen katalysierte Gasphasen-Partialoxidation von Acrolein zu Acrylsäure, kommen, wie bereits gesagt, prinzipiell alle Mo und V enthaltenden Multimetalloxidmassen als Aktivmassen für die benötigten Katalysatoren in Betracht, z. B. jene der DE-A 100 46 928.

Eine Vielzahl derselben, z. B. diejenigen der DE-A 198 15 281, lässt sich unter der allgemeinen Formel VII,

$$Mo_{12}V_aX^1_bX^2_cX^3_dX^4_eX^5_fX^6_gO_n \qquad (VII),$$

in der die Variablen folgende Bedeutung haben:

$X^1$ = W, Nb, Ta, Cr und/oder Ce,
$X^2$ = Cu, Ni, Co, Fe, Mn und/oder Zn,
$X^3$ = Sb und/oder Bi,

$X^4$ = eines oder mehrere Alkalimetalle,

$X^5$ = eines oder mehrere Erdalkalimetalle,

$X^6$ = Si, Al, Ti und/oder Zr,

a = 1 bis 6,

b = 0,2 bis 4,

c = 0,5 bis 18,

d = 0 bis 40,

e = 0 bis 2,

f = 0 bis 4,

g = 0 bis 40 und

n = eine Zahl, die durch die Wertigkeit und Häufigkeit der von Sauerstoff verschiedenen Elemente in VII bestimmt wird,

subsumieren.

Erfindungsgemäß bevorzugte Ausführungsformen innerhalb der aktiven Multimetalloxide VII sind jene, die von den nachfolgenden Bedeutungen der Variablen der allgemeinen Formel VII erfasst werden:

$X^1$ = W, Nb und/oder Cr,

$X^2$ = Cu, Ni, Co und/oder Fe,

$X^3$ = Sb,

$X^4$ = Na und/oder K,

$X^5$ = Ca, Sr und/oder Ba,

$X^6$ = Si, Al und/oder Ti,

a = 1,5 bis 5,

b = 0,5 bis 2,

c = 0,5 bis 3,

d = 0 bis 2,

e = 0 bis 0,2,

f = 0 bis 1 und

n = eine Zahl, die durch die Wertigkeit und Häufigkeit der von Sauerstoff verschiedenen Elemente in VII bestimmt wird.

Erfindungsgemäß ganz besonders bevorzugte Multimetalloxide VII sind jedoch jene der allgemeinen Formel VIII,

$$Mo_{12}V_{a'}Y^1_{b'}Y^2_{c'}Y^5_{f'}Y^6_{g'}O_{n'} \qquad (VIII),$$

mit

$Y^1$ = W und/oder Nb,

$Y^2$ = Cu und/oder Ni,

$Y^5$ = Ca und/oder Sr,

$Y^6$ = Si und/oder Al,

a' = 2 bis 4,

b' = 1 bis 1,5,

c' = 1 bis 3,

f' = 0 bis 0,5,

g' = 0 bis 8 und

n' = eine Zahl, die durch die Wertigkeit und Häufigkeit der von Sauerstoff verschiedenen Elementen in VIII bestimmt wird.

Die erfindungsgemäß geeigneten Multimetalloxidaktivmassen (VII) sind in an sich bekannter, z. B. in der DE-A 43 35 973 oder in der EP-A 714 700 offenbarter, Weise erhältlich.

Prinzipiell können für den Schritt "Acrolein → Acrylsäure" geeignete Multimetalloxidaktivmassen, insbesondere solche der allgemeinen Formel VII, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemische aus Inertgas und reduzierenden Gasen wie

H$_2$, NH$_3$, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen VII kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen VII kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die resultierenden Multimetalloxidmassen, insbesondere jene der allgemeinen Formel VII, können für die Acroleinoxidation sowohl in Pulverform (z. B. im Wirbelbettreaktor) als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm (z. B. 8,2 mm oder 5,1 mm) betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 2 909 671, der EP-A 293 859 oder aus der EP-A 714 700 bekannt ist.

Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 μm, bevorzugt im Bereich 50 bis 500 μm und besonders bevorzugt im Bereich 150 bis 250 μm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z. B. Kugeln oder Hohlzylinder mit Splittauflage, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. D. h., geeignete Kugelgeometrien können Durchmesser von 8,2 mm oder von 5,1 mm aufweisen. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige für den Schritt "Acrolein → Acrylsäure" zu verwendende Multimetalloxidaktivmassen sind ferner Massen der allgemeinen Formel IX,

$$[D]_p[E]_q \qquad (IX),$$

in der die Variablen folgende Bedeutung haben:

D = $Mo_{12}V_{a"}Z^1{}_{b"}Z^2{}_{c"}Z^3{}_{d"}Z^4{}_{e"}Z^5{}_{f"}Z^6{}_{g"}O_{x"}$,
E = $Z^7{}_{12}Cu_{h"}H_{i"}O_{y"}$,
$Z^1$ = W, Nb, Ta, Cr und/oder Ce,
$Z^2$ = Cu, Ni, Co, Fe, Mn und/oder Zn,

$Z^3$ = Sb und/oder Bi,

$Z^4$ = Li, Na, K, Rb, Cs und/oder H

$Z^5$ = Mg, Ca, Sr und/oder Ba,

$Z^6$ = Si, Al, Ti und/oder Zr,

$Z^7$ = Mo, W, V, Nb und/oder Ta, vorzugsweise Mo und/oder W

a" = 1 bis 8,

b" = 0,2 bis 5,

c" = 0 bis 23,

d" = 0 bis 50,

e" = 0 bis 2,

f" = 0 bis 5,

g" = 0 bis 50,

h" = 4 bis 30,

i" = 0 bis 20 und

x",y" = Zahlen, die durch die Wertigkeit und Häufigkeit der von Sauerstoff verschiedenen Element in IX bestimmt werden und

p,q = von Null verschiedene Zahlen, deren Verhältnis p/q 160:1 bis 1:1 beträgt,

und die dadurch erhältlich sind, dass man eine Multimetalloxidmasse E

$$Z^7_{12}Cu_{h"}H_{i"}O_{y"} \qquad (E),$$

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, $Z^1$, $Z^2$, $Z^3$, $Z^4$, $Z^5$, $Z^6$, die die vorgenannten Elemente in der Stöchiometrie D

$$Mo_{12}V_{a"}Z^1_{b"}Z^2_{c"}Z^3_{d"}Z^4_{e"}Z^5_{f"}Z^6_{g"} \qquad (D),$$

enthält (Ausgangsmasse 2), im gewünschten Mengenverhältnis p : q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind die Multimetalloxidmassen IX, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen VI-Katalysatoren enthalten z. B. die EP-A 668 104, die DE-A 197 36 105, die DE-A 100 46 928, die DE-A 197 40 493 und die DE-A 195 28 646. Hinsichtlich der Formgebung gilt bezüglich Multimetalloxidmassen IX-Katalysatoren das bei den Multimetalloxidmassen VII-Katalysatoren Gesagte.

Für den Schritt "Acrolein → Acrylsäure" in hervorragender Weise geeignete Multimetalloxidkatalysatoren sind auch jene der DE-A 198 15 281, insbesondere mit Multimetalloxidaktivmassen der allgemeinen Formel I dieser Schrift.

Vorteilhaft werden für den Schritt vom Propylen zum Acrolein Vollkatalysatorringe und für den Schritt vom Acrolein zur Acrylsäure Schalenkatalysatorringe verwendet.

Die Durchführung der Paritaloxidation des erfindungsgemäßen Verfahrens, vom Propylen zum Acrolein (sowie gegebenenfalls Acrylsäure), kann mit den beschriebenen Katalysatoren z. B. in einem Einzonen-Vielkontaktrohr-Festbettreaktor durchgeführt werden, wie ihn die DE-A 44 31 957 beschreibt. Dabei können Reaktionsgasgemisch und Wärmeträger (Wärmeaustauschmittel) über den Reaktor betrachtet im Gleichstrom oder im Gegenstrom geführt werden.

Der Reaktionsdruck liegt üblicherweise im Bereich von 1 bis 3 bar und die Gesamtraumbelastung des Katalysatorfestbetts mit Reaktionsgas B beträgt vorzugsweise 1500 bis 4000 bzw. 6000 Nl/l•h oder mehr. Die Propylenlast (die Propylenbelastung des Katalysatorfestbetts) beträgt typisch 90 bis 200 Nl/l•h oder bis 300 Nl/l•h oder mehr. Propylenlasten oberhalb von 135 Nl/l•h bzw. ≥ 140 Nl/l•h, oder ≥ 150 Nl/l•h, oder ≥ 160 Nl/l•h sind erfindungsgemäß besonders bevorzugt, da das erfindungsgemäße Reaktionsgasausgangsgemisch für die Reaktionszone B infolge des Beiseins von nicht umgesetztem Propan und gegebenenfalls molekularem Wasserstoff ein günstiges Heißpunktverhalten bedingt (alles Vorgenannte gilt auch unabhängig von der speziellen Wahl des Festbettreaktors).

Bevorzugt wird der Einzonen-Vielkontaktrohr-Festbettreaktor mit dem Beschickungsgasgemisch von oben angeströmt. Als Wärmeaustauschmittel wird zweckmäßig eine Salzschmelze, bevorzugt bestehend aus 60 Gew.-% Kaliumnitrat ($KNO_3$) und 40 Gew.-% Natriumnitrit ($NaNO_2$), oder aus 53 Gew.-% Kaliumnitrat ($KNO_3$), 40 Gew.-% Natriumnitrit ($NaNO_2$) und 7 Gew.-% Natriumnitrat ($NaNO_3$), eingesetzt.

Über den Reaktor betrachtet können, wie bereits gesagt, Salzschmelze und Reaktionsgasgemisch sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt mit Katalysator zu beschicken (für die Anströmung von unten nach oben wird die Beschickungsabfolge in zweckmäßiger Weise umgekehrt):

- zunächst auf einer Länge von 40 bis 80 bzw. bis 60 % der Kontaktrohrlänge entweder nur Katalysator, oder ein Gemisch aus Katalysator und Inertmaterial, wobei letzteres, bezogen auf die Mischung, einen Gewichtsanteil von bis zu 30 bzw. bis zu 20 Gew.-% ausmacht (Abschnitt C);

- daran anschließend auf einer Länge von 20 bis 50 bzw. bis 40% der Gesamtrohrlänge entweder nur Katalysator, oder ein Gemisch aus Katalysator und Inertmaterial, wobei letzteres, bezogen auf die Mischung, einen Gewichtsanteil von bis zu 40 Gew.-% ausmacht (Abschnitt B); und

- abschließend auf einer Länge von 10 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt.

Vorgenannte Beschickungsvariante ist insbesondere dann zweckmäßig, wenn als Katalysatoren solche gemäß Research Disclosure Nr. 497012 vom 29.08.2005, oder gemäß Beispiel 1 der DE-A 100 46 957 oder gemäß Beispiel 3 der DE-A 100 46 957 und als Inertmaterial Ringe aus Steatit mit der Geometrie 7 mm x 7 mm x 4 mm (Außendurchmesser x Höhe x Innendurchmesser) verwendet werden. Hinsichtlich der Salzbadtemperatur gilt das in der DE-A 4 431 957 Gesagte.

Die Durchführung der Partialoxidation in der Reaktionszone B, vom Propylen zum Acrolein (und gegebenenfalls Acrylsäure), kann mit den beschriebenen Katalysatoren aber auch z. B. in einem Zweizonen-Vielkontaktrohr-Festbettreaktor durchgeführt werden, wie es die DE-A 199 10 506, die DE-A 10 2005 009 885, die DE-A 10 2004 032 129, die DE-A 10 2005 013 039 und die DE-A 10 2005 009 891 sowie die DE-A 10 2005 010 111 beschreibt. In beiden vorstehend beschriebenen Fällen (sowie ganz allgemein beim erfindungsgemäßen Verfahren) liegt der erzielte Propenumsatz bei einfachem Durchgang normalerweise bei Werten ≥ 90 mol-%, bzw. ≥ 95 mol-% und die Selektivität der Acroleinbildung bei Werten ≥ 90 mol-%. Erfindungsgemäß vorteilhaft erfolgt die erfindungsgemäße Partialoxidation von Propen zu Acrolein, oder Acrylsäure oder deren Gemisch wie in der EP-A 1 159 244 und ganz besonders bevorzugt wie in der WO 04/085363 sowie in der WO 04/085362 beschrieben.

D. h., eine in der Reaktionszone B durchzuführende Partialoxidation des Propylens zu Acrolein (und gegebenenfalls Acrylsäure) lässt sich besonders vorteilhaft an einem Katalysatorfestbett mit erhöhter Propylenbelastung durchführen, das wenigstens zwei Temperaturzonen aufweist.

Diesbezüglich wird z. B. auf die EP-A 1 159 244 und die WO 04/085362 verwiesen.

Die Durchführung des zweiten Schrittes im Fall einer zweistufigen Partialoxidation von Propylen zu Acrolein, nämlich die Partialoxidation vom Acrolein zur Acrylsäure, kann mit den beschriebenen Katalysatoren z. B. in einem Einzonen-Vielkontaktrohr-Festbettreaktor durchgeführt werden, wie es die DE-A 44 31 949 beschreibt. Dabei können Reaktiongsgasgemisch und Wärmeträger über den Reaktor betrachtet im Gleichstrom geführt werden. In der Regel wird das Produktgasgemisch der vorausgehenden erfindungsgemäßen Propylenpartialoxidation zu Acrolein grundsätzlich als solches (gegebenenfalls nach erfolgter Zwischenkühlung (diese kann indirekt oder direkt durch z. B. Sekundärsauerstoff-(oder erfindungsgemäß weniger bevorzugt Sekundärluft-)-Zugabe erfolgen) desselben), d.h., ohne Nebenkomponentenabtrennung, in die zweite Reaktionsstufe, d.h. in die Acroleinpartialoxidation geführt.

Der für den zweiten Schritt, die Acroleinpartialoxidation benötigte molekulare Sauerstoff kann (dabei) schon im Reaktionsgas B-Ausgangsgemisch für die Propylenpartialoxidation zum Acrolein enthalten sein. Er kann aber auch teilweise oder vollständig dem Produktgasgemisch der ersten Reaktionsstufe, d. h., der Propylenpartialoxidation zum Acrolein, unmittelbar zugegeben werden (dies kann als Sekundärluft, vorzugsweise jedoch in Form von reinem Sauerstoff oder von Gemischen aus Inertgas und Sauerstoff (mit bevorzugt ≤ 50 Vol.-%, oder ≤ 40 Vol.-%, oder ≤ 30 Vol.-%, oder ≤ 20 Vol.-%, oder ≤ 10 Vol.-%, oder ≤ 5 Vol.-%, oder ≤ 2 Vol.-% erfolgen)). Für Druck und Temperatur der Sekundärsauerstoffquelle gilt das für die Primärsauerstoffquelle Gesagte. Unabhängig davon wie vorgegangen wird, weist das Beschickungsgasgemisch (das Reaktionsgasausgangsgemisch) einer solchen Partialoxidation des Acroleins zu Acrylsäure, mit Vorteil nachfolgende Gehalte auf:

| | |
|---|---|
| 3 bis 25 Vol.-% | Acrolein, |
| 5 bis 65 Vol.-% | molekularer Sauerstoff, |
| 6 bis 70 Vol.-% | Propan, |
| 0 bis 20 Vol.-% | molekularer Wasserstoff, |
| 8 bis 65 Vol.-% | Wasserdampf sowie |

(fortgesetzt)

| | |
|---|---|
| 0 bis 70 Vol-% | molekularer Stickstoff. |

Bevorzugt weist das vorgenannte Reaktionsgasausgangsgemisch folgende Gehalte auf:

| | |
|---|---|
| 5 bis 20 Vol.-% | Acrolein, |
| 5 bis 15 Vol.-% | molekularer Sauerstoff, |
| 10 bis 40 Vol.-% | Propan, |
| 0 bis 10 Vol.-% | molekularer Wasserstoff und |
| 5 bis 30 Vol.-% | Wasserdampf. |

Ganz besonders bevorzugt weist das vorgenannte Reaktionsgasausgangsgemisch folgende Gehalte auf:

| | |
|---|---|
| 10 bis 20 Vol.-% | Acrolein, |
| 7 bis 15 Vol.-% | molekularer Sauerstoff, |
| 20 bis 40 Vol.-% | Propan, |
| 3 bis 10 Vol.-% | molekularer Wasserstoff und |
| 15 bis 30 Vol.-% | Wasserdampf. |

Der Stickstoffgehalt in den vorgenannten Gemischen wird in der Regel ≤ 20 Vol.-%, bevorzugt ≤ 15 Vol.-%, besonders bevorzugt ≤ 10 Vol.-% und ganz besonders bevorzugt ≤ 5 Vol.-% betragen. Das Verhältnis der im Beschickungsgasgemisch für die zweite Oxidationsstufe enthaltenen molaren Mengen an $O_2$ und Acrolein, $O_2$: Acrolein, beträgt erfindungsgemäß vorteilhaft in der Regel ≥ 0,5 und ≤ 2, häufig ≥ 1 und ≤ 1,5.

Der $CO_2$-Gehalt des Beschickungsgasgemischs für die zweite Oxidationsstufe wird in der Regel ≤ 5 Vol.-% betragen.

Wie in der ersten Reaktionsstufe (Propylen → Acrolein) liegt auch in der zweiten Reaktionsstufe (Acrolein → Acrylsäure) der Reaktionsdruck üblicherweise im Bereich von > 1 bis 4 bar, vorzugsweise 1,3 bis 3 bar, vielfach 1,5 bis 2,5 bar, und die Gesamtraumbelastung des Katalysatorfestbetts mit Reaktionsgas(ausgangs)gemisch liegt vorzugsweise bei 1500 bis 4000 bzw. bis 6000 Nl/l•h oder mehr. Die Acroleinlast (die Acroleinbelastung des Katalysatorfestbetts) beträgt typisch 90 bis 190 Nl/l•h, oder bis 290 Nl/l•h oder mehr. Acroleinlasten oberhalb von 135 Nl/l•h, bzw. ≥ 140 Nl/l•h, oder ≥ 150 Nl/l•h, oder ≥ 160 Nl/l•h sind besonders bevorzugt, da das erfindungsgemäß einzusetzende Reaktionsgasausgangsgemisch infolge des Beiseins von Propan und gegebenenfalls molekularem Wasserstoff ebenfalls ein günstiges Heißpunktverhalten bedingt.

Die Temperatur des Beschickungsgasgemischs für die zweite Oxidationsstufe beträgt in der Regel 220 bis 270 °C.

Der Acroleinumsatz, bezogen auf einmaligen Durchgang des Reaktionsgasgemischs durch das Katalysatorfestbett der zweiten Oxidationsstufe, beträgt zweckmäßig normalerweise ≥ 90 mol-% und die damit einhergehende Selektivität der Acrylsäurebildung ≥ 90 mol-%.

Bevorzugt wird der Einzonen-Vielkontaktrohr-Festbettreaktor mit dem Beschickungsgasgemisch ebenfalls von oben angeströmt. Als Wärmeaustauschmittel wird auch in der zweiten Stufe in zweckmäßiger Weise eine Salzschmelze, bevorzugt aus 60 Gew.-% Kaliumnitrat ($KNO_3$) und 40 Gew.-% Natriumnitrit ($NaNO_2$) oder aus 53 Gew.-% Kaliumnitrat ($KNO_3$), 40 Gew.-% Natriumnitrit ($NaNO_2$) und 7 Gew.-% Natriumnitrat ($NaNO_3$) bestehend, eingesetzt. Über den Reaktor betrachtet können, wie bereits gesagt, Salzschmelze und Reaktionsgasgemisch sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt zu beschicken:

- zunächst auf einer Länge von 50 bis 80 bzw. bis 70 % der Kontaktrohrlänge entweder nur Katalysator oder ein Gemisch aus Katalysator und Inertmaterial, wobei letzteres, bezogen auf die Mischung, einen Gewichtsanteil von bis zu 30 (bzw. 20) Gew.-% ausmacht (Abschnitt C);

- daran anschließend auf einer Länge von 20 bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 50 bzw. bis zu 40 Gew.-% ausmacht (Abschnitt B); und

- abschließend auf einer Länge von 5 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt. Wie ganz allgemein bei der heterogen katalysierten Gasphasenpartialoxidation von Acrolein zu Acrylsäure (insbesondere bei hohen Acroleinbelastungen des Katalysatorbetts und hohen Wasserdampfgehalten des Beschickungsgasgemischs), kann der Abschnitt B auch aus zwei aufeinanderfolgenden Katalysatorverdünnungen bestehen (zum Zweck der Minimierung von Heißpunkttemperatur und Heißpunkttemperatursensitivität). Von unten nach oben zunächst mit bis zu 30 (bzw. 20) Gew.-% Inertmaterial und daran anschließend mit > 20 Gew.-% bis 50 bzw. bis 40 Gew.-% Inertmaterial. Der Abschnitt C ist dann bevorzugt unverdünnt.

Für eine Anströmung der Kontaktrohre von unten nach oben, wird die Kontaktrohrbeschickung in zweckmäßiger Weise umgekehrt.

Vorgenannte Beschickungsvariante ist insbesondere dann zweckmäßig, wenn als Katalysatoren solche gemäß Herstellungsbeispiel 5 der DE-A 100 46 928 oder solche gemäß der DE-A 198 15 281 und als Inertmaterial Ringe aus Steatit mit der Geometrie 7 mm x 7 mm x 4 mm oder 7 mm x 7 mm x 3 mm (jeweils Außendurchmesser x Höhe x Innendurchmesser) verwendet werden. Hinsichtlich der Salzbadtemperatur gilt das in der DE-A 443 19 49 Gesagte. Sie wird in der Regel so gewählt, daß der erzielte Acroleinumsatz bei einfachem Durchgang normalerweise ≥ 90 mol-%, bzw. ≥ 95 mol-% oder ≥ 99 mol-% beträgt.

Die Durchführung der Partialoxidation vom Acrolein zur Acrylsäure, kann mit den beschriebenen Katalysatoren aber auch z. B. in einem Zweizonen-Vielkontaktrohr-Festbettreaktor durchgeführt werden, wie es die DE-A 199 10 508 beschreibt. Für den Acroleinumsatz gilt das oben Genannte. Auch im Fall der Durchführung einer wie vorstehend beschriebenen Acroleinpartialoxidation als zweite Reaktionsstufe einer zweistufigen Propylenpartialoxidation zu Acrylsäure in einem Zweizonen-Vielkontaktrohr-Festbettreaktor wird man zur Erzeugung des Beschickungsgasgemisches (Reaktionsgasausgangsgemischs) zweckmäßig unmittelbar das Produktgasgemisch der auf den ersten Schritt (Propylen → Acrolein) gerichteten Partialoxidation (gegebenenfalls nach erfolgter indirekter oder direkter (z. B. durch Zufuhr von Sekundärsauerstoff) Zwischenkühlung desselben) verwenden (wie sie vorstehend bereits beschrieben wurde). Der für die Acroleinpartialoxidation benötigte Sauerstoff wird vorzugsweise als reiner molekularer Sauerstoff (gegebenenfalls aber auch als Luft oder als Gemisch aus molekularem Sauerstoff und einem Inertgas mit einem Inertgasanteil von vorzugsweise ≤ 50 Vol.-%, besonders bevorzugt ≤ 40 Vol.-%, oder ≤ 30 Vol.-%, oder ≤ 20 Vol.-%, noch besser ≤ 10 Vol.-%, oder ≤ 5 Vol.-%, oder ≤ 2 Vol.-%) zugesetzt und z. B. dem Produktgasgemisch des ersten Schritts der zweistufigen Partialoxidation (Propylen → Acrolein) unmittelbar zugegeben. Er kann aber auch, wie bereits beschrieben, bereits im Reaktionsgasausgangsgemisch für die erste Reaktionsstufe enthalten sein.

Bei einer zweistufigen Partialoxidation von Propylen zu Acrylsäure mit unmittelbarer Weiterverwendung des Produktgasgemisches des ersten Schritts der Partialoxidation zur Beschickung des zweiten Schritts der Partialoxidation, wird man in der Regel zwei Einzonen-Vielkontaktrohr-Festbettreaktoren (bei hoher Reaktandenbelastung des Katalysatorbetts ist, wie ganz allgemein gültig, eine Gleichstromfahrweise zwischen Reaktionsgas und Salzbad (Wärmeträger) über den Rohrbündelreaktor betrachtet bevorzugt) oder zwei Zweizonen-Vielkontaktrohr-Festbettreaktoren hintereinanderschalten. Eine gemischte Hintereinanderschaltung (Einzonen/Zweizonen oder umgekehrt) ist auch möglich.

Zwischen den Reaktoren kann sich ein Zwischenkühler befinden, der gegebenenfalls Inertschüttungen enthalten kann, die eine Filterfunktion ausüben können. Die Salzbadtemperatur von Vielkontaktrohrreaktoren für den ersten Schritt der zweistufigen Partialoxidation von Propylen zu Acrylsäure beträgt in der Regel 300 bis 400°C. Die Salzbadtemperatur von Vielkontaktrohrreaktoren für den zweiten Schritt der Partialoxidation von Propylen zu Acrylsäure, die Partialoxidation von Acrolein zu Acrylsäure, beträgt meist 200 bis 350°C. Außerdem werden die Wärmeaustauschmittel (bevorzugt Salzschmelzen) normalerweise in solchen Mengen durch die relevanten Vielkontaktrohr-Festbettreaktoren geführt, dass der Unterschied zwischen ihrer Eingangs- und ihrer Ausgangstemperatur in der Regel ≤ 5°C beträgt. Wie bereits erwähnt, können beide Schritte der Partialoxidation von Propylen zu Acrylsäure aber auch wie in der DE-A 101 21 592 beschrieben in einem Reaktor an einer Beschickung vollzogen werden.

Es sei hier auch erwähnt, dass ein Teil des Reaktionsgas B-Ausgangsgemischs für den ersten Schritt ("Propylen → Acrolein") aus der Trennzone II kommendes Restgas sein kann.

Der Vollständigkeit halber sei an dieser Stelle festgehalten, dass sowohl dem Beschickungsgasgemisch für die erste Oxidationsstufe als auch dem Beschickungsgasgemisch für die zweite Oxidationsstufe oder auch nur einem von beiden zusätzlich Frischpropan zudosiert werden kann. Es ist erfindungsgemäß nicht bevorzugt, kann jedoch gegebenenfalls zweckmäßig sein, um eine Zündfähigkeit der Beschickungsgasgemische auszuschließen.

Insgesamt bildet ein Rohrbündelreaktor innerhalb dessen sich die Katalysatorbeschickung längs der einzelnen Kontaktrohre mit Beendigung des ersten Reaktionsschrittes entsprechend ändert (derartige zweistufige Propylenpartialoxidationen im sogenannten "Single-Reactor" lehren z. B. die EP-A 911 313, die EP-A 979 813, die EP-A 990 636 und die DE-A 28 30 765) die einfachste Realisierungsform zweier Oxidationsstufen für die beiden Schritte der Partialoxidation von Propylen zu Acrylsäure. Gegebenenfalls wird dabei die Beschickung der Kontaktrohre mit Katalysator durch eine Inertmaterialschüttung unterbrochen.

Vorzugsweise werden die beiden Oxidationsstufen jedoch in Form zweier hintereinander geschalteter Rohrbündelsysteme realisiert. Diese können sich in einem Reaktor befinden, wobei der Übergang von einem Rohrbündel zum anderen Rohrbündel von einer nicht im Kontaktrohr untergebrachten (zweckmäßigerweise begehbaren) Schüttung aus Inertmaterial gebildet wird. Während die Kontaktrohre in der Regel von einem Wärmeträger umspült werden, erreicht dieser eine wie vorstehend beschrieben angebrachte Inertmaterialschüttung nicht. Mit Vorteil werden daher die beiden Kontaktrohrbündel in räumlich voneinander getrennten Reaktoren untergebracht. In der Regel befindet sich dabei zwischen den beiden Rohrbündelreaktoren ein Zwischenkühler, um eine gegebenenfalls erfolgende Acroleinnachverbrennung im Produktgasgemisch, das die erste Oxidationszone verlässt, zu mindern. Die Reaktionstemperatur in der ersten Reaktionsstufe (Propylen → Acrolein) liegt in der Regel bei 300 bis 450°C, bevorzugt bei 320 bis 390°C. Die Reaktionstemperatur in der zweiten Reaktionsstufe (Acrolein → Acrylsäure) liegt in der Regel bei 200 bis 370°C, häufig bei 220 bis 330°C. Der Reaktionsdruck beträgt in beiden Oxidationszonen zweckmäßig > 1 bis 4, vorteilhaft 1,3 bis 3 bar. Die Belastung (Nl/l•h) der Oxidationskatalysatoren mit Reaktionsgas beträgt in beiden Reaktionsstufen häufig 1500 bis 2500 Nl/l•h bzw. bis 4000 Nl/l•h. Die Belastung mit Propylen bzw. Acrolein kann dabei bei 100 bis 200 oder 300 und mehr Nl/l•h liegen.

Prinzipiell können die beiden Oxidationsstufen beim erfindungsgemäßen Verfahren so gestaltet werden, wie es z. B. in der DE-A 198 37 517, der DE-A 199 10 506, der DE-A 199 10 508 sowie der DE-A 198 37 519 beschrieben ist.

In beiden Reaktionsstufen wirkt sich dabei ein Überschuss an molekularem Sauerstoff relativ zur reaktionsstöchiometrisch erforderlichen Menge vorteilhaft auf die Kinetik der jeweiligen Gasphasenpartialoxidation sowie auf die Katalysatorstandzeit aus.

Prinzipiell ist die erfindungsgemäß durchzuführende heterogen katalysierte Gasphasen-Partialoxidation von Propylen zu Acrylsäure aber auch in einem einzigen Einzonenrohrbündelreaktor wie folgt realisierbar. Beide Reaktionsschritte erfolgen in einem Oxidationsreaktor der mit einem oder mehreren Katalysatoren beschickt ist, deren Aktivmasse ein die Elemente Mo, Fe und Bi enthaltendes Multimetalloxid ist, das die Umsetzung beider Reaktionsschritte zu katalysieren vermag. Selbstredend kann sich auch diese Katalysatorbeschickung längs der Reaktionskoordinate kontinuierlich oder abrupt ändern. Natürlich kann bei einer Ausführungsform einer erfindungsgemäßen zweistufigen Partialoxidation von Propylen zu Acrylsäure in Gestalt zweier hintereinandergeschalteter Oxidationsstufen aus dem das die erste Oxidationsstufe verlassenden Produktgasgemisch in selbigem enthaltenes, in der ersten Oxidationsstufe als Nebenprodukt entstandenes, Kohlenoxid und Wasserdampf bei Bedarf vor der Weiterleitung in die zweite Oxidationsstufe teilweise oder vollständig abgetrennt werden. Vorzugsweise wird man erfindungsgemäß eine Verfahrensweise wählen, die solch eine Abtrennung nicht vorsieht.

Als Quellen für eine zwischen beiden Oxidationsstufen durchgeführte Sauerstoffzwischeneinspeisung kommen, wie bereits gesagt, neben Luft (dies ist erfindungsgemäß weniger bevorzugt) sowohl reiner molekularer Sauerstoff als auch mit Inertgas wie $CO_2$, CO, Edelgasen, $N_2$ und/oder gesättigten Kohlenwasserstoffen verdünnter molekularer Sauerstoff in Betracht. Erfindungsgemäß bevorzugt enthält die Sauerstoffquelle ≤ 50 Vol.-%, vorzugsweise ≤ 40 Vol.-%, besonders bevorzugt ≤ 30 Vol.-%, ganz besonders bevorzugt ≤ 20 Vol.-%, besser ≤10 Vol.-% oder ≤ 5 Vol.-%, bzw. ≤ 2 Vol.-% an von molekularem Sauerstoff verschiedenen Gasen.

Als Materialien für die inerten Verdünnungsformkörper in den Katalysatorbetten für die beiden Oxidationsstufen kommen diejenigen in Betracht, die auch für die Verdünnung der Katalysatorbetten in der Reaktionszone A empfohlen wurden.

Durch Zudosieren von z. B. kalter Sauerstoffquelle zum Produktgasgemisch der ersten Partialoxidationsstufe kann im Rahmen des erfindungsgemäßen Verfahrens auch auf direktem Weg eine Abkühlung desselben bewirkt werden, bevor es als Bestandteil eines Reaktionsgasausgangsgemischs für die zweite Partialoxidationsstufe weiterverwendet wird.

Erfindungsgemäß vorteilhaft erfolgt die Partialoxidation von Acrolein zu Acrylsäure wie in der EP-A 1 159 246, und ganz besonders bevorzugt wie in der WO 04/085365 sowie in der WO 04/085370 beschrieben. Erfindungsgemäß bevorzugt wird dabei als Acrolein enthaltendes Reaktionsgasausgangsgemisch jedoch ein Reaktionsgasausgangsgemisch verwendet, das das Produktgasgemisch einer erfindungsgemäßen Erststufenpartialoxidation von Propylen zu Acrolein ist, das gegebenenfalls mit soviel Sekundärluft ergänzt wurde, dass das Verhältnis von molekularem Sauerstoff zu Acrolein in dem resultierenden Reaktionsgasausgangsgemisch in jedem Fall 0,5 bis 1,5 beträgt. D. h., die erfindungsgemäße Partialoxidation von Acrolein zu Acrylsäure lässt sich vorteilhaft an einem Katalysatorfestbett mit erhöhter Acroleinbelastung durchführen, das wenigstens zwei Temperaturzonen aufweist.

Insgesamt wird man eine zweistufige Partialoxidation von Propylen zu Acrylsäure bevorzugt wie in der EP-A 1 159 248 bzw. in der WO 04/085367 oder WO 04/085369 beschrieben durchführen.

Das die erfindungsgemäß durchzuführende Partialoxidation (nach der ersten und/oder zweiten Oxidationsstufe) verlassende Produktgas B enthält im wesentliche Acrolein, oder Acrylsäure, oder deren Gemisch als Zielprodukt, nicht umgesetztes Propan, gegebenenfalls molekularen Wasserstoff, (als Nebenprodukt entstandenen und/oder als Verdünnungsgas mitverwendeten) Wasserdampf, gegebenenfalls nicht umgesetzten molekularen Sauerstoff (mit Blick auf die Lebensdauer der verwendeten Katalysatoren ist es üblicherweise günstig, wenn der Sauerstoffgehalt im Produktgasgemisch beider Partialoxidationsstufen z. B. noch wenigsten 1,5 bis 4 Vol.-% beträgt), sowie andere (bei Normaldruck, d. h., 1 bar) leichter und schwerer als Wasser siedende Nebenprodukte bzw. -komponenten (wie z. B. CO, $CO_2$, niedere Aldehyde, niedere Alkancarbonsäuren (z. B. Essigsäure, Ameisensäure und Propionsäure), Maleinsäureanhydrid, Benzaldehyd, aromatische Carbonsäuren und aromatische Carbonsäureanhydride (z. B. Phthalsäureanhydrid und Benzoesäure), gegebenenfalls weitere Kohlenwasserstoffe, wie z. B. $C_4$-Kohlenwasserstoffe (z. B. Buten-1 und eventuell sonstige Butene) und gegebenenfalls weitere inerte Verdünnungsgase wie z. B. $N_2$).

Als Verfahren zur Abtrennung von im Produktgas B enthaltenem Zielprodukt, mit dem in der Regel gleichzeitig eine Abtrennung von Wasser und schwerer als Wasser siedenden Nebenkomponenten einhergeht, kommen für das erfindungsgemäße Verfahren in der zweiten Trennzone II prinzipiell alle im Stand der Technik diesbezüglich bekannten Verfahren in Betracht. Sie sind im wesentlichen dadurch gekennzeichnet, dass man das Zielprodukt z. B. durch absorptive und/oder kondensative Maßnahmen aus der gasförmigen in die kondensierte Phase überführt und anschließend die Kondensate bzw. Absorbate zum Zweck der weiteren Zielproduktabtrennung durch extraktive, destillative, kristallisative und/oder desorptive Maßnahmen aufarbeitet. Gemeinsam mit dem Zielprodukt und/oder auf die Überführung des Zielproduktes in die kondensierte Phase folgend werden bei diesen Verfahren üblicherweise auch im Reaktionsgas B enthaltener Wasserdampf und schwer als Wasser siedende Nebenkomponenten in die kondensierte Phase überführt und damit abgetrennt (erfindungsgemäß bevorzugt wird in der Trennzone II eine Wasserdampfmenge in die kondensierte Phase überführt, die wenigstens 70 mol-%, bevorzugt wenigsten 80 mol-%, besser wenigstens 90 mol-% und besonders bevorzugt wenigstens 95 mol-% der in der Reaktionszone B gebildeten (bzw. besonders bevorzugt der im Produktgas B insgesamt enthaltenen) Wasserdampfmenge beträgt).

Als Absorptionsmittel kommen dabei z. B. Wasser, wässrige Lösungen und/oder organische Lösungsmittel (z. B. Mischungen aus Diphenyl und Diphenylether oder aus Diphenyl, Diphenylether und o-Dimethylphthalat) in Betracht. Im Rahmen dieser "Kondensation" (Abtrennung) von Zielprodukt, Wasser und schwerer als Wasser siedenden Nebenkomponenten verbleibt normalerweise ein nicht in die kondensierte Phase übergehendes Restgas, das die vergleichsweise schwierig zu kondensierenden (leichter als Wasser siedenden) Bestandteile des Produktgases B umfasst. Dies sind üblicherweise vor allem diejenigen Komponenten, deren Siedepunkt bei Normaldruck (1 bar) $\leq$ -30°C beträgt (ihr Gesamtanteil am Restgas beträgt in der Regel $\geq$ 60 Vol.-%, häufig $\geq$ 70 Vol.-%, vielfach $\geq$ 80 Vol.-%, jedoch meist $\leq$ 90 Vol.-%). Dazu gehören in erster Linie nicht umgesetztes Propan, Kohlendioxid, gegebenenfalls nicht umgesetztes Propylen, gegebenenfalls nicht umgesetzter molekularer Sauerstoff, gebenenfalls molekularer Wasserstoff sowie sonstige leichter als Wasser siedende Nebenkomponenten wie z. B. CO, Ethan, Methan, gegebenenfalls $N_2$, gegebenenfalls Edelgase (z. B. He, Ne, Ar etc.). In geringem Umfang kann das Restgas auch noch Acrylsäure, Acrolein und/oder $H_2O$ enthalten. Erfindungsgemäß bevorzugt enthält das Restgas $\leq$ 10 Vol.-%, mit Vorteil $\leq$ 5 Vol.-% und mit besonderem Vorteil $\leq$ 3 Vol.-% Wasserdampf. Dieses vorgenannte Restgas bildet (bezogen auf die darin enthaltene Menge an Propan) normalerweise wenigstens die Hauptmenge (üblicherweise wenigstens 80%, bzw. wenigstens 90%, oder wenigstens 95% oder mehr) des in der zweiten Trennzone II gebildeten Restgases und wird in dieser Schrift als (Haupt)Restgas bezeichnet.

Insbesondere dann, wenn die Kondensation des Zielproduktes durch Absorption mittels eines organischen Lösungsmittels erfolgt, fällt in der Trennzone II in der Regel wenigstens ein zweites nicht umgesetztes Propan sowie gegebenenfalls nicht umgesetztes Propylen enthaltendes restliches Gas an (bezogen auf darin enthaltenes Propan ist seine Menge im Vergleich zur Menge an (Haupt)Restgas normalerweise wesentlich geringer (in der Regel $\leq$ 20%, meist $\leq$ 10%, oder $\leq$ 5%, oder $\leq$ 1%)). Dies ist darauf zurückzuführen, dass die sich bildende kondensierte Phase in gewissem Umfang auch nicht umgesetztes Propan sowie gegebenenfalls nicht umgesetztes Propylen aufnimmt.

Im weiteren Verlauf der extraktiven, destillativen, kristallisativen und/oder desorptiven Abtrennung des Zielproduktes (innerhalb der Trennzone II) aus der kondensierten Phase, wird dieses nicht umgesetzte Propan sowie gegebenenfalls Propylen normalerweise als Bestandteil wenigstens einer weiteren Gasphase rückgewonnen und in dieser Schrift als (Neben)Restgas bezeichnet.

Die Summe aus (Haupt)Restgas und (Neben)Restgas bildet in diesem Fall die in der Trennzone II insgesamt verbleibende Gesamtmenge an Restgas. Fällt kein (Neben)Restgas in der Trennzone II an, ist das (Haupt)Restgas automatisch die Gesamtmenge an Restgas (auch (Gesamt)Restgas genannt).

Erfindungsgemäß bevorzugt erfolgt die Überführung des Zielproduktes aus dem Produktgas B in die kondensierte Phase durch fraktionierende Kondensation. Dies insbesondere dann, wenn das Zielprodukt Acrylsäure ist. Grundsätzlich sind jedoch zur Zielproduktabtrennung z. B. alle in den Schriften DE-A 102 13 998, DE-A 22 63 496, US 3,433,840 (in vorgenannten Schriften beschriebene Verfahren sind insbesondere zur Acroleinabtrennung empfehlenswert), EP-A 1 388 533, EP-A 1 388 532, DE-A 102 35 847, EP-A 792 867, WO 98/01415, EP-A 1 015 411, EP-A 1 015 410, WO 99/50219, WO 00/53560, WO 02/09839, DE-A 102 35 847, WO 03/041833, DE-A 102 23 058, DE-A 102 43 625,DE-A 103 36 386, EP-A 854 129, US-A 4,317,926, DE-A 198 37 520, DE-A 196 06 877, DE-A 190 50 1325, DE-A 102 47 240, DE-A 197 40 253, EP-A 695 736, EP-A 982 287, EP-A 1 041 062, EP-A 1 171 46, DE-A 43 08 087, DE-A 43 35 172, DE-A 44 36 243, DE-A 199 24 532, DE-A 103 32 758 sowie DE-A 199 24 533 beschriebenen absorptiven und/oder kondensativen Verfahren zur "Zielproduktkondensation" und weiteren Aufarbeitung des "Kondensats" geeignet. Eine Acrylsäureabtrennung kann auch wie in der EP-A 982 287, der EP-A 982 289, der DE-A 103 36 386, der DE-A 101 15 277, der DE-A 196 06 877, der DE-A 197 40 252, der DE-A 196 27 847, der EP-A 920 408, der EP-A 1 068 174, der EP-A 1 066 239, der EP-A 1 066 240, der WO 00/53560, der WO 00/53561, der DE-A 100 53 086 und der EP-A 982 288 vorgenommen werden. Vorzugsweise wird wie in Fig. 7 der WO/0196271 bzw. wie in der DE-A 10 2004 032 129 und deren äquivalenten Schutzrechten beschrieben abgetrennt. Günstige Abtrennweisen sind auch die in den Schriften WO 04/063138, WO 04/35514, DE-A 102 43 625 und DE-A 102 35 847 beschriebenen Verfahren. Die Weiterverarbeitung einer dabei gewonnenen rohen Acrylsäure kann z. B. wie in den Schriften WO 01/77056, WO 03/041832, WO 02/055469, WO 03/078378 und WO 03/041833 beschrieben erfolgen.

Üblicherweise wird das Produktgas B der Trennzone II mit dem Arbeitsdruck zugeführt, mit dem es die Reaktionszone B verlässt.

Gemeinsames Merkmal der vorgenannten Trennverfahren ist (wie bereits erwähnt), dass z. B. am Kopf der jeweiligen trennwirksame Einbauten enthaltenden Trennkolonne, in deren unteren Teil das Produktgas B, normalerweise nach vorheriger direkter und/oder indirekter Kühlung desselben, z. B. zugeführt wird, normalerweise ein Strom an Restgas verbleibt, der hauptsächlich diejenigen Bestandteile des Produktgases B enthält, deren Siedepunkt bei Normaldruck (1 bar) tiefer als derjenige von Wasser liegt und meist ≤ -30°C beträgt (d. h., die schwer kondensierbaren oder auch leicht flüchtigen Bestandteile). Restgas kann aber z. B. auch noch Bestandteile wie Wasserdampf und Acrylsäure enthalten.

Im unteren Teil der Trennkolonne fallen normalerweise hauptsächlich die schwerer flüchtigen Bestandteile des Produktgases B, einschließlich des jeweiligen Zielprodukts, in kondensierter Phase an. Kondensierte wässrige Phase wird in der Regel über Seitenabzug und/oder über Sumpf entnommen.

In typischer Weise enthält (Haupt)Restgas nachfolgende Gehalte:

| | |
|---|---|
| 1 bis 20 Vol.-% | $H_2O$, |
| 0 bis 80 Vol.-% | $N_2$, |
| 10 bis 90 Vol.-% | Propan, |
| 0 bis 20 Vol.-% | $H_2$ |
| 0 bis 10 Vol.-% | $O_2$, |
| 1 bis 20 Vol.-% | $CO_2$ und |
| ≥ 0 bis 5 Vol.-% | CO. |

Der Gehalt an Acrolein und Acrylsäure beträgt in der Regel jeweils < 1 Vol.-%.

Bevorzugte (Haupt)Restgase enthalten:

| | |
|---|---|
| 1 bis 5 Vol.-% | $H_2O$, |
| 10 bis 80 Vol.-% | Propan, |
| 0 bis 5 Vol.-% | $N_2$, |
| 0 bis 20 Vol.-% | $H_2$, |
| 1 bis 10 Vol.-% | $O_2$, |
| 0 bis 5 Vol.-% | CO und |
| 1 bis 15 Vol.-% | $CO_2$. |

In typischer Weise verlässt das Restgas die Trennzone II beim erfindungsgemäßen Verfahren mit einem Druck von ≥ 1 bar und ≤ 3 bar, vorzugsweise ≤ 2,5 bar und in der Regel ≤ 2 bar. Die Temperatur des Restgases beträgt dabei normalerweise ≤ 100 °C, in der Regel ≤ 50 °C (üblicherweise jedoch ≥ 0 °C).

Die Absorption von in Restgas enthaltenem Propan erfolgt in der Trennzone III anwendungstechnisch zweckmäßig bei einem Druck von 5 bis 50 bar, vorzugsweise 10 bis 25 bar. In einer Verdichtungszone wird man das die Trennzone II verlassende Restgas normalerweise auf den für die absorbtive Abtrennung des Propans vorteilhaften Druck verdichten (vorteilhaft erfolgt dies mehrstufig). Erfindungsgemäß bevorzugt wird in einer ersten Verdichtungsstufe mit Hilfe eines mehrstufigen z. B. Turboverdichters (Radialverdichter; z. B. vom Typ MH4B, der Fa. Mannesmann DEMAG, DE) (hier auch Restgasverdichter genannt) eine Verdichtung des Restgases auf einen Arbeitsdruck durchgeführt, der demjenigen in der Reaktionszone A im wesentlichen entspricht (z. B. von 1,20 bar auf 2,40 bar; in der Regel erfolgt die Verdichtung in der ersten Verdichtungsstufe auf einen Druck, der etwas oberhalb des in der Reaktionszone A angewandten Arbeitsdruckes liegt). Dabei erwärmt sich das Restgas um beispielsweise bis zu 50 °C. Wird nicht die Gesamtmenge an Restgas der Propanabsorption unterzogen, teilt man das wie beschrieben verdichtete Restgas zweckmäßig in zwei Teilmengen identischer Zusammensetzung. Der Mengenanteil der kleineren Teilmenge kann sich dabei beispielsweise auf bis zu 49 %, vorzugsweise auf nicht mehr als bis zu 40 %, oder bis zu 30 %, oder bis zu 20 %, oder bis zu 10 % der Gesamtmenge belaufen. Er kann auch $\geq$ 10 % und $\geq$ 49 % der Gesamtmenge betragen.

Diese Teilmenge ist als Restgaskreisgas unmittelbar zur Rückführung in die Reaktionszone A bereit. Die andere Teilmenge wird auf den für die Propanabsorption ins Auge gefassten Arbeitsdruck weiterverdichtet. Dies kann prinzipiell in nur einer weiteren Verdichtungsstufe erfolgen. Erfindungsgemäß vorteilhaft wird man dazu jedoch wenigstens zwei weitere Verdichtungsstufen anwenden. Dies ist darauf zurückzuführen, dass mit der Verdichtung eines Gases (wie bei der ersten Verdichtungsstufe bereits angemerkt) eine Temperaturerhöhung desselben einhergeht. Umgekehrt geht mit der Entspannung (Expansion) von "Propan-gewaschenem" (und gegebenenfalls einer $H_2$-Membranabtrennung unterworfenem) Restgas eine Abkühlung desselben einher. Führt man eine solche Entspannung gleichfalls mehrstufig durch, kann man vor der jeweils nächsten Stufe einen indirekten Wärmeaustausch zwischen erwärmtem, verdichtetem ungewaschenem Restgas und abgekühltem, expandiertem "Propan-gewaschenem" Restgas vornehmen. Ein solcher Wärmeaustausch (der durch indirekte Luftkühlung des verdichteten Restgases zusätzlich unterstützt werden kann) kann auch bereits vor einer erstmaligen Expansion angewendet werden (als Folge des indirekten Wärmeaustauschs kann im Restgas Kondensationen von darin gegebenenfalls noch enthaltenem Wasserdampf eintreten). Vorgenannte Expansionen werden anwendungstechnisch vorteilhaft in (vorteilhaft gleichfalls mehrstufigen) Expansionsturbinen durchgeführt (dies dient der Rückgewinnung von Verdichtungsenergie; d. h., die bei der Entspannung gewonnene mechanische Energie kann in allen Fällen (insbesondere in den Beispielen und Vergleichsbeispielen) sowohl direkt als Mit- oder Hauptantrieb für einen der Verdichter und/oder zur Erzeugung von Strom genutzt werden). Je Verdichtungs- bzw. Expansionsstufe kann der Unterschied zwischen Eingangs- und Ausgangsdruck z. B. 2 bis 15 bar betragen.

Bevor aus dem verdichteten Restgas das darin enthaltene Propan in einer Trennzone III absorptiv in ein organisches Lösungsmittel aufgenommen wird (mit Hilfe eines organischen Lösungsmittels (Absorptionsmittels) aus dem verdichteten Restgas ausgewaschen wird), kann das verdichtete Restgas in einer weiteren Trennzone noch einer Membrantrennung unterworfen werden, um wenigstens eine Teilmenge des darin gegebenenfalls noch enthaltenen molekularen Wasserstoffs abzutrennen. Diesbezüglich geeignete Trennmembranen sind z. B. aromatische Polyimidmembranen, z. B. diejenigen von UBE Industries Ltd.. Unter letzteren kommen insbesondere die Membrantypen A, B-H, C und D in Betracht. Besonders bevorzugt ist für eine solche Abtrennung die Anwendung einer UBE Industries Ltd. Polyimidmembran vom Typ B-H. Die Wasserstoffpermeationsrate für $H_2$ beträgt für diese Membran bei 60°C $0{,}7{\cdot}10^{-3}$ [STP·cc/cm$^2$·sec·cm Hg]. Zu diesem Zweck kann das verdichtete Restgas über die, in der Regel zu einem Rohr (in Betracht kommt aber auch ein Platten- oder ein Wickelmodul) gestaltete Membran geleitet werden, die lediglich für den molekularen Wasserstoff durchlässig ist. Der so abgetrennte molekulare Wasserstoff kann im Rahmen anderer chemischer Synthesen weiterverwendet oder wenigstens teilweise in die Reaktionszone A (zweckmäßig als Bestandteil des Reaktionsgas A-Beschickungsgasgemischs) rückgeführt werden.

Die Membranabtrennung von molekularem Wasserstoff aus verdichtetem Restgas ist deshalb erfindungsgemäß zweckmäßig, weil sie vorzugsweise ebenfalls unter hohem Druck (z. B. 5 bis 50 bar, typisch 10 bis 25 bar) durchgeführt wird. Es ist deshalb erfindungsgemäß vorteilhaft, eine gegebenenfalls durchzuführende Wasserstoffmembranabtrennung aus Restgas entweder unmittelbar vor der Propanabsorption (Propanwäsche) oder, alternativ (und erfindungsgemäß bevorzugt), unmittelbar nach der Propanwäsche (Propanabsorption) des Restgases durchzuführen, um das einmal erhöhte Druckniveau 2-fach in vorteilhafter Weise zu nutzen. Neben Plattenmembranen, Wickelmembranen oder Kapillarmembranen kommen für die Wasserstoffabtrennung vor allem Schlauchmembranen (Hohlfasermembranen) in Betracht. Ihr Innendurchmesser kann z. B. wenige $\mu$m bis wenige mm betragen. Analog den Rohren eines Rohrbündelreaktors wird dazu z. B. ein Bündel derartiger Schlauchmembranen an den beiden Schlauchenden in jeweils eine Platte eingegossen. Außerhalb der Schlauchmembran herrscht vorzugsweise reduzierter Druck (< 1 bar). Das unter erhöhtem Druck befindliche Restgas (bzw. von "Propan gewaschene" Restgas) wird gegen eines der beiden Plattenenden geführt und durch das Schlauchinnere hindurch zum am gegenüber liegenden Plattenende befindlichen Schlauchausgang gedrückt. Entlang des so im Schlauchinneren vorgegebenen Strömungsweges wird über die $H_2$-durchlässige Membran molekularer Wasserstoff nach außen abgegeben.

Die Durchführung der Absorption des Propans (gemeinsam mit dem Propan wird in der Regel im Rahmen der Partialoxidation in der Reaktionszone B nicht umgesetztes Propylen absorbiert) aus dem verbliebenen Restgas unterliegt im wesentlichen keinerlei Beschränkungen. Eine einfache Möglichkeit dazu besteht z. B. darin, das verbliebene Restgas (das erfindungsgemäß zweckmäßig eine Temperatur von 10 bis 100°C, vorzugsweise 10 bis 70°C aufweist) bei einem Druck von 5 bis 50 bar, vorzugsweise 10 bis 25 bar mit einem organischen Lösungsmittel (vorzugsweise ein hydrophobes), dessen Temperatur mit Vorteil 0 bis 100°C, vorzugsweise 20 bis 50°C, bzw. bis 40°C, beträgt und in welchem Propan und Propylen (gegenüber den anderen Bestandteilen des verbliebenen Restgases zweckmäßig bevorzugt) absorbiert werden, in Kontakt zu bringen (z. B. durch einfaches Durchleiten).

Beispielsweise durch nachfolgende Desorption (Entspannungsverdampfung), Rektifikation und/oder Strippung mit einem bezüglich der Reaktionszone A sich inert verhaltenden (z. B. $N_2$) und/oder in dieser Reaktionszone als Reaktand benötigten Gas (z. B. Luft oder ein anderes Gemisch aus molekularem Sauerstoff und Inertgas; bevorzugt Wasserdampf oder ein Gemisch aus Wasserdampf und molekularem Sauerstoff, bzw. ein Gemisch aus Wasserdampf und Luft) können in einer Trennzone IV das Propan und gegebenenfalls Propylen im Gemisch aus dem Absorbat abgetrennt und als Propan enthaltender Zufuhrstrom in die Reaktionszone A rückgeführt werden.

Als Absorptionsmittel für die vorstehend beschriebene absorptive Abtrennung eignen sich grundsätzlich alle Absorptionsmittel, die in der Lage sind, Propan und Propylen zu absorbieren. Bei dem Absorptionsmittel handelt es sich vorzugsweise um ein organisches Lösungsmittel, das hydrophob und/oder hochsiedend ist. Vorteilhafterweise hat dieses Lösungsmittel einen Siedepunkt (bei einem Normaldruck von 1 bar) von mindestens 120°C, bevorzugt von mindestens 180°C, vorzugsweise von 200 bis 350°C, insbesondere von 250 bis 300°C, stärker bevorzugt von 260 bis 290°C. Zweckmäßigerweise liegt der Flammpunkt (bei einem Normaldruck von 1 bar) über 110°C. Allgemein eignen sich als Absorptionsmittel relativ unpolare organische Lösungsmittel, wie zum Beispiel aliphatische Kohlenwasserstoffe, die vorzugsweise keine nach außen wirkende polare Gruppe enthalten, aber auch aromatische Kohlenwasserstoffe. Allgemein ist es erwünscht, dass das Absorptionsmittel einen möglichst hohen Siedepunkt bei gleichzeitig möglichst hoher Löslichkeit für Propan und Propylen hat. Beispielhaft als Absorptionsmittel genannt seien aliphatische Kohlenwasserstoffe, zum Beispiel $C_8$-$C_{20}$-Alkane oder -Alkene, oder aromatische Kohlenwasserstoffe, zum Beispiel Mittelölfraktionen aus der Paraffindestillation oder Ether mit sperrigen (sterisch anspruchvollen) Gruppen am O-Atom, oder Gemische davon, wobei diesen ein polares Lösungsmittel, wie zum Beispiel das in der DE-A 43 08 087 offenbarte 1,2-Dimethylphthalat zugesetzt sein kann. Weiterhin eignen sich Ester der Benzoesäure und Phthalsäure mit geradkettigen, 1 bis 8 Kohlenstoffatome enthaltenden Alkanolen, wie Benzoesäuren-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkene, zum Beispiel 4-Methyl-4'-benzyl-diphenylmethan und dessen Isomere 2-Methyl-2'-benzyl-diphenyl-methan, 2-Methyl-4'-benzyldiphenyl-methan und 4-Methyl-2'-benzyl-diphenylmethan und Gemische solcher Isomere. Ein geeignetes Absorptionsmittel ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether, beispielsweise das im Handel erhältliche Diphyl® (z. B. von der Bayer Aktiengesellschaft bezogenes). Häufig enthält dieses Lösungsmittelgemisch ein Lösungsmittel wie Dimethylphthalat in einer Menge von 0,1 bis 25 Gew.-%, bezogen auf das gesamte Lösungsmittelgemisch, zugesetzt. Besonders geeignete Absorptionsmittel sind auch Octane, Nonane, Decane, Undecane, Dodecane, Tridecane, Tetradecane, Pentadecane, Hexadecane, Heptadecane und Octadecane, wobei sich insbesondere Tetradecane als besonders geeignet erwiesen haben. Günstig ist es, wenn das verwendete Absorptionsmittel einerseits den oben genannten Siedepunkt erfüllt, andererseits gleichzeitig aber ein nicht zu hohes Molekulargewicht aufweist. Mit Vorteil beträgt das Molekulargewicht des Absorptionsmittels $\leq$ 300 g/mol. Geeignet sind auch die in der DE-A 33 13 573 beschriebenen Paraffinöle mit 8 bis 16 Kohlenstoffatomen. Beispiele für geeignete Handelsprodukte sind von der Firma Haltermann vertriebene Produkte wie Halpasole i, wie Halpasol 250/340 i und Halpasol 250/275 i, sowie Druckfarbenöle unter den Bezeichnungen PKWF und Printosol. Bevorzugt sind an Aromaten freie Handelsprodukte, z. B. solche des Typs PKWFaf. Falls sie einen geringen Restaromatengehalt enthalten, kann dieser vorab des beschriebenen Einsatzes vorteilhaft rektifikativ und/oder adsorptiv gemindert und auf Werte signifikant unter 1000 gew.ppm gedrückt werden. Weitere geeignete Handelsprodukte sind n-Paraffin ($C_{13}$-$C_{17}$) oder Mihagol®5 der Erdöl-Raffinerie-Emsland GmbH, LINPAR®14-17 der Firma CONDEA Augusta S.p.A. (Italien) oder der SASOL Italy S.p.A., Normal parrafins (heavy) $C_{14}$-$C_{18}$ der Firma SLONAFT in der Slowakei.

Die Gehalte (angegeben in Flächenprozenten der gaschromatographischen Analyse) der vorgenannten Produkte an linearen Kohlenwasserstoffen betragen in typischer Weise:

Summe $C_9$ bis $C_{13}$: weniger als 1 %; $C_{14}$: 30 bis 40 %; $C_{15}$: 20 bis 33 %; $C_{16}$: 18 bis 26 %; $C_{17}$: bis zu 18 %; $C_{\geq 18}$: < 2 %.

Eine typische Zusammensetzung des Produktes der Fa. SASOL ist:

$C_{13}$: 0,48 %; $C_{14}$: 39,8 %; $C_{15}$: 20,8 %; $C_{16}$: 18,9 %; $C_{17}$: 17,3 %; $C_{18}$: 0,91 %; $C_{19}$: 0,21 %.

Eine typische Zusammensetzung des Produktes der Fa. Haltermann ist:

$C_{13}$: 0,58 %; $C_{14}$: 33,4 %; $C_{15}$: 32,8 %; $C_{16}$: 25,5 %; $C_{17}$: 6,8 %; $C_{\geq 18}$: < 0,2 %.

Im kontinuierlichen Betrieb wird sich die Zusammensetzung des Absorptionsmittels verfahrensbedingt entsprechend ändern.

Die Absorption kann sowohl in Kolonnen als auch in Quenchapparaten vorgenommen werden. Dabei kann im Gleichstrom oder im Gegenstrom (ist bevorzugt) gearbeitet werden. Geeignete Absorptionskolonnen sind zum Beispiel Bodenkolonnen (mit Glocken- und/oder Siebböden), Kolonnen mit strukturierten Packungen (zum Beispiel Blechpackungen mit einer spezifischen Oberfläche von 100 bis 1000, oder bis 750 m²/m³, zum Beispiel Mellapak® 250 Y) und Füllkörperkolonnen (zum Beispiel mit Raschig-Füllkörpern gefüllte). Es können aber auch Riesel- und Sprühtürme, Graphitblockabsorber, Oberflächenabsorber wie Dickschicht- und Dünnschichtabsorber sowie Tellerwäscher, Kreuzschleierwäscher und Rotationswäscher verwendet werden. Zudem kann es günstig sein, die Absorption in einer Blasensäule mit und ohne Einbauten stattfinden zu lassen.

Die Abtrennung des Propans und gegebenenfalls Propylens von dem Absorptionsmittel (aus dem Absorbat) kann durch Strippung, Entspannungsverdampfung (Flashen) und/oder Destillation (Rektifikation) erfolgen.

Die Abtrennung des Propans und Propylens von dem Absorptionsmittel erfolgt vorzugsweise durch Strippen und/oder Desorption. Die Desorption kann in üblicher Weise über eine Druck- und/oder Temperaturänderung durchgeführt werden, z. B. bei einem Druck von 0,1 bis 10 bar, oder 1 bis 5 bar, oder 1 bis 3 bar, und einer Temperatur von 0 bis 200°C, insbesondere 20 bis 100°C, stärker bevorzugt 30 bis 70°C, besonders bevorzugt 30 bis 50°C. Ein für die Strippung bevorzugtes Stripgas ist Wasserdampf. Alternativ kommt molekularer Stickstoff oder ein Gemisch aus molekularem Stickstoff und Wasserdampf in Betracht. Aber auch Kohlendioxid oder dessen Gemische mit Wasserdampf und/oder Stickstoff sind erfindungsgemäß geeignete Stripgase. Besonders geeignet für die Strippung sind auch Blasensäulen mit und ohne Einbauten.

Die Abtrennung des Propans und gegebenenfalls Propylens von dem Absorptionsmittel (vom Absorbat) kann auch über eine Destillation bzw. Rektifikation erfolgen, wobei die dem Fachmann geläufigen Kolonnen mit Packungen, Füllkörpern oder entsprechenden Einbauten verwendet werden können. Mögliche Bedingungen bei der Destillation bzw. Rektifikation sind ein Druck von 0,01 bis 5 bar, oder 0,1 bis 4 bar, oder 1 bis 3 bar, und eine Temperatur (im Sumpf) von 50 bis 300°C, insbesondere 150 bis 250°C.

Ein durch Strippen aus dem Absorbat gewonnenes Propankreisgas kann vor seiner Verwendung zur Beschickung der Reaktionszone A in der Trennzone IV noch einer weiteren Verfahrensstufe zugeführt werden, um z. B. die Verluste an mitgestripptem Absorptionsmittel zu verringern (z. B. Abscheidung in Demistern und/oder Tiefenfiltern) und die Reaktionszone so gleichzeitig vor Absorptionsmittel zu schützen. Eine solche Abtrennung des Absorptionsmittels kann nach allen dem Fachmann bekannten Verfahrensvarianten erfolgen. Eine im Rahmen des erfindungsgemäßen Verfahrens bevorzugte Ausführungsform einer solchen Abtrennung ist z. B. das Quenchen des Ausgangsstromes aus der Strippvorrichtung mit Wasser. In diesem Fall wird das Absorptionsmittel aus diesem beladenen Ausgangsstrom mit Wasser herausgewaschen und der Ausgangsstrom gleichzeitig mit Wasser beladen. Diese Wäsche, bzw. das Quenchen kann z. B. am Kopf einer Desorptionskolonne über einen Flüssigkeits-Fangboden durch Gegensprühen von Wasser oder in einem eigenen Apparat erfolgen. Zur Unterstützung des Trenneffektes können im Quenchraum die Quenchoberfläche vergrößernde Einbauten installiert werden, wie sie dem Fachmann von Rektifikationen, Absorptionen und Desorptionen her bekannt sind.

Eine Entspannung des das Propan (und gegebenenfalls Propylen) enthaltenden Absorbats auf den für die Abtrennung des Propans (und gegebenenfalls Propylens) adäquaten Druck kann beim erfindungsgemäßen Verfahren z. B. mittels eines Ventils oder mittels einer inversen Pumpe ausgeführt werden. Die im Fall der inversen Pumpe dabei freigesetzte mechanische Energie wird erfindungsgemäß zweckmäßig zur Rückverdichtung von (z. B. in der Desorptionskolonne oder in der Stripkolonne) von Propan (und gegebenenfalls Propylen) befreitem Absorptionsmittel mitverwendet. Generell kann von Propan befreites Absorbat als (erfindungsgemäß zweckmäßig mittels einer Pumpe auf den Absorptionsdruck rückverdichtetes) Absorptionsmittel zur Abtrennung von Propan aus Restgas in der Trennzone III wiederverwendet werden.

Erfindungsgemäß besonders vorteilhaft erfolgt die Abtrennung des Propans aus dem Absorbat in der Trennzone IV bei einem Druck von 1,5 bzw. 2 bis 5 bar, vorzugsweise > 2 bis 5 bar, besonders bevorzugt 2,5 bis 4,5 bar und ganz besonders bevorzugt 3 bis 4 bar.

Eine Propanabtrennung aus dem Absorbat bei vorgenannten Arbeitsdrücken ist deshalb ganz besonders vorteilhaft, weil bei diesen Arbeitsdrücken erzeugtes Propankreisgas unmittelbar in die Reaktionszone A rückgeführt werden kann. Dabei kann solches Propankreisgas als Treibstrahl für eine Dehydrierkreisgasführung nach dem Strahlpumpenprinzip gemäß der Lehre der DE-A 102 11 275 verwendet werden. Erfindungsgemäß zweckmäßig wird das Propankreisgas dabei vorab durch indirekten Wärmeaustausch mit heißem Reaktionsgas A und/oder heißem Produktgas A erfindungsgemäß zweckmäßig auf der Reaktionszone A angemessene Temperaturen erwärmt.

Typische Gehalte von durch Desorption und/oder Strippen mittels Wasserdampf in der Trennzone IV aus dem Propan enthaltenden Absorbat erzeugte Propankreisgase können beim erfindungsgemäßen Verfahren die folgenden sein:

| | |
|---|---|
| Propan | 80 bis 99,99 mol-%, |
| Propylen | 0 bis 5 mol-%, und |
| $H_2O$ | 0 bis 20 mol-%. |

Vielfach betragen die Gehalte von Propankreisgas:

| | |
|---|---|
| Propan | 80 bis 99 mol-%, |
| Propylen | 1 bis 4 mol-%, und |

(fortgesetzt)

| | |
|---|---|
| $H_2O$ | 2 bis 15 mol-%. |

Für den Fall, dass der Reaktionszone A neben Frischpropan (als Bestandteil von Roh-Propan) und Propankreisgas sowie gegebenenfalls Dehydrierkreisgas zusätzlich (zweckmäßig verdichtetes) Restgaskreisgas und/oder ein (anderes) molekularen Sauerstoff enthaltendes Gas zugeführt wird, werden das Roh-Propan sowie das Restgaskreisgas und/oder das molekularen Sauerstoff enthaltende Gas anwendungstechnisch zweckmäßig vorab zu einem ersten Gasgemisch vermischt und dieses erste Gasgemisch (vorzugsweise nach seiner Erwärmung durch indirekten Wärmeaustausch mit heißem Reaktionsgas A und/oder Produktgas A) mit Propankreisgas oder dessen Gemisch mit Dehydrierkreisgas (vorzugsweise ebenfalls nach entsprechender Erwärmung durch indirekten Wärmeaustausch mit heißem Reaktionsgas A und/oder Produktgas A) gemischt und das dabei resultierende Gasgemisch als Reaktionsgas A-Beschickungsgasgemisch in die Reaktionszone A geführt. Mit Vorteil wird dem dabei resultierenden Gasgemisch noch molekularer Wasserstoff zudosiert, bevor es als Reaktionsgas A-Beschickungsgasgemisch in die Reaktionszone A geführt wird.

Der Bedarf an molekularem Sauerstoff enthaltendem Gas wird beim erfindungsgemäßen Verfahren anwendungstechnisch zweckmäßig aus einer gemeinsamen Quelle entnommen (gedeckt).

Die Verdampfung des Roh-Propan erfolgt erfindungsgemäß zweckmäßig mittels in der Trennzone II anfallendem Kondensat (z. B. Sauerwasser) durch indirekten Wärmeaustausch (das dabei abgekühlte Kondensat kann in der Trennzone II zur Direktkühlung eingesetzt werden, um neues Kondensat zu gewinnen). Alternativ kann auch andere Flüssigphase (die zweckmäßig eine Temperatur von 20 bis 40°C, vorzugsweise von 25 bis 35°C aufweist) als Wärmeträger für den indirekten Wärmeaustausch zum Zweck der Verdampfung von Roh-Propan verwendet werden. Zur weiteren Erwärmung des Roh-Propan wird in der Regel indirekter Wärmeaustausch mit heißem Wasserdampf (der z. B. mit 5 bar und 152°C zur Verfügung stehen kann) angewendet, der üblicherweise beim erfindungsgemäßen Verfahren reichlich als Beiprodukt anfällt (z. B. im Rahmen der Abfuhr der im Rahmen der Partialoxidation anfallenden Prozesswärme).

Bei Bedarf wird aus der Trennzone IV kontinuierlich eine geringe Menge an Absorptionsmittel ausgeschleust und durch frisches Absorptionsmittel ergänzt. Das ausgeschleuste Absorptionsmittel kann rektifikativ zu frischem Absorptionsmittel aufgearbeitet werden. Das wie bereits beschrieben entspannte, zuvor "Propan-gewaschene" und nachfolgend gegebenenfalls einer Membranwasserstoffabtrennung unterworfene Restgas, wird in der Regel einer Rückstandsverbrennung (Abgasverbrennung) zugeführt. In diese Verbrennung, die gegebenenfalls durch Zufütterung von Erdgas unterstützt und unter Verwendung von Luft als Quelle für Verbrennungssauerstoff ausgeführt wird, können auch in der Trennzone II abgetrennte Schwersieder sowie in der Trennzone II sowie gegebenenfalls zwischen der Trennzone II und der Trennzone III gebildete wässrige Kondensate (z. B. Sauerwasser) geführt werden. Das heiße Abgas dieser Verbrennung wird üblicherweise im indirekten Wärmeaustausch mit Wasser zur Erzeugung von Wasserdampf verwendet und nachfolgend in der Regel in die Atmosphäre entlassen. Normalerweise ist es ausschließlich aus molekularem Sauerstoff, molekularem Stickstoff, molekularem Wasserstoff und Kohlendioxid zusammengesetzt. Schließlich sei noch festgehalten, dass beim erfindungsgemäßen Verfahren immer dann, wenn Acrylsäure und/oder Acrolein enthaltende kondensierte Phasen auftreten, den selben Polymerisationsinhibitor zugesetzt wird. Als solche kommen grundsätzlich alle bekannten Prozessinhibitoren in Betracht. Erfindungsgemäß besonders geeignet sind z. B. Phenothiazin und der Methylether des Hydrochinons. Durch ein Beisein von molekularem Sauerstoff wird die Wirksamkeit der Polymerisationsinhibitoren erhöht.

Nach dem erfindungsgemäßen Verfahren erzeugtes Acrolein kann zu den in den Schriften US-A 6,166,263 und US-A 6,187,963 genannten Acroleinfolgeprodukten umgesetzt werden. Dazu zählen 1,3-Propandiol, Methionin, Glutaraldehyd sowie 3-Picolin.

Bevorzugt sind erfindungsgemäße Verfahren, die in der Trennzone III den maximalen Arbeitsdruck aufweisen.

Mit Vorteil gelten für die Arbeitsdrücke P (jeweils bestimmt am Eingang in die jeweilige Zone) in den verschiedenen Zonen des erfindungsgemäßen Verfahrens nachfolgende Korrelationen (Beziehungen):

$$P_{\text{Reaktionszone A}} > P_{\text{Trennzone I}} > P_{\text{Reaktionszone B}} > P_{\text{Trennzone II}} < P_{\text{Trennzone III}} > P_{\text{Trennzone IV}} > P_{\text{Reaktionszone A}},$$

wobei die Trennzone I auch entfallen kann.

Ferner ist es für das erfindungsgemäße Verfahren vorteilhaft, die Temperatur von Reaktionsgasen A, insbesondere dann, wenn diese molekularen Sauerstoff enthalten, außerhalb von Katalysator- und/oder Inertschüttungen bei Werten $\leq 460$ °C, vorzugsweise $\leq 440$ °C zu halten, um so unerwünschte Verbrennungsreaktionen sowie thermische Zersetzungen, insbesondere des Propans, zu minimieren.

Beispiele und Vergleichsbeispiele

I. Langzeitbetrieb einer heterogen katalysierten zweistufigen Partialoxidation von Propylen zu Acrylsäure in Abwesenheit und im Beisein von molekularem Wasserstoff

A) Allgemeiner Versuchsaufbau der Reaktionsvorrichtung

Reaktor für die erste Oxidationsstufe

Der Reaktor bestand aus einem doppelwandigen Zylinder aus Edelstahl (zylindrisches Führungsrohr, umgeben von einem zylindrischen Außenbehälter). Die Wanddicken betrugen überall 2 bis 5 mm.
Der Innendurchmesser des äußeren Zylinders betrug 91 mm. Der Innendurchmesser des Führungsrohres betrug ca. 60 mm.
Oben und unten war der doppelwandige Zylinder durch einen Deckel beziehungsweise Boden abgeschlossen.
Das Kontaktrohr (400 cm Gesamtlänge, 26 mm Innendurchmesser, 30 mm Außendurchmesser, 2 mm Wandstärke, Edelstahl) war im Führungsrohr des zylindrischen Behälters so untergebracht, dass es am oberen bzw. unteren Ende desselben (abgedichtet) durch den Deckel bzw. Boden jeweils gerade herausragte. Das Wärmeaustauschmittel (Salzschmelze, bestehend aus 53 Gew.-% Kaliumnitrat, 40 Gew.-% Natriumnitrit und 7 Gew.-% Natriumnitrat) war im zylindrischen Behälter eingeschlossen. Um über die gesamte im zylindrischen Behälter befindliche Kontaktrohrlänge (400 cm) möglichst gleichmäßige thermische Randbedingungen an der Außenwand des Kontaktrohres zu gewährleisten, wurde das Wärmeaustauschmittel mittels einer Propellerpumpe umgepumpt.
Durch eine auf den Außenmantel aufgebrachte elektrische Heizung konnte die Temperatur des Wärmeaustauschmittels auf das gewünschte Niveau geregelt werden. Im übrigen bestand Luftkühlung.

| | |
|---|---|
| Reaktorbeschickung: | Über den Erststufenreaktor betrachtet wurden die Salzschmelze und das Beschickungsgasgemisch des Erststufenreaktors im Gleichstrom geführt. Das Beschickungsgasgemisch trat unten in den Erststufenreaktor ein. Es wurde jeweils mit einer Temperatur von 165°C ins Reaktionsrohr geführt. |
| | Die Salzschmelze trat unten mit einer Temperatur $T^{ein}$ in das zylindrische Führungsrohr ein und oben mit einer Temperatur $T^{aus}$ aus dem zylindrischen Führungsrohr aus, die bis zu 2°C oberhalb von $T^{ein}$ lag. |
| | $T^{ein}$ wurde so eingestellt, dass sich am Ausgang der ersten Oxidationsstufe stets ein Propylenumsatz von 97,8 $\pm$ 0,1 mol-% bei einfachem Durchgang ergab. |
| Kontaktrohrbeschickung: (von unten nach oben) | Abschnitt A: 90 cm Länge Vorschüttung aus Steatit-Kugeln eines Durchmessers von 4-5 mm. |
| | Abschnitt B: 100 cm Länge Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt C. |
| | Abschnitt C: 200 cm Länge Katalysatorbeschickung mit ringförmigem (5mm x 3 mm x 2 mm = Außendurchmesser x Länge x Innendurchmesser) Vollkatalysator gemäß Beispiel 1 der DE-A 100 46 957 (Stöchiometrie: $[Bi_2W_2O_9 x 2WO_3]_{0,5}$ $[Mo_{12}Co_{5,5}Fe_{2,94}Si_{1,59}K_{0,08}O_x]_1$). |
| | Abschnitt D: 10 cm Länge Nachschüttung aus Steatit-Ringen der Geometrie 7 mm x 3 mm x 4 mm (Außendurchmesser x Länge x Innendurchmesser) |

Zwischenkühlung und Sauerstoffzwischeneinspeisung (reiner $O_2$ als Sekundärgas)
Das den ersten Festbettreaktor verlassende Produktgasgemisch wurde zum Zweck der Zwischenkühlung (indirekt mittels Luft) durch ein Verbindungsrohr (40 cm Länge, 26 mm Innendurchmesser, 30 mm Außendurchmesser, 2 mm Wandstärke, Edelstahl, umwickelt mit 1 cm Isoliermaterial) geführt, das auf einer Länge von 20 cm zentriert untergebracht, mit einer Inertschüttung aus Steatit-Ringen der Geometrie 7 mm x 3 mm x 4 mm (Außendurchmesser x Länge x Innendurchmesser) beschickt und unmittelbar an das Erststufenkontaktrohr angeflanscht war.

Das Produktgasgemisch trat stets mit einer Temperatur von > $T^{ein}$ (erste Stufe) in das Verbindungsrohr ein und verließ es mit einer oberhalb von 200°C und unterhalb von 270°C gelegenen Temperatur.

Am Ende des Verbindungsrohres wurde dem abgekühlten Produktgasgemisch auf dem Druckniveau des Produktgasgemisches befindlicher molekularer Sauerstoff zudosiert. Das resultierende Gasgemisch (Beschickungsgasgemisch für die zweite Oxidationsstufe) wurde unmittelbar in das Zweitstufenkontaktrohr geführt, an welchem das oben genannte Verbindungsrohr mit seinem anderen Ende ebenfalls angeflanscht war. Die Menge an zudosiertem molekularem Sauerstoff wurde so bemessen, dass das molare Verhältnis von im resultierenden Gasgemisch befindlichen $O_2$ zu im resultierenden Gasgemisch befindlichen Acrolein 1,3 betrug.

Reaktor für die zweite Oxidationsstufe

Es wurde ein Kontaktrohr-Festbettreaktor verwendet, der mit jenem für die erste Oxidationsstufe baugleich war. Salzschmelze und Beschickungsgasgemisch wurden über den Reaktor betrachtet im Gleichstrom geführt. Die Salzschmelze trat unten ein, das Beschickungsgasgemisch ebenfalls. Die Eintrittstemperatur $T^{ein}$ der Salzschmelze wurde so eingestellt, dass sich am Ausgang der zweiten Oxidationsstufe stets ein Acroleinumsatz von 99,3 ± 0,1 mol-% bei einfachem Durchgang ergab. $T^{aus}$ der Salzschmelze lag bis zu 2 °C oberhalb von $T^{ein}$.

Die Kontaktrohrbeschickung (von unten nach oben) war:

Abschnitt A:     70 cm Länge
Vorschüttung aus Steatit-Ringen der Geometrie 7 mm x 3 mm x 4 mm (Außendurchmesser x Länge x Innendurchmesser).

Abschnitt B:     100 cm Länge
Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschnitt C.

Abschnitt C:     200 cm Länge
Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: $Mo_{12}V_3W_{1,2}Cu_{2,4}O_x$).

Abschnitt D:     30 cm Länge
Nachschüttung aus Steatit-Kugeln eines Durchmessers von 4-5 mm.

B) In Abhängigkeit von der Zusammensetzung des Beschickungsgasgemischs der ersten Oxidationsstufe erzielte Ergebnisse (die Propenbelastung wurde auf 150 Nl/l•h eingestellt, die Selektivität der Acrylsäurebildung betrug stets ≥ 94 mol-%).

a) Die Zusammensetzung des Beschickungsgasgemischs für die erste Oxidationsstufe betrug im wesentlichen:

7 Vol.-%     Propylen,
12 Vol.-%    $O_2$,
20 Vol.-%    $H_2$,
5 Vol.-%     $H_2O$ und
56 Vol.-%    $N_2$.

Zu Beginn der frisch mit Katalysator beschickten Reaktionsvorrichtung betrugen die Eingangstemperaturen:

$T^{ein}$ (erste Oxidationsstufe):     320°C
$T^{ein}$ (zweite Oxidationsstufe):    268°C

Nach 3-monatiger Betriebsdauer betrugen die Eingangstemperaturen:

$T^{ein}$ (erste Oxidationsstufe): 330°C

$T^{ein}$ (zweite Oxidationsstufe): 285°C

b) Die Zusammensetzung des Beschickungsgasgemischs für die erste Oxidationsstufe betrug im wesentlichen:

7 Vol.-% Propylen,

12 Vol.-% $O_2$,

5 Vol.-% $H_2O$ und

76 Vol.-% $N_2$.

Zu Beginn der frisch mit Katalysator beschickten Reaktionsvorrichtung betrugen die Eingangstemperaturen:

$T^{ein}$ (erste Oxidationsstufe): 320°C

$T^{ein}$ (zweite Oxidationsstufe): 268°C

Nach 3-monatiger Betriebsdauer betrugen die Eingangstemperaturen:

$T^{ein}$ (erste Oxidationsstufe): 324°C

$T^{ein}$ (zweite Oxidationsstufe):276°C

II. Erstes beispielhaftes Verfahren zur Herstellung von Acrylsäure aus Propan (beschrieben wird ein stationärer Betriebszustand)

Die Reaktionszone A besteht aus einem als Hordenreaktor ausgeführten und adiabat gestalteten Schachtofenreaktor, der drei in Strömungsrichtung hintereinander angeordnete Katalysatorfestbetten aufweist. Das jeweilige Katalysatorfestbett ist eine auf einem Edelstahldrahtnetz aufgebrachte Schüttung aus (in Strömungsrichtung in der genannten Reihenfolge angeordnet) Inertmaterial (Schütthöhe: 26 mm; Steatitkugeln des Durchmessers 1,5 bis 2,5 mm) und einer Mischung aus frischem Dehydrierkatalysator und Steatitkugeln (1,5 bis 2,5 mm Durchmesser) im Schüttvolumenverhältnis Dehydrierkatalysator : Steatitkugeln = 1 : 3 (alternativ kann an dieser Stelle auch die gleiche Katalysatormenge aber unverdünnt eingesetzt werden).

Vor jedem Festbett befindet sich ein statischer Gasmischer. Der Dehydrierkatalysator ist eine Pt/Sn-Legierung, die mit den Elementen Cs, K und La in oxidischer Form promoviert ist und die auf die äußere und innere Oberfläche von $ZrO_2 \cdot SiO_2$ Mischoxidträgerstränglingen (mittlere Länge (gaußverteilt im Bereich von 3 mm bis 12 mm mit Maximum bei ca. 6 mm): 6 mm, Durchmesser: 2 mm) in der Elementstöchiometrie (Massenverhältnis einschließlich Träger) $Pt_{0,3}Sn_{0,6}La_{3,0}Cs_{0,5}K_{0,2}(ZrO_2)_{88,3}(SiO_2)_{7,1}$ aufgebracht ist (Katalysatorvorläuferherstellung und Aktivierung zum aktiven Katalysator wie in Beispiel 4 der DE-A 10219879).

Die heterogen katalysierte partielle Propandehydrierung wird im beschriebenen Hordenreaktor im geraden Durchgang mit Dehydrierkreisgasfahrweise durchgeführt. Die Belastung der Katalysatorgesamtmenge (ohne Inertmaterial gerechnet) aller Horden mit Propan beträgt 1500 Nl/l·h.

Dem in Strömungsrichtung ersten Katalysatorbett werden 7,30 Nm³/h Reaktionsgas A-Beschickungsgasgemisch (Ausgangsgemisch; P = 2,13 bar) zugeführt, das folgende Gehalte aufweist:

Propan 61,22 Vol.-%,

Propylen 12,17 Vol.-%,

Ethylen 0,86 Vol.-%,

Methan 2,56 Vol.-%,

$H_2$ 3,78 Vol.-%,

$O_2$ 2,10 Vol.-%,

$H_2O$ 12,08 Vol.-%,

CO 0,38 Vol.-%, und

$CO_2$ 3,35 Vol.-%.

Das Reaktionsgas A-Beschickungsgasgemisch ist eine Mischung aus einem ersten (2,34 $Nm^3$/h) und einem zweiten (4,96 $Nm^3$/h) Gasgemisch, das durch Zusammenführen der beiden Gasgemische über einen statischen Mischer erzeugt wird. Das erste Gasgemisch weist eine Temperatur von 400°C und einen Druck von 2,13 bar auf und ist zusammengesetzt aus:

| | |
|---|---|
| 1,19 $Nm^3$/h | verdichtetem Restgaskreisgas (T = 70°C, P = 2,40 bar), |
| 1,05 $Nm^3$/h | Roh-Propan, das zu > 98 Vol.-% Propan (Frischpropan) enthält (T = 237°C, P = 2,90 bar), und |
| 0,098 $Nm^3$/h | $O_2$ (T = 139°C, P = 2,33 bar, Reinheit > 99 Vol.-%). |

Durch indirekten Wärmeaustausch in einem ersten Wärmeaustauscher mit aus der Reaktionszone A heraus und in Richtung der Reaktionszone B geführtem Produktgas A wird die Temperatur von 400°C eingestellt. Das zweite Gasgemisch ist zusammengesetzt aus:

3,68 $Nm^3$/h Dehydrierkreisgas (T = 551°C, P = 2,05 bar) und
1,28 $Nm^3$/h Propankreisgas (T = 400°C, P = 3,26 bar).

Es weist einen Druck von 2,13 bar und eine Temperatur von 506°C auf.

Durch indirekten Wärmeaustausch mit dem im ersten Wärmeaustauscher auf eine Temperatur von 435°C abgekühlten Produktgas A wird die Temperatur des Propankreisgases eingestellt. Die Dehydrierkreisgasführung erfolgt nach dem Strahlpumpenprinzip (vgl. DE-A 10211275), wobei das auf 400°C erwärmte Propankreisgas als Treibstrahl fungiert.

Die Eintrittstemperatur des Reaktionsgas A-Beschickungsgasgemischs und die Schütthöhe des vom Reaktionsgas A-Beschickungsgasgemisch durchströmten ersten Katalysatorbetts werden so eingestellt, dass das Reaktionsgas A dieses Katalysatorbett mit einer Temperatur von 515°C und einem Druck von 2,11 bar mit folgenden Gehalten verlässt:

| | |
|---|---|
| Propan | 55,8 Vol.-%, |
| Propylen | 15,85 Vol.-%, |
| Ethylen | 0,88 Vol.-%, |
| Methan | 2,89 Vol.-%, |
| $H_2$ | 3,78 Vol.-%, |
| $O_2$ | 0 Vol.-%, |
| $H_2O$ | 15,5 Vol.-%, und |
| $CO_2$ | 3,67 Vol.-%. |

Die Verlassmenge beträgt 7,46 $Nm^3$/h.

Hinter dem ersten Katalysatorbett werden dem Reaktionsgas A 0,12 $Nm^3$/h an molekularem Sauerstoff (Reinheit > 99 Vol.-%) zudosiert (T = 139°C, P = 2,33 bar). Die Zudosierung erfolgt durch Eindrosseln, so dass der resultierende Druck des resultierenden Reaktionsgases A nach wie vor 2,11 bar beträgt.

Die Schütthöhe des zweiten Katalysatorbetts ist so beschaffen, dass das Reaktionsgas A das zweite Katalysatorbett mit einer Temperatur von 533°C und einem Druck von 2,08 bar mit folgenden Gehalten verlässt:

| | |
|---|---|
| Propan | 49,2 Vol.-%, |
| Propylen | 19,24 Vol.-%, |
| Ethylen | 1,04 Vol.-%, |
| Methan | 3,14 Vol.-%, |
| $H_2$ | 4,43 Vol.-%, |
| $O_2$ | 0 Vol.-%, |
| $H_2O$ | 17,98 Vol.-%, und |
| $CO_2$ | 3,52 Vol.-%. |

Die Verlassmenge beträgt 7,79 $Nm^3$/h.

Diesem Reaktionsgas A werden vor dem vor dem dritten Katalysatorbett befindlichen statischen Mischer 0,13 $Nm^3$/h an molekularem Sauerstoff (Reinheit > 99 Vol.-%) zudosiert (T = 139°C, auf einem Druck von 2,33 bar befindlich eingedrosselt). Der resultierende Druck des resultierenden Reaktionsgases A beträgt weiterhin 2,08 bar.

Die Schütthöhe des dritten Katalysatorbetts ist so beschaffen, dass das Reaktionsgas A das dritte Katalysatorbett als Produktgas A mit einer Temperatur von 551°C und einem Druck von 2,05 bar mit folgenden Gehalten verlässt:

| | |
|---|---|
| Propan | 42,19 Vol.-%, |
| Propylen | 22,79 Vol.-%, |
| Ethylen | 1,14 Vol.-%, |
| Methan | 3,42 Vol.-%, |
| $H_2$ | 5,28 Vol.-%, |
| $O_2$ | 0 Vol.-%, |
| $H_2O$ | 20,32 Vol.-%, und |
| $CO_2$ | 3,36 Vol.-%. |

Die Verlassmenge beträgt 8,17 $Nm^3$/h.

Das Produktgas A wird durch einen Strömungsteiler in zwei Teilmengen identischer Zusammensetzung aufgeteilt. Die erste Teilmenge beträgt 3,68 $Nm^3$/h und die zweite Teilmenge beträgt 4,49 $Nm^3$/h. Die erste Teilmenge wird als Bestandteil des Reaktionsgas A-Beschickungsgasgemisches in die Reaktionszone A rückgeführt. Zum Zweck der Dehydrierkreisgasführung der ersten Teilmenge wird mit dem wie beschrieben erwärmten Propankreisgas als Treibstrahl eine Strahlpumpe betrieben, wobei die Förderrichtung des durch eine Treibdüse über eine Mischstrecke und einen Diffusor entspannten Treibstrahls in Richtung des Eingangs der Reaktionszone A sowie der Saugstutzen in Richtung der ersten Strömungsteilmenge des Produktgases A weist und die Verbindung "Saugstutzen - Mischstrecke - Diffusor" die alleinige Verbindungslinie zwischen der zurückzuführenden ersten Teilmenge des Produktgases A und dem Zugang zur Reaktionszone A bildet. Die zweite Teilmenge des Produktgases A wird aus der Reaktionszone A herausgeführt und zunächst durch einen ersten indirekten Wärmeaustausch mit dem am Reaktionsgas A-Ausgangsgemisch (Beschickungsgasgemisch) beteiligten ersten Gasgemisch aus verdichtetem Restgaskreisgas, Roh-Propan und molekularem Sauerstoff von 551 °C auf 435°C abgekühlt. Dabei fällt der Arbeitsdruck im aus der Reaktionszone A herausgeführten Produktgas A von 2,05 bar auf 1,95 bar. Das erste Gasgemisch wird dabei von 164°C auf 506°C erwärmt und erleidet einen Druckverlust von 2,40 bar auf 2,13 bar.

In einem nachfolgenden zweiten indirekten Wärmeaustausch mit Propankreisgas aus der Trennzone IV, wird das Produktgas A von 435°C auf 335°C abgekühlt. Der Arbeitsdruck im Produktgas A fällt dabei auf 1,94 bar. Die Temperatur des Propankreisgases steigt umgekehrt von 69°C auf 400°C.

Anschließend werden aus dem auf 335°C abgekühlten Produktgas A 0,76 $Nm^3$/h Wasserdampf kondensativ abgetrennt. Dazu wird das auf 335°C abgekühlte Produktgas A zunächst zu 3,73 $Nm^3$/h an Produktgas A* (T = 40°C, P = 1,68 bar), aus dem zuvor die entsprechende Wasserdampfmenge bereits kondensativ abgetrennt worden ist, im indirekten Wärmeaustausch geführt und dabei auf 108°C abgekühlt. Die Temperatur des Produktgases A* erhöht sich gleichzeitig auf 305°C. In einem nachfolgenden Luftkühler wird das Produktgas A (T = 108°C, P = 1,94 bar) durch indirekten Wärmeaustausch mit Außenluft auf 54°C abgekühlt (der Arbeitsdruck des Produktgases A fällt dabei auf 1,73 bar) und weist danach bereits Zweiphasigkeit auf.

Durch Direktkühlung mit versprühtem gekühltem (auf 29°C), zuvor aus Produktgas A durch entsprechende Direktkühlung abgetrenntem, wässrigem Kondensat, wird die vorgenannte Wasserdampfmenge kondensativ aus dem Produktgas A abgetrennt und ein Produktgas A* erzeugt (P = 1,68 bar, T = 40°C, 3,73 $Nm^3$/h). Dieses wird wie vorstehend beschrieben auf 305°C erwärmt. Dann werden in das Produktgas A* 1,39 $Nm^3$/h molekularer Sauerstoff (Reinheit > 99 Vol.-%, T = 139°C, P = 2,33 bar) eingedrosselt. Dadurch entsteht ein Reaktionsgas B-Ausgangsgemisch (Beschickungsgasgemisch), das eine Temperatur von 288°C und einen Arbeitsdruck von 1,63 bar aufweist.

Das Reaktionsgas B-Ausgangsgemisch (Beschickungsgasgemisch) weist folgende Gehalte auf:

| | |
|---|---|
| Propan | 37,01 Vol.-%, |
| Propylen | 19,99 Vol.-%, |
| Ethylen | 0,99 Vol.-%, |
| Methan | 2,99 Vol.-%, |
| $H_2$ | 4,63 Vol.-%, |
| $O_2$ | 26,79 Vol.-%, |
| $H_2O$ | 3,14 Vol.-%, und |
| $CO_2$ | 2,95 Vol.-%. |

Mit ihm wird die Reaktionszone B beschickt.

Die in Strömungsrichtung des Reaktionsgases B erste Oxidationsstufe derselben ist ein zwei Temperaturzonen aufweisender Vielrohrreaktor. Die Reaktionsrohre sind wie folgt beschaffen: V2A-Stahl; 30 mm Außendurchmesser, 2 mm Wandstärke, 26 mm Innendurchmesser, Länge 350 cm. Von oben nach unten sind die Reaktionsrohre wie folgt beschickt:

| Abschnitt 1: | 50 cm Länge |
| --- | --- |
| | Steatitringe der Geometrie 7 mm x 7 mm x 4 mm (Außendurchmesser x Länge x Innendurchmesser) als Vorschüttung. |

| Abschnitt 2: | 140 cm Länge |
| --- | --- |
| | Katalysatorbeschickung mit einem homogenen Gemisch aus 20 Gew.-% (alternativ 30 Gew.-%) an Steatitringen der Geometrie 5 mm x 3 mm x 2 mm (Außendurchmesser x Länge x Innendurchmesser) und 80 Gew.-% (alternativ 70 Gew.-%) Vollkatalysator aus Abschnitt 3. |

| Abschnitt 3: | 160 cm Länge |
| --- | --- |
| | Katalysatorbeschickung mit ringförmigem (5 mm x 3 mm x 2 mm = Außendurchmesser x Länge x Innendurchmesser) Vollkatalysator gemäß Beispiel 1 der DE-A 100 46 957 (Stöchiometrie : $[Bi_2W_2O_9 x 2WO_3]_{0,5}[Mo_{12}Co_{5,5}Fe_{2,94}Si_{1,59}K_{0,08}O_x]_1$). |
| | Alternativ kann hier auch einer der Katalysatoren BVK 1 bis BVK 11 aus Research Disclosure Nr. 497012 vom 29.08.2005 eingesetzt werden (die spezifischen Oberflächen der Aktivmassen sind dort versehentlich in $cm^2/g$ angegeben; die korrekte Dimension ist bei gleichem Zahlenwert jedoch $m^2/g$). |

Von oben nach unten werden die ersten 175 cm mittels eines im Gegenstrom zum Reaktionsgas B gepumpten Salzbades A thermostatisiert. Die zweiten 175 cm werden mittels eines im Gegenstrom zum Reaktionsgas B gepumpten Salzbades B thermostatisiert.

Die in Strömungsrichtung des Reaktionsgases B zweite Oxidationsstufe ist ebenfalls ein zwei Temperaturzonen aufweisender Vielrohrreaktor. Die Reaktionsrohre sind von oben nach unten wie folgt beschickt:

| Abschnitt 1: | 20 cm Länge |
| --- | --- |
| | Steatitringe der Geometrie 7 mm x 7 mm x 4mm (Außendurchmesser x Länge x Innendurchmesser) als Vorschüttung. |

| Abschnitt 2: | 90 cm Länge |
| --- | --- |
| | Katalysatorbeschickung mit einem homogenen Gemisch aus 25 Gew.-% (alternativ 30 Gew.-%) Steatit-Ringen der Geometrie 7 mm x 3mm x 4mm (Aussendurchmesser x Länge x Innendurchmesser) und 75 Gew.-% (alternativ 70 Gew.-%) Schalenkatalysator aus Abschnitt 4. |

| Abschnitt 3: | 50 cm Länge |
| --- | --- |
| | Katalysatorbeschickung mit einem homogenen Gemisch aus 15 Gew.-% (alternativ 20 Gew.-%) an Steatit-Ringen der Geometrie 7 mm x 3 mm x 4 mm (Außendurchmesser x Länge x Innendurchmesser) und 85 Gew.-% (alternativ 80 Gew.-%) Schalenkatalysator aus Abschnitt 4. |

| Abschnitt 4: | 190 cm Länge |
| --- | --- |
| | Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 100 46 928 (Stöchiometrie : $Mo_{12}V_3W_{1,2}Cu_{2,4}O_x$). |

Von oben nach unten werden die ersten 175 cm mittels eines im Gegenstrom zum Reaktionsgas gepumpten Salzbades C thermostatisiert. Die zweiten 175 cm werden mittels eines im Gegenstrom zum Reaktionsgas gepumpten Salzbad D thermostatisiert.

In beiden Oxidationsstufen werden die Salzbäderjeweils durch Umlenkbleche mäandrierend um die Reaktionsrohre geführt.

Zwischen den beiden Oxidationsstufen befindet sich ein mittels Salzbad gekühlter Rohrbündelwärmetauscher, mit dem das Produktgas der ersten Oxidationsstufe abgekühlt werden kann. Vor dem Eintritt in die zweite Oxidationsstufe befindet sich ein Ventil zur Zufuhr von molekularem Sauerstoff (Reinheit > 99 Vol.-%).

Die Propylenbelastung der Katalysatorbeschickung der ersten Oxidationsstufe wird zu 130 Nl/l•h gewählt. Die Salzschmelzen (53 Gew.-% $KNO_3$, 40 Gew.-% $NaNO_2$, 7 Gew.-% $NaNO_3$) weisen folgende Einrittstemperaturen auf:

$$T_A = 324°C \qquad T_B = 328°C$$
$$T_C = 265°C \qquad T_D = 269°C$$

Dem Produktgasgemisch der ersten Oxidationsstufe wird soviel molekularer Sauerstoff (139°C, 2,33 bar) zudosiert (eingedrosselt), dass das molare Verhältnis $O_2$: Acrolein im resultierenden Beschickungsgasgemisch für die zweite Oxidationsstufe 0,73 beträgt. Die Acroleinbelastung der Katalysatorbeschickung der zweiten Oxidationsstufe beträgt 110 Nl/l•h. Der Druck am Eingang der zweiten Oxidationsstufe beträgt 1,57 bar. Das Reaktionsgas B verlässt den Zwischenkühler mit einer Temperatur von 260°C und die Eintrittstemperatur des Beschickungsgasgemischs in die zweite Oxidationsstufe beträgt 256°C. Das Produktgasgemisch der ersten Oxidationsstufe weist folgende Gehalte auf:

| | |
|---|---|
| Acrolein | 16,84 Vol.-%, |
| Acrylsäure | 1,13 Vol.-%, |
| Propan | 36,88 Vol.-%, |
| Propylen | 0,99 Vol.-%, |
| Methan | 2,99 Vol.-%, |
| $H_2$ | 4,61 Vol.-%, |
| Ethylen | 0,99 Vol.-%, |
| $O_2$ | 4,32 Vol.-%, |
| $H_2O$ | 23,96 Vol.-%, |
| CO | 0,847 Vol.-%, und |
| $CO_2$ | 4,93 Vol.-%. |

Die Temperatur des Produktgases der ersten Oxidationsstufe beträgt vor Eintritt in den Nachkühler 335°C.

Das Produktgasgemisch der zweiten Oxidationsstufe (das Produktgas B) weist eine Temperatur von 270°C und einen Druck von 1,55 bar sowie die folgenden Gehalte auf:

| | |
|---|---|
| Acrolein | 0,089 Vol.-%, |
| Acrylsäure | 17,02 Vol.-%, |
| Essigsäure | 0,46 Vol.-%, |
| Propan | 36,79 Vol.-%, |
| Propylen | 0,99 Vol.-%, |
| Methan | 2,98 Vol.-%, |
| Ethylen | 0,99 Vol.-%, |
| $H_2$ | 4,59 Vol.-%, |
| $O_2$ | 2,69 Vol.-%, |
| $H_2O$ | 24,57 Vol.-%, |
| CO | 1,35 Vol.-% und |
| $CO_2$ | 5,91 Vol.-%. |

Das Produktgas B (5,15 $Nm^3$/h) wird wie in der WO 2004/035514 beschrieben in einer Bodenkolonne (Trennzone II) fraktionierend kondensiert.

Der Rückstandsverbrennung werden als erster Brennstoff 13,7 g/h Schwersieder (Polyacrylsäuren (Michael-Addukte) etc.) zugeführt.

Vom zweiten Fangboden oberhalb der Zufuhr des Produktgases B in die Bodenkolonne werden 2,80 kg/h einer kondensierten rohen Acrylsäure entnommen, die eine Temperatur von 15°C und 96,99 Gew.-% Acrylsäure aufweist. Diese wird wie in der WO 2004/035514 beschrieben nach Zugabe einer geringen Menge Wasser suspensionskristallisiert, das Suspensionskristallisat in hydraulischen Waschkolonnen von der Mutterlauge getrennt und die Mutterlauge wie in der WO 2004/035514 beschrieben in die Kondensationskolonne rückgeführt. Die Reinheit des gewaschenen Suspensionskristallisats beträgt > 99,87 Gew.-% Acrylsäure und eignet sich unmittelbar zur Herstellung von Wasser "superabsorbierenden" Polymerisaten zur Verwendung im Hygienebereich.

Die vom dritten Fangboden oberhalb der Zufuhr des Produktgases B in die Kondensationskolonne entnommene, nicht in die Kondensationskolonne rückgeführte Menge an Sauerwasserkondensat beträgt 1,10 kg/h, weist eine Temperatur von 33°C auf und hat folgende Gehalte:

| | |
|---|---|
| 0,80 Gew.-% | Acrolein, |
| 4,92 Gew.-% | Acrylsäure, |
| 5,73 Gew.-% | Essigsäure und |
| 87,0 Gew.-% | Wasser. |

Sie wird ebenfalls der Rückstandsverbrennung zugeführt (als Sauerstoffquelle werden in die Rückstandsverbrennung 5,49 Nm$^3$/h Luft geführt).

Am Kopf der Kondensationskolonne verlassen 2,98 Nm$^3$/h an Restgas die Trennzone II mit einer Temperatur von 33°C und einem Druck von 1,20 bar und folgenden Gehalten:

| | |
|---|---|
| Acrolein | 0,03 Vol.-%, |
| Acrylsäure | 0,02 Vol.-%, |
| Essigsäure | 0,01 Vol.-%, |
| Propan | 63,7 Vol.-%, |
| Propylen | 1,72 Vol.-%, |
| $H_2$ | 6,90 Vol.-%, |
| Methan | 5,16 Vol.-%, |
| Ethylen | 1,72 Vol.-%, |
| $O_2$ | 4,68 Vol.-%, |
| $H_2O$ | 2,07 Vol.-%, |
| CO | 2,34 Vol.-% und |
| $CO_2$ | 10,18 Vol.-%. |

In der ersten Verdichterstufe eines mehrstufigen Radialverdichters wird das Restgas von 1,20 bar auf 2,40 bar verdichtet, wobei die Temperatur des Restgases auf 70°C ansteigt. Dann wird das verdichtete Restgas in zwei Teilmengen identischer Zusammensetzung aufgeteilt. Die erste Teilmenge (1,19 Nm$^3$/h) wird als (verdichtetes) Restgaskreisgas der Gestaltung des Beschickungsgasgemisches für die Reaktionszone A zugeführt. Die andere Teilmenge (1,79 Nm$^3$/h) wird in einer zweiten Verdichterstufe von 2,40 bar auf 6,93 bar verdichtet. Dabei erwärmt es sich auf 130°C.

In einem indirekten Wärmeaustauscher wird es auf 113°C abgekühlt, ohne dass dabei Kondensatbildung erfolgt (Kühlmittel ist das "Propan-gewaschene" Restgas (im folgenden wird es auch als "Abgas" bezeichnet), das nach Erwärmung in einem indirekten Wärmeaustauscher von 30°C auf 86°C in der ersten Expansionsstufe einer mehrstufigen Expansionsturbine von 20 bar ausgehend entspannt wird und dabei abkühlt). Das Abgas (0,58 Nm$^3$/h) erwärmt sich dabei. In einer zweiten Expansionsstufe wird das Abgas auf 1,10 bar entspannt (es hat dann eine Temperatur von 25 °C) und anschließend der Rückstandsverbrennung zugeführt.

In einem indirekten Luftkühler wird das verdichtete Restgas (P = 6,93 bar und T = 113°C) auf 59°C abgekühlt. In einer dritten Verdichterstufe wird es von 6,93 bar auf 20 bar verdichtet, wobei es sich gleichzeitig auf 116°C erwärmt.

In einem nachfolgenden indirekten Wärmeaustauscher wird es auf 107°C abgekühlt (Kühlmittel ist das "Propangewaschene" Restgas, das sich dabei von 30°C auf 86°C erwärmt), ohne dass dabei Kondensatbildung erfolgt. In einem sich anschließenden weiteren indirekten Wärmeaustauscher wird das 107°C aufweisende Restgas auf 35°C abgekühlt (Kühlmittel ist ca. 25°C aufweisendes Oberflächenwasser; anstelle von Oberflächenwasser kann in dieser Schrift stets auch sonstiges Kühlwasser eingesetzt werden). Dabei kondensieren 29 g/h Wasser aus dem Restgas aus. Dieses wässrige Kondensat (das vorzugsweise mit Tropfenabschneidern abgeschieden wird) wird ebenfalls der Rückstandsverbrennung zugeführt und in selbiger gemeinsam mit den anderen genannten Rückständen verbrannt (unter Zufuhr von 5,49 Nm$^3$/h Luft, wie bereits gesagt). Die heißen Verbrennungsgase werden unter Erzeugung von Wasserdampf (52 bar, 267°C, 4,6 kg/h) abgekühlt und in die Umgebung entlassen.

Die jetzt noch 1,75 Nm$^3$/h an Propan enthaltendem Restgas (20 bar, 35°C) werden in den unteren Teil einer Füllkörperkolonne (Trennzone III) zugeführt. Am Kopf dieser Waschkolonne werden 12,96 kg/h mit einer Aufgabetemperatur von 30°C (P = 20 bar) technisches Tetradecan der Fa. Haltermann, DE, vom Typ PKWF 4/7 af als Absorptionsmittel aufgegeben (gaschromatographische Analyse mittels F/D-Detektion ergibt zu Beginn (frisch) nachfolgende GC-Flächen-%-Zusammensetzung:

| | |
|---|---|
| n-Tridecan | 7,6 %, |
| n-Tetradecan | 47,3 %, |
| n-Pentadecan | 7,0 %, |
| n-Hexadecan | 3,2 %, |
| n-Heptadecan | 14,1 % und |
| Restsumme | 20,7 %). |

Am Kopf der Waschkolonne entweicht "Propan-gewaschenes" Restgas (Abgas), das bei einem Druck von 20 bar und einer Temperatur von 30°C bei einer Menge von 0,58 Nm$^3$/h folgende Gehalte aufweist:

| | |
|---|---|
| Propan | 1,97 Vol.-%, |
| Propylen | 0,049 Vol.-%, |
| Methan | 15,95 Vol.-%, |
| Ethylen | 5,32 Vol.-%, |
| $H_2$ | 21,29 Vol.-%, |
| $O_2$ | 14,46 Vol.-%, |
| $H_2O$ | 0,82 Vol.-%, |
| CO | 7,23 Vol.-% und |
| $CO_2$ | 31,43 Vol.-%. |

Dieses Abgas wird wie beschrieben entspannt sowie indirektem Wärmeaustausch unterworfen und dann der Rückstandsverbrennung zugeführt bzw. über eine Fackel verbrannt.

Dem Sumpf der "Propan-Waschkolonne", die von außen weder gekühlt noch beheizt wird, werden 15,27 kg/h an Absorbat entnommen, das einen Druck von 20 bar und eine Temperatur von 59°C aufweist. Es enthält 14,79 Gew.-% Propan und 0,38 Gew.-% Propylen.

Bevor das Absorbat zum Kopf der nachfolgenden Desorptionskolonne geführt wird, wird es in einem indirekten Wärmeaustauscher auf 69°C erwärmt. Als Wärmeträger wird der flüssige Ablauf aus der Desorptionskolonne verwendet (12,96 kg/h), der bei einem Druck von 3,26 bar eine Temperatur von 80°C aufweist und noch 0,09 Gew.-% Propan gelöst enthält. Mittels einer Pumpe wird er wieder auf 20 bar komprimiert und nach indirekter Abkühlung mit Oberflächenwasser (25°C) auf 30°C zur Propan-Absorption auf den Kopf der "Propan-Waschkolonne" rückgeführt.

Nach der Erwärmung auf 69°C wird das Absorbat (z. B. in einer inversen Pumpe oder mittels eines Ventils) auf einen Druck von 3,26 bar entspannt (die im Fall der inversen Pumpe dabei frei gesetzte mechanische Energie wird zweckmäßig zur Rückverdichtung von in der Desorptionskolonne von Propan befreitem Absorptionsmittel (dem flüssigen Ablauf der Desorptionskolonne) mitverwendet) und das dabei erzeugte zweiphasige Gemisch am Kopf in die Desorptionskolonne geführt.

In die Desorptionskolonne (ebenfalls eine Füllkörperkolonne) wird als Stripgas von unten im Gegenstrom zum vom Desorptionskolonnenkopf ablaufenden Absorbat mit einem Druck von 5 bar und einer Temperatur von 152°C 0,1 kg/h Wasserdampf zugeführt.

In der Desorptionskolonne befindet sich eine Heizschlange, durch die im Gegenstrom zum in der Desorptionskolonne aufsteigenden Wasserdampf ebenfalls Wasserdampf der Temperatur 152°C, P = 5,00 bar in einer Menge von 0,5 kg/h geführt wird.

Am Kopf der Desorptionskolonne entweichen 1,28 Nm$^3$/h an Propankreisgas, das eine Temperatur von 69°C und einen Arbeitsdruck von 3,26 bar aufweist. Nach Erwärmung auf 400°C durch indirekten Wärmeaustausch mit Produktgas A der Temperatur T = 435°C, wird das Propankreisgas als Treibstrahl zum Betrieb der Strahlpumpe eingesetzt, mit der das Dehydrierkreisgas und das Propankreisgas der Beschickung der Reaktionszone A mit Reaktionsgas A-Ausgangsgemisch zugeführt werden.

Das Propankreisgas weist folgende Gehalte auf:

| | |
|---|---|
| Propan | 88,45 Vol.-%, |
| Propylen | 2,39 Vol.-%, und |
| $H_2O$ | 8,66 Vol.-%. |

III. Zweites beispielhaftes Verfahren zur Herstellung von Acrylsäure aus Propan (beschrieben wird ein stationärer Betriebszustand)

Die Reaktionszone A ist ein wie unter II. beschriebener Hordenreaktor. Die heterogen katalysierte partielle Propandehydrierung wird in diesem Hordenreaktor im geraden Durchgang mit Dehydrierkreisgasfahrweise durchgeführt. Die Belastung der Katalysatorgesamtmenge (ohne Intermaterial gerechnet) aller Horden mit Propan beträgt 1500 Nl/l·h.

Dem in Strömungsrichtung ersten Katalysatorbett werden 8,23 Nm$^3$/h Reaktionsgas A-Beschickungsgasgemisch (Ausgangsgemisch; P = 1,84 bar) zugeführt, das folgende Gehalte aufweist:

| | |
|---|---|
| Propan | 64,20 Vol.-%, |
| Propylen | 16,0 Vol.-%, |
| Methan | 1,39 Vol.-%, |
| Ethylen | 0,46 Vol.-%, |
| $H_2$ | 2,76 Vol.-%, |
| $O_2$ | 1,06 Vol.-%, und |
| $H_2O$ | 13,13 Vol.-%. |

Das Reaktionsgas A-Beschickungsgasgemisch ist eine Mischung aus einem ersten (1,15 Nm$^3$/h) und einem zweiten (7,08 Nm$^3$/h) Gasgemisch, das durch Zusammenführen der beiden Gasgemische über einen statischen Mischer erzeugt wird. Das erste Gasgemisch weist eine Temperatur von 450°C und einen Druck von 1,84 bar auf, und ist zusammengesetzt aus:

| | |
|---|---|
| 1,06 Nm$^3$/h | Roh-Propan, das zu > 98 Vol.-% Propan (Frischpropan) enthält (T = 237°C, P = 2,90 bar), und |
| 0,087 Nm$^3$/h | $O_2$ (T = 143°C, P = 1,84 bar, Reinheit > 99 Vol.-%). |

Durch indirekten Wärmeaustausch in einem ersten Wärmeaustauscher mit aus der Reaktionszone A heraus- und in Richtung der Reaktionszone B geführtem Produktgas A wird die Temperatur von 450°C eingestellt.

Das zweite Gasgemisch ist zusammengesetzt aus:

5,04 Nm$^3$/h Dehydrierkreisgas (T = 540°C, P = 1,73 bar), und
2,04 Nm$^3$/h Propankreisgas (T = 400°C, P = 3,26 bar).

Es weist einen Druck von 1,84 bar und eine Temperatur von 486°C auf. Durch indirekten Wärmeaustausch mit dem im ersten Wärmeaustauscher auf eine Temperatur von 476°C abgekühlten Produktgas A wird die Temperatur des Propankreisgases eingestellt. Die Dehydrierkreisgasführung erfolgt nach dem Strahlpumpenprinzip (vgl. DE-A 10211275), wobei das auf 400°C erwärmte Propankreisgas als Treibstrahl fungiert.

Die Eintrittstemperatur des Reaktionsgas A-Beschickungsgasgemischs und die Schütthöhe des vom Reaktionsgas A-Beschickungsgasgemisch durchströmten ersten Katalysatorbetts werden so eingestellt, dass das Reaktionsgas A dieses Katalysatorbett mit einer Temperatur von 503°C und einem Druck von 1,81 bar mit folgenden Gehalten verlässt:

| | |
|---|---|
| Propan | 60,7 Vol.-%, |
| Propylen | 18,19 Vol.-%, |
| Methan | 1,59 Vol.-%, |
| Ethylen | 0,53 Vol.-%, |
| $H_2$ | 2,94 Vol.-%, |
| $O_2$ | 0 Vol.-%, und |
| $H_2O$ | 15,0 Vol.-%. |

Die Verlassmenge beträgt 8,35 Nm$^3$/h.

Hinter dem ersten Katalysatorbett werden dem Reaktionsgas A 0,13 Nm$^3$/h an molekularem Sauerstoff (Reinheit > 99 Vol.-%) zudosiert (T = 143°C, P = 2,40 bar). Die Zudosierung erfolgt durch Eindrosseln, so dass der resultierende Druck des resultierenden Reaktionsgases A nach wie vor 1,81 bar beträgt.

Die Schütthöhe des zweiten Katalysatorbetts ist so beschaffen, dass das Reaktionsgas A das zweite Katalysatorbett mit einer Temperatur von 520°C und einem Druck von 1,78 bar mit folgenden Gehalten verlässt:

| | |
|---|---|
| Propan | 54,2Vol.-%, |
| Propylen | 21,3 Vol.-%, |
| Methan | 1,90 Vol.-%, |
| Ethylen | 0,63 Vol.-%, |
| $H_2$ | 3,61 Vol.-%, |
| $O_2$ | 0 Vol.-%, und |
| $H_2O$ | 17,3 Vol.-%. |

Die Verlassmenge beträgt 8,70 $Nm^3$/h.

Diesem Reaktionsgas A werden vor dem vor dem dritten Katalysatorbett befindlichen statischen Mischer 0,17 $Nm^3$/h an molekularem Sauerstoff (Reinheit > 99 Vol.-%) zudosiert (T = 143°C, auf einen Druck von 1,78 bar eingedrosselt). Der resultierende Druck des resultierenden Reaktionsgases beträgt weiterhin 1,78 bar.

Die Schütthöhe des dritten Katalysatorbetts ist so beschaffen, dass das Reaktionsgas A das dritte Katalysatorbett als Produktgas A mit einer Temperatur von 540°C und einem Druck von 1,73 bar mit folgenden Gehalten verlässt:

| | |
|---|---|
| Propan | 46,30 Vol.-%, |
| Propylen | 25,12 Vol.-%, |
| Ethylen | 0,75 Vol.-%, |
| Methan | 2,27 Vol.-%, |
| $H_2$ | 4,50 Vol.-%, |
| $O_2$ | 0 Vol.-%, und |
| $H_2O$ | 20,05 Vol.-%. |

Die Verlassmenge beträgt 9,17 $Nm^3$/h.

Durch einen Strömungsteiler wird das Produktgas A in zwei Teilmengen identischer Zusammensetzung aufgeteilt. Die erste Teilmenge beträgt 5,04 $Nm^3$/h und die zweite Teilmenge beträgt 4,13 $Nm^3$/h.

Die erste Teilmenge wird als Bestandteil des Reaktionsgas-A-Beschickungsgasgemischs in die Reaktionszone A rückgeführt. Zum Zweck der Dehydrierkreisgasführung der ersten Teilmenge wird mit dem wie beschrieben erwärmten Propankreisgas als Treibstrahl eine Strahlpumpe betrieben, wobei die Förderrichtung des durch eine Treibdüse über eine Mischstrecke und einen Diffusor entspannten Treibstrahls in Richtung des Eingangs der Reaktionszone A sowie der Saugstutzen in Richtung der ersten Strömungsteilmenge des Produktgases A weist und die Verbindung "Saugstutzen-Mischstrecke-Diffusor" die alleinige Verbindungslinie zwischen der zurückzuführenden ersten Teilmenge des Produktgases A und dem Zugang zur Reaktionszone A bildet.

Die zweite Teilmenge des Produktgases A wird aus der Reaktionszone A herausgeführt und zunächst durch einen ersten indirekten Wärmeaustausch mit dem am Reaktionsgas A-Ausgangsgemisch (Beschickungsgasgemisch) beteiligten ersten Gasgemisch aus Roh-Propan und molekularem Sauerstoff von 540 °C auf 476 °C abgekühlt. Der Arbeitsdruck des Produktgases A bleibt dabei im wesentlichen erhalten.

Das erste Gasgemisch wird dabei von 235 °C auf 450 °C erwärmt, während der Arbeitsdruck dieses Gasgemischs von 1,84 bar im wesentlichen erhalten bleibt.

In einem nachfolgenden zweiten indirekten Wärmeaustausch mit Propankreisgas aus der Trennzone IV, wird das Produktgas A von 476 °C auf 307 °C abgekühlt. Die Temperatur des Propankreisgases steigt umgekehrt von 69 °C auf 400 °C.

Anschließend werden aus dem auf 307 °C abgekühlten Produktgas A 0,52 $Nm^3$/h Wasserdampf kondensativ abgetrennt. Dazu wird das auf 307 °C abgekühlte Produktgas A zunächst zu 3,60 $Nm^3$/h an Produktgas A* (T = 54 °C, P = 1,73 bar), aus dem zuvor die entsprechende Wasserdampfmenge bereits kondensativ abgetrennt worden ist, im indirekten Wärmeaustausch geführt und dabei auf 111 °C abgekühlt.

Die Temperatur des Produktgases A* erhöht sich gleichzeitig auf 277 °C.

Durch Direktkühlung mit versprühtem gekühltem (auf 29 °C), zuvor aus Produktgas A durch entsprechende Direktkühlung abgetrenntem, wässrigem Kondensat, wird die vorgenannte Wasserdampfmenge kondensativ aus dem Produktgas A abgetrennt und ein Produktgas A* erzeugt (P = 1,73 bar, T = 54 °C, 3,60 $Nm^3$/h). Dieses wird wie vorstehend beschrieben auf 277 °C erwärmt. Dann werden in das Produktgas A* 1,39 $Nm^3$/h molekularer Sauerstoff (Reinheit > 99 Vol.-%, T = 143 °C, P = 2,40 bar) eingedrosselt. Dadurch entsteht ein Reaktionsgas B-Ausgangsgemisch (Beschickungsgasgemisch), das eine Temperatur von 262 °C und einen Arbeitsdruck von 1,63 bar aufweist.

Das Reaktionsgas B-Ausgangsgemisch (Beschickungsgasgemisch) weist folgende Gehalte auf:

| Propan   | 38,26 Vol.-%,      |
|----------|--------------------|
| Propylen | 20,77 Vol.-%,      |
| Ethylen  | 0,62 Vol.-%,       |
| Methan   | 1,87 Vol.-%,       |
| $H_2$    | 3,73 Vol.-%,       |
| $O_2$    | 27,53 Vol.-%, und  |
| $H_2O$   | 6,20 Vol.-%.       |

Mit ihm wird die Reaktionszone B beschickt, die wie diejenige aus II. aufgebaut ist.

Die Propylenbelastung der Katalysatorbeschickung der ersten Oxidationsstufe wird zu 164 Nl/l•h gewählt. Die Salzschmelzen (53 Gew.-% $KNO_3$, 40 Gew.-% $NaNO_2$, 7 Gew.-% $NaNO_3$) weisen folgende Eintrittstemperaturen auf:

$$T_A = 331\ °C \qquad T_B = 339\ °C$$
$$T_C = 275\ °C \qquad T_D = 282\ °C$$

Dem Produktgasgemisch der ersten Oxidationstufe wird soviel molekularer Sauerstoff (143 °C, 2,40 bar) zudosiert (eingedrosselt), dass das molare Verhältnis $O_2$: Acrolein im resultierenden Beschickungsgasgemisch für die zweite Oxidationsstufe 0,72 beträgt. Die Acroleinbelastung der Katalysatorbeschickung der zweiten Oxidationsstufe beträgt 139 Nl/l•h. Der Druck am Eingang der zweiten Oxidationsstufe beträgt 1,57 bar.

Das Reaktionsgas B verlässt den Zwischenkühler mit einer Temperatur von 260 °C und die Eintrittstemperatur das Beschickungsgasgemischs in die zweite Oxidationsstufe beträgt 256 °C.

Das Produktgasgemisch der ersten Oxidationstufe weist folgende Gehalten auf:

| Acrolein   | 17,51 Vol.-%,     |
|------------|-------------------|
| Acrylsäure | 1,18 Vol.-%,      |
| Propan     | 38,12 Vol.-%,     |
| Propylen   | 1,03 Vol.-%,      |
| Methan     | 1,86 Vol.-%,      |
| $H_2$      | 3,71 Vol.-%,      |
| Ethylen    | 0,62 Vol.-%,      |
| $O_2$      | 4,18 Vol.-%,      |
| $H_2O$     | 27,83 Vol.-%,     |
| CO         | 0,88 Vol.-%, und  |
| $CO_2$     | 2,07 Vol.-%.      |

Die Temperatur des Produktgases der ersten Oxidationsstufe beträgt vor Eintritt in den Nachkühler 335 °C.

Das Produktgasgemisch der zweiten Oxidationsstufe (das Produktgas B) weist eine Temperatur von 270 °C und einen Druck von 1,55 bar sowie die folgenden Gehalte auf:

| Acrolein   | 0,089 Vol.-%,     |
|------------|-------------------|
| Acrylsäure | 17,67 Vol.-%,     |
| Essigsäure | 0,52 Vol.-%,      |
| Propan     | 37,99 Vol-%,      |
| Propylen   | 1,02 Vol.-%,      |
| Methan,    | 1,86 Vol.-%,      |
| Ethylen    | 0,62 Vol.-%,      |
| $H_2$      | 3,70 Vol-%,       |
| $O_2$      | 2,61 Vol.-%,      |
| $H_2O$     | 28,42 Vol.-%,     |
| CO         | 1,41 Vol.-%, und  |
| $CO_2$     | 3,10 Vol.%        |

Das Produktgas B (5,03 Nm$^3$/h) wird wie in der WO 2004/035514 beschrieben in einer Bodenkolonne (Trennzone II) fraktionierend kondensiert.

Der Rückstandsverbrennung werden als erster Brennstoff 13,9 g/h Schwersieder (Polyacrylsäuren (Michael-Addukte) etc.) zugeführt.

Vom zweiten Fangboden oberhalb der Zufuhr des Produktgases B in die Bodenkolonne werden 2,82 kg/h einer kondensierten rohen Acrylsäure entnommen, die eine Temperatur von 15°C und 96,99 Gew.-% Acrylsäure aufweist. Diese wird wie in der WO 2004/035514 beschrieben nach Zugabe einer geringen Menge Wasser suspensionskristallisiert, das Suspensionskristallisat in hydraulischen Waschkolonnen von der Mutterlauge getrennt und die Mutterlauge wie in der WO 2004/035514 beschrieben in die Kondensationskolonne rückgeführt. Die Reinheit des gewaschenen Suspensionskristallisats beträgt > 99,87 Gew.-% Acrylsäure und eignet sich unmittelbar zur Herstellung von Wasser "superabsorbierenden" Polymerisaten zur Verwendung im Hygienebereich.

Die vom dritten Fangboden oberhalb der Zufuhr des Produktgases B in die Kondensationskolonne entnommene, nicht in die Kondensationskolonne rückgeführte Menge an Sauerwasserkondensat beträgt 1,24 kg/h, weist eine Temperatur von 33°C auf und hat folgende Gehalte:

| | |
|---|---|
| 0,73 Gew.-% | Acrolein, |
| 4,95 Gew.-% | Acrylsäure, |
| 5,16 Gew.-% | Essigsäure und |
| 88,1 Gew.-% | Wasser. |

Am Kopf der Kondensationskolonne verlassen 2,72 Nm$^3$/h an Restgas die Trennzone II mit einer Temperatur von 33°C und einem Druck von 1,20 bar und folgenden Gehalten:

| | |
|---|---|
| Acrolein | 0,03 Vol.-%, |
| Acrylsäure | 0,02 Vol.-%, |
| Essigsäure | 0,01 Vol.-%, |
| Propan | 70,26 Vol.-%, |
| Propylen | 1,91 Vol.-%, |
| Ethylen | 1,15 Vol.-%, |
| $H_2$ | 6,93 Vol.-%, |
| Methan | 3,44 Vol.-%, |
| $O_2$ | 4,83 Vol.-%, |
| $H_2O$ | 2,11 Vol.-%, |
| CO | 2,59 Vol.-% und |
| $CO_2$ | 5,73 Vol.-%. |

In der ersten Verdichterstufe eines mehrstufigen Radialverdichters wird das Restgas von 1,20 bar auf 6,93 bar verdichtet, wobei die Temperatur des Restgases auf 127 °C ansteigt.

In einem indirekten Wärmeaustauscher wird es auf 114 °C abgekühlt, ohne dass dabei Kondensatbildung erfolgt (Kühlmittel ist das "Propan-gewaschene" Restgas (im folgenden auch als "Abgas" bezeichnet), das nach Erwärmung in einem indirekten Wärmeaustauscher von 30 °C auf 84 °C in der ersten Expansionsstufe einer mehrstufigen Expansionsturbine von 20 bar ausgehend entspannt wird und dabei abkühlt). Das Abgas (0,69 Nm$^3$/h) erwärmt sich dabei. In einer zweiten Expansionsstufe wird das Abgas auf 1,10 bar entspannt (es hat dann eine Temperatur von 18 °C) und anschließend der Rückstandsverbrennung zugeführt.

In einem indirekten Luftkühler wird das verdichtete Restgas (P = 6,93 bar und T = 114 °C) auf 59 °C abgekühlt. In einer weiteren Verdichterstufe wird es von 6,93 bar auf 20 bar verdichtet, wobei es sich gleichzeitig auf 114 °C erwärmt. In einem nachfolgenden indirekten Wärmeaustauscher wird es auf 107 °C abgekühlt (Kühlmittel ist das "Propan-gewaschene" Restgas, das sich dabei von 30 °C auf 84 °C erwärmt), ohne dass dabei Kondensatbildung erfolgt. In einem sich anschließenden weiteren indirekten Wärmeaustauscher wird das 107 °C aufweisende Restgas auf 35 °C abgekühlt (Kühlmittel ist ca. 25 °C aufweisendes Oberflächenwasser).

Dabei kondensieren 44,8 g/h Wasser aus dem Restgas aus. Dieses wässrige Kondensat wird ebenfalls der Rückstandsverbrennung zugeführt und in selbiger gemeinsam mit den anderen genannten Rückständen (unter Zufuhr von 5,95 Nm$^3$/h Luft) verbrannt. Die heißen Verbrennungsgase werden unter Erzeugung von Wasserdampf (52 bar, 267 °C, 5,16 kg/h) abgekühlt und in die Umgebung entlassen.

Die jetzt noch 2,67 Nm$^3$/h an Propan enthaltendem Restgas (20 bar, 35 °C) werden in den unteren Teil einer Füllkörperkolonne (Trennzone III) geführt.

Am Kopf dieser Waschkolonne werden 20,39 kg/h mit einer Aufgabetemperatur von 30 °C (P = 20 bar) technisches Tetradecan der Fa. Haltermann, DE, (wie in II. beschrieben) aufgegeben.

Am Kopf der Waschkolonne entweicht "Propan-gewaschenes" Restgas (Abgas), das bei einem Druck von 20 bar und einer Temperatur von 30 °C sowie einer Menge von 0,69 Nm³/h folgende Gehalte aufweist:

| | |
|---|---|
| Propan | 1,38 Vol.-%, |
| Propylen | 0,04 Vol.-%, |
| Ehtylen | 4,48 Vol.-%, |
| Methan | 13,44 Vol.-%, |
| $H_2$ | 27,12 Vol.-%, |
| $O_2$ | 18,89 Vol.-%, |
| $H_2O$ | 1,04 Vol.-%, |
| CO | 10,16 Vol.-%, und |
| $CO_2$ | 22,44 Vol.-%. |

Dieses Abgas wird wie beschrieben entspannt sowie indirektem Wärmeaustausch unterworfen und dann der Rückstandsverbrennung zugeführt bzw. über eine Fackel verbrannt.

Dem Sumpf der "Propan-Waschkolonne", die von außen weder gekühlt noch beheizt wird, werden 24,27 kg/h an Absorbat entnommen, das einen Druck von 20 bar und eine Temperatur von 61 °C aufweist. Es enthält 15,63 Gew.-% Propan und 0,41 Gew.-% Propylen.

Bevor das Absorbat zum Kopf der nachfolgenden Desorptionskolonne geführt wird, wird es in einem indirekten Wärmeaustauscher auf 69 °C erwärmt. Als Wärmeträger wird der flüssige Ablauf aus der Desorptionskolonne verwendet (20,39 kg/h), der bei einem Druck von 3,26 bar eine Temperatur von 80 °C aufweist und noch 0,089 Gew.-% Propan gelöst enthält. Mittels einer Pumpe wird er wieder auf 20 bar komprimiert und nach indirekter Abkühlung mit Oberflächenwasser (25 °C) auf 30 °C zur Propan-Absorption auf den Kopf der "Propan-Waschkolonne" rückgeführt.

Nach der Erwärmung auf 69 °C wird das Absorbat (z. B. in einer inversen Pumpe oder mittels eines Ventils) auf einen Druck von 3,26 bar entspannt (die im Fall der inversen Pumpe dabei frei gesetzte mechanische Energie wird zweckmäßig zur Rückverdichtung von in der Desorptionskolonne von Propan befreitem Absorptionsmittel (dem flüssigen Ablauf der Desorptionskolonne) mitverwendet) und das dabei erzeugte zweiphasige Gemisch am Kopf in die Desorptionskolonne geführt.

In die Desorptionskolonne (ebenfalls eine Füllkörperkolonne) wird als Stripgas von unten im Gegenstrom zum vom Desorptionskolonnenkopf ablaufenden Absorbat mit einem Druck von 5 bar und einer Temperatur von 152 °C 0,1 kg/h Wasserdampf zugeführt.

In der Desorptionskolonne befindet sich eine Heizschlange, durch die im Gegenstrom zum in der Desorptionskolonne aufsteigenden Wasserdampf ebenfalls Wasserdampf der Temperatur 152 °C, P = 5,00 bar in einer Menge von 0,8 kg/h geführt wird.

Am Kopf der Desorptionskolonne entweichen 2,04 Nm³/h an Propankreisgas, das eine Temperatur von 69 °C und einen Arbeitsdruck von 3,26 bar aufweist. Nach Erwärmung auf 400 °C durch indirekten Wärmeaustausch mit Produktgas A der Temperatur T = 476 °C, wird das Propankreisgas als Treibstrahl zum Betrieb der Strahlpumpe eingesetzt, mit der das Dehydrierkreisgas und das Propankreisgas im Gemisch der Beschickung der Reaktionszone A mit Reaktionsgas A-Ausgangsgemisch zugeführt werden.

Das Propankreisgas weist folgende Gehalte auf:

| | |
|---|---|
| Propan | 93,07 Vol.-%, |
| Propylen | 2,52 Vol.-%, und |
| $H_2O$ | 3,40 Vol.-%. |

IV. Drittes beispielhaftes Verfahren zur Herstellung von Acrylsäure aus Propan (beschrieben wird ein stationärer Betriebszustand)

Die Reaktionszone A ist ein wie unter II beschriebener Hordenreaktor.

Die heterogen katalysierte partielle Propandehydrierung wird in diesem Hordenreaktor im geraden Durchgang ohne Dehydrierkreisgasfahrweise durchgeführt.

Die Belastung der Katalysatorgesamtmenge (ohne Inertmaterial gerechnet) aller Horden mit Propan beträgt 1500 Nl/l•h.

Dem in Strömungsrichtung ersten Katalysatorbett werden 3,80 Nm³/h Reaktionsgas A-Beschickungsgasgemisch (Ausgangsgemisch; T = 418 °C; P = 1,77 bar) zugeführt, das folgende Gehalte aufweist:

| | |
|---|---|
| Propan | 75,21 Vol.-%, |
| Propylen | 1,35 Vol.-%, |
| Methan | 0,01 Vol.-%, |
| $H_2$ | 8,60 Vol.-%, |
| $O_2$ | 4,32 Vol.-%, |
| $N_2$ | 0,96 Vol.-%, |
| $H_2O$ | 7,68 Vol.-%, |
| CO | 0,02 Vol.-%, und |
| $CO_2$ | 0,83 Vol.-%. |

Das Reaktionsgas A-Beschickungsgasgemisch ist eine Mischung aus einem ersten (1,59 $Nm^3$/h) und einem zweiten (2,21 $Nm^3$/h) Gasgemisch, das durch zusammenführen der beiden Gasgemische über einen statischen Mischer erzeugt wird. Das erste Gasgemisch weist eine Temperatur von 450 °C und einen Druck von 1,77 bar auf, und ist zusammengesetzt aus:

1,02 $Nm^3$/h Roh-Propan, das zu > 98 Vol.-% Propan (Frischpropan) enthält (T = 237 °C; P = 2,90 bar),

0,16 $Nm^3$/h $O_2$ (T = 143 °C; P = 1,77 bar, Reinheit > 99 Vol.-%) und

0,41 $Nm^3$/h an überwiegend molekularem Wasserstoff enthaltendem Gas, das aus dem "Propan-gewaschenen" Restgas (Abgas) durch Membranabtrennung abgetrennt wird (T = 30 °C; es wird auf 1,77 bar eingedrosselt).

Durch indirekten Wärmeaustausch in einem ersten Wärmeaustauscher mit aus der Reaktionszone A heraus- und in Richtung der Reaktionszone B geführtem Produktgas A wird die Temperatur von 450 °C eingestellt.

Das zweite Gasgemisch ist Propankreisgas (2,21 $Nm^3$/h; 400 °C; 1,77 bar). Durch indirekten Wärmeaustausch mit dem im ersten Wärmeaustauscher auf eine Temperatur von 500 °C abgekühlten Produktgas A wird die Temperatur des Propankreisgases eingestellt.

Die Eintrittstemperatur des Reaktionsgas A-Beschickungsgasgemischs und die Schütthöhe des vom Reaktionsgas A-Beschickungsgasgemisch durchströmten ersten Katalysatorbetts werden so eingestellt, dass das Reaktionsgas A dieses Katalysatorbett mit einer Temperatur von 507 °C und einem Druck von 1,76 bar mit folgenden Gehalten verlässt:

| | |
|---|---|
| Propan | 64,42 Vol.-%, |
| Propylen | 9,10 Vol.-%, |
| Ethylen | 0,079 Vol.-%, |
| Methan | 0,33 Vol.-%, |
| $H_2$ | 7,63 Vol.-%, |
| $O_2$ | 0 Vol.-%, |
| $N_2$ | 0,92 Vol.-%, |
| $H_2O$ | 15,69 Vol.-%, |
| CO | 0 Vol.-%, und |
| $CO_2$ | 0,82 Vol.-%. |

Die Verlassmenge beträgt 3,95 $Nm^3$/h.

Hinter dem ersten Katalysatorbett werden dem Reaktionsgas A 0,13 $Nm^3$/h an molekularem Sauerstoff (Reinheit > 99 Vol.-%) zudosiert (T = 143 °C; P = 2,40 bar). Die Zudosierung erfolgt durch Eindrosseln, so dass der resultierende Druck des resultierenden Reaktionsgases A nach wie vor 1,76 bar beträgt.

Die Schütthöhe des zweiten Katalysatorbetts ist so beschaffen, dass das Reaktionsgas A das zweite Katalysatorbett mit einer Temperatur von 544 °C und einem Druck von 1,75 bar mit folgenden Gehalten verlässt:

| | |
|---|---|
| Propan | 51,72 Vol.-%, |
| Propylen | 15,97 Vol.-%, |
| Ethylen | 0,149 Vol.-%, |
| Methan | 0,60 Vol.-%, |

(fortgesetzt)

| | |
|---|---|
| $H_2$ | 8,68 Vol.-%, |
| $O_2$ | 0 Vol.-%, |
| $N_2$ | 0,85 Vol.-%, |
| $H_2O$ | 20,26 Vol.-%, |
| CO | 0 Vol.-%, und |
| $CO_2$ | 0,76 Vol.-%. |

Die Verlassmenge beträgt 4,29 $Nm^3$/h.

Diesem Reaktionsgas A werden vor dem vor dem dritten Katalysatorbett befindlichen statischen Mischer 0,12 $Nm^3$/h an molekularem Sauerstoff (Reinheit > 99 Vol.-%) zudosiert (T = 143 °C, auf einen Druck von 1,75 bar eingedrosselt). Der resultierende Druck des resultierenden Reaktionsgases beträgt weiterhin 1,75 bar.

Die Schütthöhe des dritten Katalysatorbetts ist so beschaffen, dass das Reaktionsgas A das dritte Katalysatorbett als Produktgas A mit einer Temperatur von 571 °C und einem Druck von 1,73 bar mit folgenden Gehalten verlässt:

| | |
|---|---|
| Propan | 40,12 Vol.-%, |
| Propylen | 22,2 Vol.-%, |
| Ethylen | 0,22 Vol.-%, |
| Methan | 0,87 Vol.-%, |
| $H_2$ | 10,11 Vol.-%, |
| $O_2$ | 0 Vol.-%, |
| $N_2$ | 0,79 Vol.-%, |
| $H_2O$ | 23,96 Vol.-%, |
| CO | 0 Vol.-%, und |
| $CO_2$ | 0,70 Vol.-%. |

Das Produktgas A wird aus der Reaktionszone A herausgeführt und zunächst durch einen ersten indirekten Wärmeaustausch mit dem am Reaktionsgas A-Ausgangsgemisch (Beschickungsgasgemisch) beteiligten ersten Gasgemisch aus Roh-Propan , molekularen Wasserstoff enthaltendem Gas und molekularem Sauerstoff von 571 °C auf 500 °C abgekühlt. Der Arbeitsdruck des Produktgases A bleibt dabei im wesentlichen erhalten. Das erste Gasgemisch wird dabei von 213 °C auf 450 °C erwärmt, während der Arbeitsdruck dieses Gasgemisches von 1,77 bar im wesentlichen erhalten bleibt.

In einem nachfolgenden zweiten indirekten Wärmeaustausch mit Propankreisgas aus der Trennzone IV, wird das Produktgas A von 500 °C auf 337 °C abgekühlt.
Die Temperatur des Propankreisgases steigt umgekehrt von 69 °C auf 400 °C.

Anschließend werden aus dem auf 337 °C abgekühlten Produktgas A 0,79 $Nm^3$/h Wasserdampf kondensativ abgetrennt. Dazu wird das auf 307 °C abgekühlte Produktgas A zunächst zu 3,85 $Nm^3$/h an Produktgas A* (T = 54 °C; P = 1,73 bar) aus dem zuvor die entsprechende Wasserdampfmenge bereits kondensativ abgetrennt worden ist, im indirekten Wärmeaustausch geführt und dabei auf 120 °C abgekühlt.

Die Temperatur das Produktgases A* erhöht sich gleichzeitig auf 307 °C.

Durch Direktkühlung mit versprühtem gekühltem (auf 29 °C), zuvor aus Produktgas A durch entsprechende Direktkühlung abgetrenntem, wässrigem Kondensat, wird die vorgenannte Wasserdampfmenge kondensativ aus dem Produktgas A abgetrennt und ein Produktgas A* erzeugt (P = 1,73 bar; T = 54 °C; 3,85 $Nm^3$/h).
Dieses wird wie vorstehend beschrieben auf 307 °C erwärmt. Dann werden in das Produktgas A* 1,41 $Nm^3$/h molekularer Sauerstoff (Reinheit > 99 Vol.-%; T = 143 °C; P = 2,40 bar) eingedrosselt. Dadurch entsteht ein Reaktionsgas B-Ausgangsgemisch (Beschickungsgasgemisch), das eine Temperatur von 290 °C und einen Arbeitsdruck von 1,63 bar aufweist.
Das Reaktionsgas B-Ausgangsgemisch (Beschickungsgasgemisch) weist folgende Gehalte auf:

| | |
|---|---|
| Propan | 35,35 Vol.-%; |
| Propylen | 19,60 Vol.-%, |
| Ethylen | 0,19 Vol.-%, |
| Methan | 0,76 Vol.-%, |
| $H_2$ | 8,91 Vol.-%, |

(fortgesetzt)

| | |
|---|---|
| $O_2$ | 26,60 Vol.-%, |
| $N_2$ | 0,69 Vol.-%, |
| $H_2O$ | 6,27 Vol.-%, |
| CO | 0 Vol-%, und |
| $CO_2$ | 0,62 Vol.-%. |

Mit ihm wird die Reaktionszone B beschickt, die wie diejenige aus II. aufgebaut ist. Die Propylenbelastung der Katalysatorbeschickung der ersten Oxidationsstufe wird zu 130 Nl/l•h gewählt. Die Salzschmelzen (53 Gew.-% $KNO_3$, 40 Gew.-% $NaNO_2$, 7 Gew.-% $NaNO_3$) weisen folgende Eintrittstemperaturen auf:

$$T_A = 322\ °C \qquad T_B = 327\ °C$$
$$T_C = 261\ °C \qquad T_D = 268\ °C$$

Dem Produktgasgemisch der ersten Oxidationsstufe wird soviel molekularer Sauerstoff (143 °C; 2,40 bar) zudosiert (eingedrosselt), dass das molare Verhältnis $O_2$ : Acrolein im resultierenden Beschickungsgasgemisch für die zweite Oxidationsstufe 0,745 beträgt. Die Acroleinbelastung der Katalysatorbeschickung der zweiten Oxidationsstufe beträgt 110 Nl/l•h. Der Druck am Eingang der zweiten Oxidationsstufe beträgt 1,57 bar. Das Reaktionsgas B verlässt den Zwischenkühler mit einer Temperatur von 260 °C und die Eintrittstemperatur des Beschickungsgasgemischs in die zweite Oxidationsstufe beträgt 256 °C.

Das Produktgasgemisch der ersten Oxidationsstufe weist folgende Gehalte auf:

| | |
|---|---|
| Acrolein | 16,51 Vol.-%, |
| Acrylsäure | 1,11 Vol.-%, |
| Propan | 35,23 Vol.-%, |
| Propylen | 0,98 Vol.-%, |
| Ethylen | 0,19 Vol.-%, |
| Methan | 0,76 Vol.-%, |
| $H_2$ | 8,88 Vol.-%, |
| $O_2$ | 4,57 Vol.-%, |
| $N_2$ | 0,69 Vol.-%, |
| $H_2O$ | 26,66 Vol.-%, |
| CO | 0,83 Vol.-%, und |
| $CO_2$ | 2,56 Vol.-%. |

Die Temperatur des Produktgases der ersten Oxidationsstufe beträgt vor Eintritt in den Nachkühler 335 °C.
Das Produktgasgemisch der zweiten Oxidationsstufe (das Produktgas B) weist eine Temperatur von 270 °C und einen Druck von 1,55 bar sowie die folgenden Gehalte auf:

| | |
|---|---|
| Acrolein | 0,08 Vol.-%, |
| Acrylsäure | 16,71 Vol.-%, |
| Essigsäure | 0,495 Vol.-%, |
| Propan | 35,20 Vol.-%, |
| Propylen | 0,89 Vol.-%, |
| Ethylen | 0,19 Vol.-%, |
| Methan | 0,76 Vol.-%, |
| $H_2$ | 8,87 Vol.-%, |
| $O_2$ | 2,86 Vol.-%, |
| $N_2$ | 0,69 Vol.-%, |
| $H_2O$ | 27,28 Vol.-%, |
| CO | 1,33 Vol.-%, und |
| $CO_2$ | 3,54 Vol.-%. |

Das Produktgas B (5,29 Nm$^3$/h) wird wie in der WO 2004/035514 beschrieben in einer Bodenkolonne (Trennzone II) fraktionierend kondensiert.

Der Rückstandsverbrennung werden als erster Brennstoff 13,8 g/h Schwersieder (Polyacrylsäuren (Michael-Addukte) etc.) zugeführt.

Vom zweiten Fangboden oberhalb der Zufuhr des Produktgases B in die Bodenkolonne werden 2,80 kg/h einer kondensierten rohen Acrylsäure entnommen, die eine Temperatur von 15 °C und 96,99 Gew.-% Acrylsäure aufweist. Diese wird wie in der WO 2004/035514 beschrieben nach Zugabe einer geringen Menge Wasser suspensionskristallisiert, das Suspensionskristallisat in hydraulischen Waschkolonnen von der Mutterlauge getrennt und die Mutterlauge wie in der WO 2004/035514 beschrieben in die Kondensationskolonne rückgeführt.

Die Reinheit des gewaschenen Suspensionskristallisats beträgt > 99,87 Gew.-% Acrylsäure und eignet sich unmittelbar zur Herstellung von Wasser "superabsorbierenden" Polymerisaten zur Verwendung im Hygienebereich.

Die vom dritten Fangboden oberhalb der Zufuhr des Produktgases B in die Kondensationskolonne entnommene, nicht in die Kondensationskolonne rückgeführte Menge an Sauerwasserkondensat beträgt 1,25 kg/h, weist eine Temperatur von 33 °C auf und hat folgende Gehalte:

| | |
|---|---|
| Acrolein | 0,72 Gew.-%, |
| Acrylsäure | 4,95 Gew.-%, |
| Essigsäure | 5,09 Gew.-%, und |
| Wasser | 88,24 Gew.-%. |

Am Kopf der Kondensationskolonne verlassen 2,98 Nm$^3$/h an Restgas die Trennzone II mit einer Temperatur von 33 °C und einem Druck von 1,20 bar und folgenden Gehalten:

| | |
|---|---|
| Acrolein | 0,02 Vol.-%, |
| Acrylsäure | 0,02 Vol.-%, |
| Propan | 62,39 Vol.-%, |
| Propylen | 1,73 Vol.-%, |
| Ethylen | 0,34 Vol.-%, |
| Methan | 1,35 Vol.-%, |
| $H_2$ | 16,23 Vol.-%, |
| $O_2$ | 5,05 Vol.-%, |
| $N_2$ | 1,27 Vol.-%, |
| $H_2O$ | 1,92 Vol.-%, |
| CO | 2,36 Vol.-%, und |
| $CO_2$ | 6,33 Vol.-%. |

In der ersten Verdichterstufe eines mehrstufigen Radialverdichters wird das Restgas von 1,20 bar auf 6,93 bar verdichtet, wobei die Temperatur des Restgases auf 134 °C ansteigt.

In einem indirekten Wärmeaustauscher wird es auf 123 °C abgekühlt, ohne dass dabei Kondensatbildung erfolgt (Kühlmittel ist das "Propan-gewaschene" Restgas (im folgenden auch als "Abgas" bezeichnet), das nach Abtrennung von molekularem Wasserstoff über eine Trennmembran sowie Erwärmung in einem indirekten Wärmeaustauscher von 30 °C auf 102 °C in der ersten Expansionsstufe einer mehrstufigen Expansionsturbine von 20 bar ausgehend entspannt wird und dabei abkühlt). Das Abgas (0,59 Nm$^3$/h) erwärmt sich dabei. In einer zweiten Expansionsstufe wird das Abgas auf 1,10 bar entspannt (es hat dann eine Temperatur von 23 °C) und anschließend der Rückstandsverbrennung zugeführt.

In einem indirekten Luftkühler wird das verdichtete Restgas (P = 6,93 bar und T = 134 °C) auf 59 °C abgekühlt. In einer weiteren Verdichterstufe wird es von 6,93 bar auf 20 bar verdichtet, wobei es sich gleichzeitig auf 117 °C erwärmt. In einem nachfolgenden indirekten Wärmeaustauscher wird es auf 110°C abgekühlt (Kühlmittel ist das "Propan-gewaschene" Restgas, nach erfolgter $H_2$-Membranabtrennung, das sich dabei von 30 °C auf 102 °C erwärmt) ohne dass dabei Kondensatbildung erfolgt. In einem sich anschließenden weiteren indirekten Wärmeaustauscher wird das 110 °C aufweisende Restgas auf 35 °C abgekühlt (Kühlmittel ist ca. 25 °C aufweisendes Oberflächenwasser).

Dabei kondensieren 44,2 g/h Wasser aus dem Restgas aus. Dieses wässrige Kondensat wird ebenfalls der Rückstandsverbrennung zugeführt und in selbiger gemeinsam mit den anderen genannten Rückständen unter Zufuhr von 4,12 Nm$^3$/h Luft verbrannt. Die heißen Verbrennungsgase werden unter Erzeugung von Wasserdampf (52 bar; 267 °C; 3,41 kg/h) abgekühlt und in die Umgebung entlassen.

Die jetzt noch 2,93 Nm$^3$/h an Propan enthaltendem Restgas (20 bar, 35 °C) werden in den unteren Teil einer Füllkörperkolonne (Trennzone III) geführt. Am Kopf dieser Waschkolonne werden 21,62 kg/h mit einer Aufgabetemperatur von 30 °C (P = 20 bar) technisches Tetradecan der Fa. Haltermann, DE (wie in II. beschrieben) aufgegeben.

Am Kopf der Waschkolonne entweicht "Propan-gewaschenes" Restgas (Abgas), das bei einem Druck von 20 bar und einer Temperatur von 30 °C bei einer Menge von 1,01 Nm$^3$/h, folgende Gehalte aufweist:

|  |  |
|---|---|
| Propan | 0,92 Vol.-%, |
| Propylen | 0,03 Vol.-%, |
| Ethylen | 0,99 Vol.-%, |
| Methan | 3,99 Vol.-%, |
| $H_2$ | 47,97 Vol.-%, |
| $O_2$ | 14,94 Vol.-%, |
| $N_2$ | 3,75 Vol.-%, |
| $H_2O$ | 0,78 Vol.-%, |
| CO | 6,95 Vol.-%, und |
| $CO_2$ | 18,69 Vol.-%. |

Dieses "Propan-gewaschene" Abgas wird auf ein Bündel eingegossener Schlauchmembranen (Außendruck: 1,77 bar; Polyimidmembran vom Typ B-H der Fa. UBE Industries Ltd.) geführt und verlässt selbiges (P = 20 bar; T = 30 °C) unter Abgabe von 0,41 Nm$^3$/h eines Gasstromes (Permeatstromes), der folgende Gehalte aufweist:

|  |  |
|---|---|
| Ethylen | 0,02 Vol.-%, |
| Methan | 0,059 Vol.-%, |
| $H_2$ | 79,15 Vol.-%, |
| $O_2$ | 1,53 Vol.-%, |
| $N_2$ | 8,83 Vol.-%, |
| $H_2O$ | 1,52 Vol.-%, |
| CO | 0,22 Vol.-%, und |
| $CO_2$ | 7,68 Vol.-%. |

Dieser Gasstrom wird dem ersten Gasgemisch zur Erzeugung des Reaktionsgas A-Beschickungsgasgemischs zugeführt (eingedrosselt).

Das verbliebene Abgas wird wie beschrieben entspannt sowie indirektem Wärmeaustausch unterworfen und dann der Rückstandsverbrennung zugeführt bzw. in einer Fackel verbrannt.

Dem Sumpf der "Propan-Waschkolonne", die von außen weder gekühlt noch beheizt wird, werden 25,40 kg/h an Absorbat entnommen, das einen Druck von 20 bar und eine Temperatur von 59 °C aufweist. Es enthält 14,40 Gew.-% Propan und 0,38 Gew.-% Propylen.

Bevor das Absorbat zum Kopf der nachfolgenden Desorptionskolonne geführt wird, wird es in einem indirekten Wärmeaustauscher auf 69 °C erwärmt. Als Wärmeträger wird der flüssige Ablauf aus der Desorptionskolonne verwendet (21,62 kg/h), der bei einem Druck von 3,26 bar eine Temperatur von 80 °C aufweist und noch 0,09 Gew.-% Propan gelöst enthält. Mittels einer Pumpe wird er wieder auf 20 bar komprimiert und nach indirekter Abkühlung mit Oberflächenwasser (25 °C) auf 30 °C zur Propan-Absorption auf den Kopf der "Propan-Waschkolonne" rückgeführt.

Nach Erwärmung auf 69 °C wird das Absorbat (z. B. in einer inversen Pumpe oder mittels eines Ventils) auf einen Druck von 3,26 bar entspannt (die im Fall der inversen Pumpe dabei frei gesetzte mechanische Energie wird zweckmäßig zur Rückverdichtung von in der Desorptionskolonne von Propan befreitem Absorptionsmittel (dem flüssigen Ablauf der Desorptionskolonne) mitverwendet) und das dabei erzeugte zweiphasige Gemisch am Kopf in die Desorptionskolonne geführt.

In die Desorptionskolonne (ebenfalls eine Füllkörperkolonne) wird als Stripgas von unten im Gegenstrom zum vom Desorptionskolonnenkopf ablaufenden Absorbat mit einem Druck von 5 bar und einer Temperatur von 152 °C 0,2 kg/h Wasserdampf zugeführt.

In der Desorptionskolonne befindet sich eine Heizschlange, durch die im Gegenstrom zum in der Desorptionskolonne aufsteigenden Wasserdampf ebenfalls Wasserdampf der Temperatur 152 °C, P = 5,00 bar in einer Menge von 0,8 kg/h geführt wird.

Am Kopf der Desorptionskolonne entweichen 2,21 Nm$^3$/h an Propankreisgas, das eine Temperatur von 69 °C und einen Arbeitsdruck von 3,26 bar aufweist.

Nach Erwärmung auf 400 °C durch indirekten Wärmeaustausch mit Produktgas A der Temperatur T = 476 °C, wird das Propankreisgas der Beschickung der Reaktionszone A mit Reaktionsgas A-Ausgangsgemisch zugeführt. Das Propankreisgas weist folgende Gehalte auf:

| | |
|---|---|
| Propan | 83,73 Vol.-%, |
| Propylen | 2,32 Vol.-%, und |
| $H_2O$ | 12,94 Vol.-% |

V. Viertes beispielhaftes Verfahren zur Herstellung von Acrylsäure aus Propan (beschrieben wird ein stationärer Betriebszustand)

Die Reaktionszone A ist ein wie unter II. beschriebener Hordenreaktor, hinter den jedoch in diesem Beispiel ein weiterer adiabat gestalteter Schachtofenreaktor geschaltet ist, der nur ein Katalysatorfestbett aufweist, das in seiner Gestaltung der in Strömungsrichtung ersten Festbetthorde im Hordenreaktor entspricht. Zwischen die beiden Reaktoren ist ein indirekter Wärmeaustauscher geschaltet. Die heterogen katalysierte partielle Propandehydrierung wird im Hordenreaktor im geraden Durchgang mit Dehydrierkreisgasfahrweise durchgeführt. Die Belastung der Katalysatorgesamtmenge (ohne Inertmaterial gerechnet) aller Horden mit Propan beträgt 1500 Nl/l•h. Im nachgeschalteten Schachtofenreaktor erfolgt im wesentlichen nur eine heterogen katalysierte Verbrennung von molekularem Wasserstoff.

Dem in Strömungsrichtung ersten Katalysatorbett des Hordenreaktors werden 6,93 $Nm^3$/h Reaktionsgas A-Beschickungsgasgemisch (Ausgangsgemisch; P = 2,46 bar) zugeführt, das folgende Gehalte aufweist:

| | |
|---|---|
| Propan | 60,54 Vol.-%, |
| Propylen | 10,38 Vol.-%, |
| Ethylen | 0,28 Vol.-%, |
| Methan | 0,87 Vol.-%, |
| $H_2$ | 2,44 Vol.-%, |
| $O_2$ | 1,19 Vol.-%, |
| $N_2$ | 16,48 Vol.-%, |
| $H_2O$ | 6,82 Vol.-%, |
| CO | 0 Vol.-%, und |
| $CO_2$ | 0,01 Vol.-%. |

Das Reaktionsgas A-Beschickungsgasgemisch ist eine Mischung aus einem ersten (1,47 $Nm^3$/h) und einem zweiten (5,46 $Nm^3$/h) Gasgemisch, das durch zusammenführen der beiden Gasgemische über einen statischen Mischer erzeugt wird.

Das erste Gasgemisch weist eine Temperatur von 500 °C und einen Druck von 2,46 bar auf, und ist zusammengesetzt aus:

1,06 $Nm^3$/h    Roh-Propan, das zu > 98 Vol.-% Propan (Frischpropan) enthält (T = 237 °C; P = 2,90 bar);

und

0,41 $Nm^3$/h    verdichtete Luft (T = 159 °C; P = 2,66 bar).

Durch indirekten Wärmeaustausch (im zwischen beiden Reaktoren befindlichen Zwischenkühler, Wärmeaustauscher) mit aus dem Hordenreaktor heraus- und in Richtung des nachgeschalteten Schachtreaktor geführtem Reaktionsgas A wird die Temperatur von 500 °C eingestellt.

Das zweite Gasgemisch ist zusammengesetzt aus:

3,48 $Nm^3$/h    Dehydrierkreisgas (T = 554 °C; P = 2,37 bar),

und

1,98 $Nm^3$/h    Propankreisgas (T = 500 °C; P = 2,97 bar)

Es weist einen Druck von 2,46 bar und eine Temperatur von 529 °C auf.

Durch indirekten Wärmeaustausch mit aus dem nachgeschalteten Schachtreaktor geführten Produktgas A wird die Temperatur des Propankreisgases eingestellt.

Die Dehydrierkreisgasführung erfolgt nach dem Strahlpumpenprinzip (vgl. DE-A 10211275), wobei das auf 500 °C erwärmte Propankreisgas als Treibstrahl fungiert.

Die Eintrittstemperatur des Reaktionsgas A-Beschickungsgasgemischs und die Schütthöhe des vom Reaktionsgas A-Beschickungsgasgemisch in Strömungsrichtung im Hordenreaktor durchströmten ersten Katalysatorbett werden so eingestellt, dass das Reaktionsgas A dieses Katalysatorbett mit einer Temperatur von 522 °C und einem Druck von 2,44 bar mit folgenden Gehalten verlässt:

| | |
|---|---|
| Propan | 53,77 Vol.-%, |
| Propylen | 14,45 Vol.-%, |
| Ethylen | 0,29 Vol.-%, |
| Methan | 1,26 Vol.-%, |
| $H_2$ | 4,31 Vol.-%, |
| $O_2$ | 0 Vol.-%, |
| $N_2$ | 15,91 Vol.-%, |
| $H_2O$ | 8,88 Vol.-%, |
| CO | 0 Vol.-%, |
| $CO_2$ | 0,01 Vol.-%. |

Die Verlassmenge beträgt 7,18 $Nm^3$/h. Hinter dem ersten Katalysatorbett des Hordenreaktors werden dem Reaktionsgas A 0,64 $Nm^3$/h Luft zudosiert (T = 159 °C; P = 2,66 bar). Die Zudosierung erfolgt durch Eindrosseln, so dass der resultierende Druck des resultierenden Reaktionsgases A nach wie vor 2,44 bar beträgt.

Die Schütthöhe des zweiten Katalysatorbetts des Hordenreaktors ist so beschaffen, dass das Reaktionsgas A das zweite Katalysatorbett mit einer Temperatur von 539 °C und einem Druck von 2,41 bar mit folgenden Gehalten verlässt:

| | |
|---|---|
| Propan | 43,71 Vol.-%, |
| Propylen | 17,02 Vol.-%, |
| Ethylern | 0,51 Vol.-%, |
| Methan | 1,52 Vol.-%, |
| $H_2$ | 4,53 Vol.-%, |
| $O_2$ | 0 Vol.-%, |
| $N_2$ | 20,35 Vol.-%, |
| $H_2O$ | 11,37 Vol.-%, |
| CO | 0 Vol.-%, und |
| $CO_2$ | 0,01 Vol.-%. |

Die Verlassmenge beträgt 8,04 $Nm^3$/h.

Diesem Reaktionsgas A werden vor dem vor dem dritten Katalysatorbett des Hordenreaktors befindlichen statischen Mischer 0,63 $Nm^3$/h an Luft zudosiert (T = 159 °C, auf einem Druck von 2,66 bar befindlich eingedrosselt). Der resultierende Druck des resultierenden Reaktionsgases A beträgt weiterhin 2,41 bar.

Die Schütthöhe des dritten Katalysatorbetts des Hordenreaktors ist so beschaffen, dass das Reaktionsgas A das dritte Katalysatorbett mit einer Temperatur von 554 °C und einem Druck von 2,37 bar mit folgenden Gehalten verlässt:

| | |
|---|---|
| Propan | 35,58 Vol.-%, |
| Propylen | 19,15 Vol.-%, |
| Ethylen | 0,57 Vol.-%, |
| Methan | 1,72 Vol.-%, |
| $H_2$ | 4,82 Vol.-%, |
| $O_2$ | 0 Vol.-%, |
| $N_2$ | 23,83 Vol.-%, |
| $H_2O$ | 13,31 Vol.-%, |
| CO | 0 Vol.-%, und |

(fortgesetzt)

| | |
|---|---|
| $CO_2$ | 0,01 Vol.-%. |

Die Verlassmenge beträgt 8,89 Nm$^3$/h.

Durch einen Strömungsteiler wird das Reaktionsgas A am Ausgang des Hordenreaktors in zwei Teilmengen identischer Zusammensetzung aufgeteilt. Die erste Teilmenge beträgt 3,49 Nm$^3$/h und die zweite Teilmenge beträgt 5,40 Nm$^3$/h. Die erste Teilmenge wird als Bestandteil des Reaktionsgas A-Beschickungsgasgemischs in die Reaktionszone A rückgeführt. Zum Zweck der Dehydrierkreisgasführung der ersten Teilmenge wird mit dem wie beschrieben erwärmten Propankreisgas als Treibstrahl eine Strahlpumpe betrieben, wobei die Förderrichtung des durch eine Treibdüse über eine Mischstrecke und einen Diffusor entspannten Treibstrahls in Richtung des Eingangs der Reaktionszone A sowie der Saugstutzen in Richtung der ersten Strömungsteilmenge des Reaktionsgases A weist und die Verbindung "Saugstutzen-Mischstrecke-Diffusor" die alleinige Verbindungslinie zwischen der zurückzuführenden ersten Teilmenge des Reaktionsgases A und dem Zugang zur Reaktionszone A bildet.

Die zweite Teilmenge des Reaktionsgases A wird aus der Reaktionszone A herausgeführt und im zwischen dem Hordenreaktor und dem auf selbigen folgenden Schachtreaktor befindlichen Zwischenkühler durch einen ersten indirekten Wärmeaustausch mit dem am Reaktionsgas A-Ausgangsgemisch (Beschickungsgasgmisch) beteiligten ersten Gasgemisch aus Roh-Propan und molekularem Sauerstoff von 554 °C auf 473 °C abgekühlt. Das erste Gasgemisch wird dabei von 229 °C auf 500 °C erwärmt, die Arbeitsdrucke bleiben im wesentlichen erhalten.

Nun werden der wie beschrieben abgekühlten zweiten Teilmenge des Reaktionsgases A 6406 Nm$^3$/h an Luft zudosiert (T = 159 °C, von 2,66 bar befindlich eingedrosselt). Dann wird das Gasgemisch durch den nachgeschalteten Schachtreaktor geführt.

Die Schütthöhe des im nachgeschaltetem Schachtreaktor befindlichen ("vierten") Katalysatorbetts ist so beschaffen, dass das Reaktionsgas A dieses Katalysatorbett als Produktgas A mit einer Temperatur von 580 °C und einem Druck von 2,26 bar mit folgenden Gehalten verlässt:

| | |
|---|---|
| Propan | 32,53 Vol.-%, |
| Propylen | 17,48 Vol.-%, |
| Ethylen | 0,52 Vol.-%, |
| Methan | 1,57 Vol.-%, |
| $H_2$ | 0 Vol.-%, |
| $O_2$ | 0 Vol.-%, |
| $N_2$ | 30,08 Vol.-% |
| $H_2O$ | 16,79 Vol.-%, |
| CO | 0 Vol.-%, und |
| $CO_2$ | 0,01 Vol.-%. |

In einem nachfolgenden (zweiten) indirekten Wärmeaustausch mit Propankreisgas aus der Trennzone IV, wird das Produktgas A (5,92 Nm$^3$/h) von 580 °C auf 388 °C abgekühlt. Die Temperatur des Propankreisgases steigt umgekehrt von 69 °C auf 500 °C.

Anschließend werden aus dem auf 388 °C abgekühlten Produktgas A 0,82 Nm$^3$/h Wasserdampf kondensativ abgetrennt. Dazu wird das auf 388 °C abgekühlte Produktgas A zunächst zu 5,10 Nm$^3$/h an Produktgas A* (T = 40 °C; P = 2,01 bar), aus dem zuvor die entsprechende Wasserdampfmenge bereits kondensativ abgetrennt worden ist, im indirekten Wärmeaustausch geführt und dabei auf 112 °C abgekühlt. Die Temperatur des Produktgases A* erhöht sich gleichzeitig auf 358 °C. In einem nachfolgenden Luftkühler wird das Produktgas A (T = 112 °C; P = 2,26 bar) durch indirekten Wärmeaustausch mit Außenluft auf 54 °C abgekühlt (der Arbeitsdruck des Produktgases A fällt dabei auf 2,06 bar) und weist danach bereits Zweisphasigkeit auf.

Durch Direktkühlung mit versprühtem gekühltem (auf 29 °C), zuvor aus Produktgas A durch entsprechende Direktkühlung abgetrenntem, wässrigem Kondensat, wird die vorgenannte Wasserdampfmenge kondensativ aus dem Produktgas A abgetrennt und ein Produktgas A* erzeugt (P = 2,01 bar; T = 40 °C; 5,10 Nm$^3$/h). Dieses wird wie vorstehend beschrieben auf 358 °C erwärmt. Dann werden in das Produktgas A* 8,55 Nm$^3$/h Luft (T = 159 °C; P = 2,66 bar) eingedrosselt. Dadurch entsteht ein Reaktionsgas B-Ausgangsgemisch (Beschickungsgasgemisch), das eine Temperatur von 283 °C und einen Arbeitsdruck von 1,96 bar aufweist.

Das Reaktionsgas B-Ausgangsgemisch (Beschickungsgasgemisch) weist folgende Gehalte auf:

| | |
|---|---|
| Propan | 14,11 Vol.-%, |

(fortgesetzt)

| | |
|---|---|
| Propylen | 7,58 Vol.-%, |
| Ethylen | 0,23 Vol.-%, |
| Methan | 0,68 Vol.-%, |
| $H_2$ | 0 Vol.-% |
| $O_2$ | 12,69 Vol.-%, |
| $N_2$ | 60,9 Vol.-%, |
| $H_2O$ | 2,75 Vol.-%, |
| CO | 0 Vol.-%, und |
| $CO_2$ | 0,03 Vol.-%. |

Mit ihm wird die Reaktionszone B beschickt, die wie diejenige aus II. aufgebaut ist.

Die Propylenbelastung der Katalysatorbeschickung der ersten Oxidationsstufe wird zu 130 Nl/l•h gewählt. Die Salzschmelzen (53 Gew.-% $KNO_3$, 40 Gew.-% $NaNO_2$, 7 Gew.-% $NaNO_3$) weisen folgende Eintrittstemperaturen auf:

$$T_A = 327°C \qquad T_B = 329 \,°C$$
$$T_C = 267 \,°C \qquad T_D = 268 \,°C$$

Dem Produktgasgemisch der ersten Oxidationsstufe wird soviel Luft (159 °C; 2,66 bar) zudosiert (eingedrosselt), dass das molare Verhältnis $O_2$: Acrolein im resultierenden Beschickungsgasgemisch für die zwei Oxidationsstufe 1,01 beträgt.

Die Acroleinbelastung der Katalysatorbeschickung der zweiten Oxidationsstufe beträgt 110 Nl/l•h. Der Druck am Eingang der zweiten Oxidationsstufe beträgt 1,68 bar.

Das Reaktionsgas B verlässt den Zwischenkühler mit einer Temperatur von 260 °C und die Eintrittstemperatur des Beschickungsgasgemischs in die zweite Oxidationsstufe beträgt 253 °C.

Das Produktgasgemisch der ersten Oxidationsstufe weist folgende Gehalte auf:

| | |
|---|---|
| Acrolein | 6,40 Vol.-%, |
| Acrylsäure | 0,44 Vol.-%, |
| Propan | 14,09 Vol.-%, |
| Propylen | 0,39 Vol.-%, |
| Methan | 0,68 Vol.-%, |
| $H_2$ | 0 Vol.-%, |
| Ethylen | 0,23 Vol.-%, |
| $O_2$ | 4,18 Vol.-%, |
| $N_2$ | 60,87 Vol.-%, |
| $H_2O$ | 10,64 Vol.-%, |
| CO | 0,33 Vol.-%, und |
| $CO_2$ | 0,78 Vol.-%. |

Die Temperatur des Produktgases der ersten Oxidationsstufe beträgt vor Eintritt in den Nachkühler 335 °C.

Das Produktgasgemisch der zweiten Oxidationsstufe (das Produktgas B) weist eine Temperatur von 270 °C und einen Druck von 1,55 bar sowie die folgenden Gehalte auf:

| | |
|---|---|
| Acrolein | 0,03 Vol.-%, |
| Acrylsäure | 5,97 Vol.-%, |
| Essigsäure | 0,18 Vol.-%, |
| Propan | 13,0 Vol.-%, |
| Propylen | 0,36 Vol.-%, |
| Methan | 0,63 Vol.-%, |
| Ethylen | 0,21 Vol.-%, |
| $H_2$ | 0 Vol.-%, |
| $O_2$ | 2,60 Vol.-%, |

(fortgesetzt)

| | |
|---|---|
| $N_2$ | 64,2 Vol.-%, |
| $H_2O$ | 10,29 Vol.-%, |
| CO | 0,48 Vol.-%, und |
| $CO_2$ | 1,08 Vol.-%. |

Das Produktgas B (14,81 $Nm^3$/h) wird wie in der WO 2004/035514 beschrieben in einer Bodenkolonne (Trennzone II) fraktionierend kondensiert.

Der Rückstandsverbrennung werden als erster Brennstoff 13,8 g/h Schwersieder (Polyacrylsäuren (Michael-Addukte) etc.) zugeführt.

Vom zweiten Fangboden oberhalb der Zufuhr des Produktgases B in die Bodenkolonne werden 2,80 kg/h einer kondensierten rohen Acrylsäure entnommen, die eine Temperatur von 15°C und 96,99 Gew.-% Acrylsäure aufweist. Diese wird wie in der WO 2004/035514 beschrieben nach Zugabe einer geringen Menge Wasser suspensionskristallisiert, das Suspensionskristallisat in hydraulischen Waschkolonnen von der Mutterlauge getrennt und die Mutterlauge wie in der WO 2004/035514 beschrieben in die Kondensationskolonne rückgeführt. Die Reinheit des gewaschenen Suspensionskristallisats beträgt > 99,87 Gew.-% Acrylsäure und eignet sich unmittelbar zur Herstellung von Wasser "superabsorbierenden" Polymerisaten zur Verwendung im Hygienebereich.

Die vom dritten Fangboden oberhalb der Zufuhr des Produktgases B in die Kondensationskolonne entnommene, nicht in die Kondensationskolonne rückgeführte Menge an Sauerwasserkondensat beträgt 1,19 kg/h, weist eine Temperatur von 34 °C auf und hat folgende Gehalte:

| | |
|---|---|
| Acrolein | 0,35 Gew.-%, |
| Acrylsäure | 4,95 Gew.-%, |
| Essigsäure | 5,31 Gew.-%, und |
| Wasser | 88,40 Gew.-%. |

Am Kopf der Kondensationskolonne verlassen 12,56 $Nm^3$/h an Restgas die Trennzone II mit einer Temperatur von 33 °C und einem Druck von 1,20 bar und folgenden Gehalten:

| | |
|---|---|
| Acrolein | 0,02 Vol.-%, |
| Acrylsäure | 0,02 Vol.-%, |
| Essigsäure | 0 Vol.-%, |
| Propan | 15,35 Vol.-%, |
| Propylen | 0,42 Vol.-%, |
| Ethylen | 0,25 Vol.-%, |
| Methan | 0,74 Vol.-%, |
| $H_2$ | 0 Vol.-%, |
| $O_2$ | 3,07 Vol.-%, |
| $N_2$ | 75,64 Vol.-%, |
| $H_2O$ | 1,68 Vol.-%, |
| CO | 0,55 Vol.-%, und |
| $CO_2$ | 1,27 Vol.-%. |

In der ersten Verdichterstufe eines mehrstufigen Radialverdichters wird das Restgas von 1,20 bar auf 6,93 bar verdichtet, wobei die Temperatur des Restgases auf 217 °C ansteigt.

In einem indirekten Wärmeaustauscher wird es auf 111 °C abgekühlt, ohne dass dabei Kondensatbildung erfolgt (Kühlmittel ist das "Propan-gewaschene" Restgas (im folgenden auch als "Abgas" bezeichnet), das nach Erwärmung in einem indirekten Wärmeaustauscher von 30 °C auf 138 °C in der ersten Expansionsstufe einer mehrstufigen Expansionsturbine von 20 bar ausgehend entspannt wird und dabei abkühlt). Das Abgas (10,39 $Nm^3$/h) erwärmt sich dabei. In einer zweiten Expansionsstufe wird das Abgas auf 1,10 bar entspannt (es hat dann eine Temperatur von 78 °C) und anschließend der Rückstandsverbrennung zugeführt.

In einem indirekten Luftkühler wird das verdichtete Restgas (P = 6,93 bar und T = 111 °C) auf 59 °C abgekühlt. In einer zweiten Verdichterstufe wird es von 6,93 bar auf 20 bar verdichtet, wobei es sich gleichzeitig auf 168 °C erwärmt. In einem nachfolgenden indirekten Wärmeaustauscher wird es auf 101 °C abgekühlt (Kühlmittel ist das "Propan-gewaschene" Restgas, das sich dabei von 30 °C auf 138 °C erwärmt), ohne dass dabei Kondensatbildung erfolgt. In einem sich anschließenden weiteren indirekten Wärmeaustauscher wird das 101 °C aufweisende Restgas auf 35 °C abgekühlt (Kühlmittel ist ca. 25 °C aufweisendes Oberflächenwasser).

Dabei kondensieren 159,7 g/h Wasser aus dem Restgas aus. Dieses wässrige Kondensat wird ebenfalls der Rückstandsverbrennung zugeführt und in selbiger gemeinsam mit den anderen genannten Rückständen unter Zufuhr von 5,59 Nm$^3$/h Luft verbrannt. Die heißen Verbrennungsgase werden unter Erzeugung von Wasserdampf (52 bar; 267 °C; 3,78 kg/h) abgekühlt und in die Umgebung entlassen.

Die jetzt noch 12,37 Nm$^3$/h an Propan enthaltendem Restgas (20 bar, 35 °C) werden in den unteren Teil einer Füllkörperkolonne (Trennzone III) geführt.

Am Kopf dieser Waschkolonne werden 66,37 kg/h mit einer Aufgabetemperatur von 30 °C (P = 20 bar) technisches Tetradecan der Fa. Haltermann, DE, (wie in II. beschrieben) aufgegeben.

Am Kopf der Waschkolonne entweicht "Propan-gewaschenes" Restgas (Abgas), das bei einem Druck von 20 bar und einer Temperatur von 30 °C bei einer Menge von 10,39 Nm$^3$/h, folgende Gehalte aufweist:

| | |
|---|---|
| Propan | 0,19 Vol.-%, |
| Propylen | 0,01 Vol.-%, |
| Ethylen | 0,29 Vol.-%, |
| Methan | 0,89 Vol.-%, |
| $H_2$ | 0 Vol.-%, |
| $O_2$ | 3,70 Vol.-%, |
| $N_2$ | 91,38 Vol.-%, |
| $H_2O$ | 0,32 Vol.-%, |
| CO | 0,67 Vol.-%, und |
| $CO_2$ | 1,53 Vol.-%. |

Dieses Abgas wird, wie beschrieben, entspannt sowie indirektem Wärmeaustausch unterworfen und dann der Rückstandsverbrennung zugeführt bzw. in einer Fackel verbrannt.

Dem Sumpf der "Propan-Waschkolonne", die von außen weder gekühlt noch beheizt wird, werden 70,25 kg/h an Absorbat entnommen, das einen Druck von 20 bar und eine Temperatur von 41 °C aufweist. Es enthält 5,41 Gew.-% Propan und 0,14 Gew.-% Propylen.

Bevor das Absorbat zum Kopf der nachfolgenden Desorptionskolonne geführt wird, wird es in einem indirekten Wärmeaustauscher auf 69 °C erwärmt. Als Wärmeträger wird der flüssige Ablauf aus der Desorptionskolonne verwendet (66,37 kg/h), der bei einem Druck von 2,97 bar eine Temperatur von 80 °C aufweist und noch 0,03 Gew.-% Propan gelöst enthält. Mittels einer Pumpe wird er wieder auf 20 bar komprimiert und nach indirekter Abkühlung mit Oberflächenwasser (25 °C) auf 30 °C zur Propan-Absorption auf den Kopf der "Propan-Waschkolonne" rückgeführt.

Nach der Erwärmung auf 69 °C wird das Absorbat (z. B. in einer inversen Pumpe oder mittels eines Ventils) auf einen Druck von 2,97 bar entspannt (die im Fall der inversen Pumpe dabei frei gesetzte mechanische Energie wird zweckmäßig zur Rückverdichtung von in der Desorptionskolonne von Propan bereitem Absorptionsmittel (dem flüssigen Ablauf der Desorptionskolonne) mitverwendet) und das dabei erzeugte zweiphasige Gemisch am Kopf in die Desorptionskolonne geführt.

In der Desorptionskolonne befindet sich eine Heizschlange, durch die im Gleichstrom zum in der Desorptionskolonne absteigenden Absorbat Wasserdampf der Temperatur 152 °C, P = 5,00 bar, in einer Menge von 1,3 kg/h geführt wird.

Am Kopf der Desorptionskolonne entweichen 1,98 Nm$^3$/h an Propankreisgas, das eine Temperatur von 69 °C und einen Arbeitsdruck von 2,97 bar aufweist. Nach Erwärmung auf 500 °C durch indirekten Wärmeaustausch mit Produktgas A der Temperatur T = 580 °C, wird das Propankreisgas als Treibstrahl zum Betrieb der Strahlpumpe eingesetzt, mit der das Dehydrierkreisgas und das Propankreisgas der Beschickung der Reaktionszone A mit Reaktionsgas A-Ausgangsgemisch zugeführt werden.

Das Propankreisgas weist folgende Gehalte auf:

| | |
|---|---|
| Propan | 96,42 Vol.-% und |
| Propylen | 2,58 Vol.-%. |

VI. Erstes Vergleichsbeispiel eines Verfahrens zur Herstellung von Acrylsäure aus Propan (beschrieben wird ein stationärer Betriebszustand)

Die Reaktionszone A besteht aus einem adiabat gestalteten Schachtofenreaktor mit nur einem Katalysatorfestbett, das in seiner Gestaltung der in Strömungsrichtung ersten Festbetthorde im Hordenreaktor aus II. entspricht.

Die heterogen katalysierte partielle Propandehydrierung wird im Schachtreaktor im geraden Durchgang mit Dehydrierkreisgasfahrweise durchgeführt. Die Belastung der Katalysatorgesamtmenge (ohne Inertmaterial gerechnet) mit Propan beträgt 1500 Nl/l•h.

Dem Schachtofenreaktor werden 24,87 $Nm^3$/h Reaktionsgas A-Beschickungsgasgemisch (Ausgangsgemisch; T = 450°C; P = 2,82 bar) zugeführt, das folgende Gehalte aufweist:

|  |  |
|---|---|
| Propan | 22,3 Vol.-%, |
| Propylen | 3,65 Vol.-%, |
| Ethan | 0,07 Vol.-%, |
| Methan | 0,17 Vol.-%, |
| $H_2$ | 4,18 Vol.-%, |
| $O_2$ | 2,09 Vol.-%, |
| $N_2$ | 60,32 Vol.-%, |
| $H_2O$ | 5,05 Vol.-%, |
| CO | 0 Vol.-%, und |
| $CO_2$ | 1,17 Vol.-%. |

Das Reaktionsgas A-Beschickungsgasgemisch ist eine Mischung aus einem ersten (1,69 $Nm^3$/h) und einem zweiten (23,18 $Nm^3$/h) Gasgemisch, das durch zusammenführen der beiden Gasgemische über einen statischen Mischer erzeugt wird.

Das erste Gasgemisch ist ein Gemisch aus 0,013 $Nm^3$/h Wasserdampf (T = 134°C; P = 3 bar), 1,06 $Nm^3$/h Roh-Propan, das zu > 98 Vol.-% Propan (Frischpropan) enthält (T = 122°C; P = 3,50 bar) und 0,62 $Nm^3$/h molekularem Wasserstoff (Reinheit > 99 Vol.- %; T = 35°C; P = 2,20 bar) und das zweite Gasgemisch ist ein Gemisch aus 11,41 $Nm^3$/h Dehydrierkreisgas (T = 534°C; P = 2,36 bar), 11,16 $Nm^3$/h verdichtetem Restgaskreisgas (T = 444 °C; P = 2,82 bar) sowie 0,61 $Nm^3$/h verdichteter Luft (T = 175°C, P = 3,00 bar).

Durch indirekten Wärmeaustausch mit aus der Reaktionszone A heraus- und in Richtung der Reaktionszone B geführtem Produktgas A wird die Temperatur des verdichteten Restgaskreisgases von 444°C eingestellt.

Die Dehydrierkreisgasführung erfolgt nach dem Strahlpumpenprinzip (vgl. DE-A 102 112 75), wobei das auf 444°C eingestellte verdichtete Restgaskreisgas als Treibstrahl fungiert. Dem dabei resultierenden Gemisch wird die verdichtete Luft zudosiert (eingedrosselt).

Die Schütthöhe des vom Reaktionsgas A-Beschickungsgasgemisch durchströmten Katalysatorfestbetts werden so eingestellt, dass das Produktgas A dieses Katalysatorbett mit einer Temperatur von 534°C und einem Druck von 2,36 bar mit folgenden Gehalten verlässt:

|  |  |
|---|---|
| Propan | 17,83 Vol.-%, |
| Propylen | 7,51 Vol.-%, |
| Ethan | 0,15 Vol.-%, |
| Methan | 0,37 Vol.-%, |
| $H_2$ | 3,76 Vol.-%, |
| $O_2$ | 0 Vol.-%, |
| $N_2$ | 59,18 Vol.-%, |
| $H_2O$ | 9,06 Vol.-%, |
| CO | 0 Vol.-%, und |
| $CO_2$ | 1,14 Vol.-%. |

Die Verlassmenge beträgt 25,35 $Nm^3$/h.

Durch einen Strömungsteiler wird das Produktgas A am Ausgang des Schachtreaktors in zwei Teilmengen identischer Zusammensetzung aufgeteilt.

Die erste Teilmenge beträgt 11,41 $Nm^3$/h und die zweite Teilmenge beträgt 13,94 $Nm^3$/h. Die erste Teilmenge wird als Bestandteil des Reaktionsgas A-Beschickungsgasgemisches in die Reaktionszone A rückgeführt. Zum Zweck der Dehydrierkreisgasführung der ersten Teilmenge wird mit dem wie beschrieben erwärmten verdichteten Restgaskreisgas als Treibstrahl eine Strahlpumpe betrieben, wobei die Förderrichtung des durch eine Treibdüse über eine Mischstrecke und einen Diffusor entspannten Treibstrahls in Richtung Eingang in die Reaktionszone A sowie der Saugstutzen in Richtung der ersten Strömungsteilmenge des Produktgases A weist und die Verbindung "Saugstutzen-Mischstrecke-Diffusor" die alleinige Verbindungslinie zwischen der zurückzuführenden ersten Teilmenge des Produktgases A und dem Zugang zur Reaktionszone A bildet.

Die zweite Teilmenge des Produktgases A wird aus der Reaktionszone A herausgeführt und in einem hinter der selben befindlichen indirekten Wärmeaustauscher durch indirekten Wärmeaustausch mit verdichtetem Restgaskreisgas (11,16 $Nm^3$/h; T = 131°C; P = 3,00 bar) auf 317°C abgekühlt (der Arbeitsdruck bleibt im wesentlichen erhalten). Das Restgaskreisgas erwärmt sich dabei auf 444°C und mindert seinen Arbeitsdruck auf 2,82 bar.

Anschließend wird das Produktgas A in einem zweiten indirekten Wärmeaustausch mit "Propan-gewaschenem" Produktgas A (9,06 $Nm^3$/h; T = 30°C, P = 10,50 bar) auf 221 °C abgekühlt, wobei gleichzeitig eine Minderung seines Arbeitsdrucks auf 2,18 bar einhergeht.

Das "Propan-gewaschene" Produktgas A (nachfolgend auch als "Abgas" bezeichnet) erwärmt sich dabei auf 287°C und ändert seinen Arbeitsdruck auf 10,10 bar. Anschließend wird das Abgas in der ersten Expansionsstufe einer mehrstufigen Expansionsturbine von 10,10 bar auf 3,50 bar entspannt und kühlt dabei von 287°C auf 176°C ab. Das die erste Expansionsstufe verlassende Abgas wird nun in indirektem Wärmeaustausch zum 221 °C aufweisenden Produktgas A geführt. Letzteres behält dabei seinen Arbeitsdruck im wesentlichen bei und kühlt sich dabei auf 215°C ab. Das Abgas erwärmt sich dabei unter weitgehendem Beibehalt seines Arbeitsdruckes auf 191°C. Nun wird das Abgas in der zweiten Expansionsstufe der mehrstufigen Expansionsturbine auf 1,23 bar entspannt, wobei es sich auf 89°C abkühlt. Dann wird das Abgas der Rückstandsverbrennung zugeführt.

Das 215°C und 2,18 bar aufweisende Produktgas A wird anschließend zunächst in einem mit Luft gekühlten indirekten Wärmeaustauscher auf 60°C und danach in einem mit Kühlwasser gekühlten indirekten Wärmeaustauscher auf 39°C abgekühlt. Mittels Tropfenabscheidern wird das im Rahmen der Abkühlungen auskondensierende Wasser abgetrennt (0,68 kg/h).

Die verbliebenen 13,09 $Nm^3$/h an Produktgas A werden in der ersten Verdichterstufe eines mehrstufigen Radialverdichters auf 4,78 bar verdichtet. Dabei erwärmt sich das Produktgas A von 39°C auf 106°C. Nachfolgend wird das Produktgas A mittels Luft in einem indirekten Wärmeaustauscher auf 54°C abgekühlt und das dabei auskondensierende Wasser (8,0 g/h) mittels Tropfenabscheidern abgetrennt. In einer zweiten Verdichtungsstufe werden die verbliebenen 13,08 $Nm^3$/h an Produktgas A auf 10,50 bar verdichtet. Damit einher geht eine Erwärmung des Produktgases A auf 123°C. Durch Kühlung mit Luft in einem indirekten Wärmeaustauscher wird das Produktgas A danach zunächst auf 54°C und nachfolgend durch indirekten Wärmeaustausch mit Kühlwasser auf 29°C abgekühlt. Mittels Tropfenabscheidern wird das im Rahmen der Abkühlungen auskondensierende Wasser abgetrennt (0,29 kg/h). Die jetzt noch verbliebenen 12,71 $Nm^3$/h Produktgas A (deren Arbeitsdruck im wesentlichen noch immer 10,50 bar beträgt) werden in den unteren Teil einer Füllkörperkolonne geführt. Ihre Gehalte betragen:

| | |
|---|---|
| Propan | 19,55 Vol.-%, |
| Propylen | 8,24 Vol.-%, |
| Ethan | 0,16 Vol.-%, |
| Methan | 0,41 Vol.-%, |
| $H_2$ | 4,13 Vol.-%, |
| $O_2$ | 0 Vol.-%, |
| $N_2$ | 64,9 Vol.-%, |
| $H_2O$ | 0,38 Vol.-%, |
| CO | 0 Vol.-%, und |
| $CO_2$ | 1,25 Vol.-%. |

Am Kopf der Propan-Waschkolonne werden 120,32 kg/h mit einer Aufgabetemperatur von 30°C (P = 10,50 bar) technisches Tetradecan der Fa. Haltermann, DE, (wie in II. beschrieben) aufgegeben.

Am Kopf der Waschkolonne entweicht "Propan-gewaschenes" Produktgas A (Abgas), das bei einem Druck von 10,50 bar und einer Temperatur von 30°C sowie einer Menge von 9,06 $Nm^3$/h folgende Gehalte aufweist:

| | |
|---|---|
| Propan | 0,02 Vol.-%, |
| Propylen | 0,01 Vol.-%, |
| Ethan | 0,14 Vol.-%, |
| Methan | 0,56 Vol.-%, |

(fortgesetzt)

| | |
|---|---|
| $H_2$ | 5,79 Vol.-%, |
| $O_2$ | 0 Vol.-%, |
| $N_2$ | 91,0 Vol.-%, |
| $H_2O$ | 0,40 Vol.-%, |
| CO | 0 Vol.-%, und |
| $CO_2$ | 1,07 Vol.-%. |

Nach wie bereits beschriebenem indirektem Wärmeaustausch mit aus der Reaktionszone A herausgeführtem Produktgas A sowie mehrstufiger Entspannung auf 1,23 bar in einer mehrstufigen Expansionsturbine wird das Abgas der Rückstandsverbrennung zugeführt bzw. in einer Fackel verbrannt. Bevor das Abgas entspannt wird, kann es großtechnisch zweckmäßig sein, den darin enthaltenen molekularen Wasserstoff abzutrennen. Dies kann z. B. dadurch erfolgen, dass man das Abgas einer wie bereits beschriebenen Membranabtrennung unterwirft. Der so abgetrennte molekulare Wasserstoff kann vollständig oder teilweise in die Reaktionszone A rückgeführt werden (zweckmäßig als Bestandteil des Reaktionsgas A-Beschickungsgasgemisches). Alternativ kann er z. B. in Brennstoffzellen verbrannt werden.

Dem Sumpf der "Propan-Waschkolonne", die von außen weder gekühlt noch beheizt wird, werden 127,37 kg/h an Absorbat entnommen, das einen Druck von 10,5 bar und eine Temperatur von 40°C aufweist. Es enthält 3,83 Gew.-% Propan, 1,54 Gew.-% Propylen, 0,1 Gew.-% Ethan und 0,1 Gew.-% $CO_2$. Bevor das Absorbat zum Kopf der nachfolgenden Desorptionskolonne geführt wird, wird es auf einen Druck von 2,01 bar entspannt (z. B. in einer inversen Pumpe oder mittels eines Ventils; die im Fall der inversen Pumpe dabei freigesetzte mechanische Energie wird zweckmäßig zur Rückverdichtung von in der Desorptionskolonne von Propan befreitem Absorptionsmittel (dem flüssigen Ablauf der Desorptionskolonne) mitverwendet). Das dabei erzeugte zweiphasige Gemisch wird am Kopf der Desorptionskolonne in selbige geführt.

In die Desorptionskolonne (ebenfalls eine Füllkörperkolonne) werden als Stripgas von unten im Gegenstrom zum zum vom Desorptionskolonnenkopf ablaufenden Absorbat auf einen Druck von 3,00 bar verdichtete 8,00 $Nm^3/h$ Luft (diese hat sich dabei auf 175°C erwärmt) geführt.

Der flüssige Ablauf der Desorptionslolonne (120,32 kg/h) wird mittels einer Pumpe wieder auf 10,50 bar komprimiert und nach indirekter Kühlung mit Kühlwasser mit einer Temperatur von 30°C zur Propan-Absorption auf den Kopf der Propan-Waschkolonne (Absorptionskolonne) rückgeführt.

Am Kopf der Desorptionskolonne entweichen 11,64 $Nm^3/h$ Gasgemisch mit einer Temperatur von 33°C und einem Druck von 2,01 bar sowie den folgenden Gehalten:

| | |
|---|---|
| Propan | 21,33 Vol.-%, |
| Propylen | 8,99 Vol.-%, |
| Ethan | 0,07 Vol.-%, |
| $O_2$ | 13,93 Vol.-%, |
| $N_2$ | 52,6 Vol.-%, |
| $H_2O$ | 1,53 Vol.-%, |
| CO | 0 Vol.-%, und |
| $CO_2$ | 0,55 Vol.-%. |

Durch indirekten Wärmeaustausch mit Wasserdampf wird die Temperatur des Gasgemischs auf 140°C erhöht (der Arbeitsdruck verringert sich dabei auf 1,91 bar).

Mit diesem Gasgemisch wird als Reaktionsgas B-Ausgangsgemisch (Beschickungsgasgemisch) eine Reaktionszone B beschickt, die wie diejenige aus II. aufgebaut ist.

Die Propylenbelastung der Katalysatorbeschickung der ersten Oxidationsstufe wird zu 130 Nl/l•h gewählt. Die Salzschmelzen (53 Gew.-% $KNO_3$, 40 Gew.-% $NaNO_2$, 7 Gew.-% $NaNO_3$) weisen folgende Eintrittstemperaturen auf:

$$T_A = 329°C \quad T_B = 330°C$$
$$T_C = 265°C \quad T_D = 266°C$$

Das Reaktionsgas B verlässt den Zwischenkühler in einer Menge von 11,66 $Nm^3/h$ mit einer Temperatur von 260°C, einem Druck von 1,68 bar und folgenden Gehalten:

| Acrolein | 7,51 Vol.-%, |
|---|---|
| Acrylsäure | 0,51 Vol.-%, |
| Propan | 21,3 Vol.-%, |
| Propylen | 0,53 Vol.-%, |
| Ethan | 0,07 Vol.-%, |
| $O_2$ | 3,92 Vol.-%, |
| $N_2$ | 52,52 Vol.-%, |
| $H_2O$ | 10,81 Vol.-%, |
| CO | 0,38 Vol.-%, und |
| $CO_2$ | 1,43 Vol.-%. |

Ihm werden 2,17 $Nm^3$/h in einem Radialverdichter auf 3 bar verdichtete Luft (diese erwärmt sich dabei auf 175°C) zudosiert (eingedrosselt), so dass die Eintrittstemperatur des Reaktionsgases in die zweite Oxidationsstufe 252°C bei einem Eingangsdruck von 1,68 bar beträgt (Acroleinbelastung = 110 Nl/l•h).

Das Produktgasgemisch der zweiten Oxidationsstufe (das Produktgas B) weist eine Temperatur von 270°C und einen Druck von 1,55 bar sowie die folgenden Gehalte auf:

| Acrolein | 0,03 Vol.-%, |
|---|---|
| Acrylsäure | 6,62 Vol.-%, |
| Essigsäure | 0,2 Vol.-%, |
| Propan | 18,51 Vol.-%, |
| Propylen | 0,46 Vol.-%, |
| Ethan | 0,06 Vol.-%, |
| $O_2$ | 2,95 Vol.-%, |
| $N_2$ | 58,0 Vol.-%, |
| $H_2O$ | 10,0 Vol.-%, |
| CO | 0,52 Vol.-%, und |
| $CO_2$ | 1,64 Vol.-%. |

Das Produktgas B (13,42 $Nm^3$/h) wird wie in der WO 2004/035514 beschrieben, in einer Bodenkolonne fraktionierend kondensiert.

Der Rückstandsverbrennung werden 13,8 g/h Schwersieder (Polyacrylsäuren (Michael-Addukte) etc.) zugeführt.

Vom zweiten Fangboden oberhalb der Zufuhr des Produktgases B in die Bodenkolonne werden 2,82 kg/h einer kondensierten rohen Acrylsäure entnommen, die eine Temperatur von 15°C und 96,99 Gew.-% Acrylsäure aufweist. Diese wird wie in der WO 2004/035514 beschrieben, nach Zugabe einer geringen Menge Wasser suspensionskristallisiert, das Suspensionskristallisat in hydraulischen Waschkolonnen von der Mutterlauge getrennt und die Mutterlauge wie in der WO 2004/035514 beschrieben, in die Kondensationskolonne rückgeführt. Die Reinheit des gewaschenen Suspensionskristallisats beträgt > 99,87 Gew.-% Acrylsäure und eignet sich unmittelbar zur Herstellung von Wasser "superabsorbierenden" Polymerisaten zur Verwendung im Hygienebereich.

Die vom dritten Fangboden oberhalb der Zufuhr des Produktgases B in die Kondensationskolonne entnommene, nicht in die Kondensationskolonne rückgeführte Menge an Sauerwasserkondensat beträgt 1,04 kg/h und weist eine Temperatur von 33°C auf. Sie wird ebenfalls der Rückstandsverbrennung zugeführt.

Am Kopf der Kondensationskolonne verlassen 11,16 $Nm^3$/h an Restgas die Kondensationskolonne mit einer Temperatur von 33°C und einem Druck von 1,20 bar und folgenden Gehalten:

| Propan | 22,06 Vol.-%, |
|---|---|
| Propylen | 0,46 Vol.-%, |
| $O_2$ | 3,54 Vol.-%, |
| $N_2$ | 69,75 Vol.-%, |
| $H_2O$ | 1,76 Vol.-%, |
| CO | 0 Vol.-%, und |
| $CO_2$ | 1,43 Vol.-% |

Das Restgas wird in seiner Gesamtmenge auf 3 bar verdichtet (dabei erwärmt es sich auf 131 °C) und wie beschrieben nach indirektem Wärmeaustausch mit aus der Reaktionszone A herausgeführtem Produktgas A als Restgaskreisgas in die Reaktionszone A rückgeführt (als Treibstrahl für die zur Dehydrierkreisgasführung eingesetzte Strahlpumpe).

IV. Zweites Vergleichsbeispiel eines Verfahrens zur Herstellung von Acrylsäure aus Propan (beschrieben wird ein stationärer Betriebszustand)

Die Reaktionszone A besteht aus einem adiabat gestalteten Schachtofenreaktor mit nur einem Katalysatorfestbett, das in seiner Gestaltung der in Strömungsrichtung ersten Festbetthorde im Hordenreaktor aus II. entspricht.
Die heterogen katalysierte partielle Propandehydrierung wird im Schachtreaktor im geraden Durchgang mit Dehydrierkreisgasfahrweise durchgeführt. Die Belastung der Katalysatorgesamtmenge (ohne Inertmaterial gerechnet) mit Propan beträgt 1500 Nl/l·h.
Dem Schachtofenreaktor werden 23,79 Nm$^3$/h Reaktionsgas A-Beschickungsgasgemisch (Ausgangsgemisch; T = 456°C; P = 2,82 bar) zugeführt, das folgende Gehalte aufweist:

|  |  |
|---|---|
| Propan | 19,31 Vol.-%, |
| Propylen | 3,82 Vol.-%, |
| Ethan | 0,11 Vol.-%, |
| Methan | 0,33 Vol.-%, |
| $H_2$ | 4,27 Vol.-%, |
| $O_2$ | 2,14 Vol.-%, |
| $N_2$ | 62,72 Vol.-%, |
| $H_2O$ | 5,11 Vol.-%, |
| CO | 0 Vol.-%, und |
| $CO_2$ | 1,22 Vol.-%. |

Das Reaktionsgas A-Beschickungsgasgemisch ist eine Mischung aus einem ersten (1,69 Nm$^3$/h) und einem zweiten (22,10 Nm$^3$/h) Gasgemisch, das durch zusammenführen der beiden Gasgemische über einen statischen Mischer erzeugt wird.
Das erste Gasgemisch ist ein Gemisch aus 0,013 Nm$^3$/h Wasserdampf (T = 134°C; P = 3 bar), 1,08 Nm$^3$/h Roh-Propan, das zu > 98 Vol.-% Propan (Frischpropan) enthält (T = 122°C; P = 3,50 bar) und 0,60 Nm$^3$/h molekularem Wasserstoff (Reinheit > 99 Vol.-%; T = 35°C; P = 2,20 bar) und das zweite Gasgemisch ist ein Gemisch aus 10,93 Nm$^3$/h Dehydrierkreisgas (T = 546°C; P = 2,82 bar), 10,52 Nm$^3$/h verdichtetem Restgaskreis (P = 3 bar; T = 455 °C) sowie 0,65 Nm$^3$/h verdichteter Luft (T = 175°C, P = 3,00 bar).
Durch indirekten Wärmeaustausch mit aus der Reaktionszone A heraus- und in Richtung der Reaktionszone B geführtem Produktgas A wird die Temperatur des verdichteten Restgaskreisgases von 455°C eingestellt.
Die Dehydrierkreisgasführung erfolgt nach dem Strahlpumpenprinzip (vgl. DE-A 102 112 75), wobei das auf 455°C eingestellte verdichtete Restgaskreisgas als Treibstrahl fungiert. Dem dabei resultierenden Gemisch wird die verdichtete Luft zudosiert (eingedrosselt).
Die Schütthöhe des vom Reaktionsgas A-Beschickungsgasgemisch durchströmten Katalysatorfestbetts ist so beschaffen, dass das Produktgas A dieses Katalysatorbett mit einer Temperatur von 546°C und einem Druck von 2,39 bar mit folgenden Gehalten verlässt:

|  |  |
|---|---|
| Propan | 14,60 Vol.-%, |
| Propylen | 7,84 Vol.-%, |
| Ethan | 0,24 Vol.-%, |
| Methan | 0,70 Vol.-%, |
| $H_2$ | 3,79 Vol.-%, |
| $O_2$ | 0 Vol.-%, |
| $N_2$ | 61,44 Vol.-%, |
| $H_2O$ | 9,19 Vol.-%, |
| CO | 0 Vol.-%, und |
| $CO_2$ | 1,19 Vol.-%. |

Die Verlassmenge beträgt 24,29 Nm$^3$/h.

Durch einen Strömungsteiler wird das Produktgas A am Ausgang des Schachtreaktors in zwei Teilmengen identischer Zusammensetzung aufgeteilt.

Die erste Teilmenge beträgt 10,93 Nm$^3$/h und die zweite Teilmenge beträgt 13,36 Nm$^3$/h. Die erste Teilmenge wird als Bestandteil des Reaktionsgas A-Beschickungsgasgemisches in die Reaktionszone A rückgeführt. Zum Zweck der Dehydrierkreisgasführung der ersten Teilmenge wird mit dem wie beschrieben erwärmten verdichteten Restgaskreisgas als Treibstrahl eine Strahlpumpe betrieben, wobei die Förderrichtung des durch eine Treibdüse über eine Mischstrecke und einen Diffusor entspannten Treibstrahls in Richtung Eingang in die Reaktionszone A sowie der Saugstutzen in Richtung der ersten Strömungsteilmenge des Produktgases A weist und die Verbindung "Saugstutzen-Mischstrecke-Diffusor" die alleinige Verbindungslinie zwischen der zurückzuführenden ersten Teilmenge des Produktgases A und dem Zugang zur Reaktionszone A bildet.

Die zweite Teilmenge des Produktgases A wird aus der Reaktionszone A herausgeführt und in einem hinter der selben befindlichen indirekten Wärmeaustauscher durch indirekten Wärmeaustausch mit verdichtetem Restgaskreisgas (10,52 Nm$^3$/h; T = 135 °C; P = 3,00 bar) auf 330 °C abgekühlt (der Arbeitsdruck bleibt im wesentlichen erhalten). Das Restgaskreisgas erwärmt sich dabei auf 455 °C und mindert seinen Arbeitsdruck auf 2,82 bar.

Anschließend wird das Produktgas A in einem zweiten indirekten Wärmeaustausch mit "Propan-gewaschenem" Produktgas A (9,06 Nm$^3$/h; T = 30 °C, P = 10,50 bar) auf 220 °C abgekühlt, wobei gleichzeitig eine Minderung seines Arbeitsdrucks auf 2,21 bar einhergeht.

Das "Propan-gewaschene" Produktgas A (nachfolgend auch als "Abgas" bezeichnet) erwärmt sich dabei auf 300°C und ändert seinen Arbeitsdruck auf 10,10 bar. Anschlie-ßend wird das Abgas in der ersten Expansionsstufe einer mehrstufigen Expansionsturbine von 10,10 bar auf 3,50 bar entspannt und kühlt dabei von 300 °C auf 187°C ab. Das die erste Expansionsstufe verlassende Abgas wird nun in indirektem Wärmeaustausch zum 220 °C aufweisenden Produktgas A geführt. Letzteres behält dabei seinen Arbeitsdruck im wesentlichen bei und kühlt sich auf 219 °C ab. Das Abgas erwärmt sich dabei unter weitgehendem Beibehalt seines Arbeitsdruckes auf 190 °C. Nun wird das Abgas in der zweiten Expansionsstufe der mehrstufigen Expansions-turbine auf 1,23 bar entspannt, wobei es sich auf 88 °C abkühlt. Dann wird das Abgas der Rückstandsverbrennung zugeführt.

Das 219°C und 2,21 bar aufweisende Produktgas A wird anschließend zunächst in einem mit Luft gekühlten indirekten Wärmeaustauscher auf 60°C und danach in einem mit Kühlwasser gekühlten indirekten Wärmeaustauscher auf 39°C abgekühlt. Mittels Tropfenabscheidern wird das im Rahmen der Abkühlungen auskondensierende Wasser abgetrennt (0,68 kg/h).

Die verbliebenen 12,52 Nm$^3$/h an Produktgas A werden in der ersten Verdichterstufe eines mehrstufigen Radialverdichters auf 4,81 bar verdichtet. Dabei erwärmt sich das Produktgas A von 39°C auf 108°C. Nachfolgend wird das Produktgas A mittels Luft in einem indirekten Wärmeaustauscher auf 54°C abgekühlt und das dabei auskondensierende Wasser (5,7 g/h) mittels Tropfenabscheidern abgetrennt. In einer zweiten Verdichtungsstufe werden die verbliebenen 12,51 Nm$^3$/h an Produktgas A auf 10,50 bar verdichtet. Damit einher geht eine Erwärmung des Produktgases A auf 126°C. Durch Kühlung mit Luft in einem indirekten Wärmeaustauscher wird das Produktgas A danach zunächst auf 54°C und nachfolgend durch indirekten Wärmeaustausch mit Kühlwasser auf 29°C abgekühlt. Mittels Tropfenabscheider wird das im Rahmen der Abkühlungen auskondensierende Wasser abgetrennt (0,28 kg/h). Die jetzt noch verbliebenen 12,17 Nm$^3$/h Produktgas A (deren Arbeitsdruck im wesentlichen noch immer 10,50 bar beträgt) werden in den unteren Teil einer Füllkörperkolonne geführt. Ihre Gehalte betragen:

| | |
|---|---|
| Propan | 16,0 Vol.-%, |
| Propylen | 8,60 Vol.-%, |
| Ethan | 0,26 Vol.-%, |
| Methan | 0,77 Vol.-%, |
| $H_2$ | 4,17 Vol.-%, |
| $O_2$ | 0 Vol.-%, |
| $N_2$ | 67,48 Vol.-%, |
| $H_2O$ | 0,39 Vol.-%, |
| CO | 0 Vol.-%, und |
| $CO_2$ | 1,31 Vol.-%. |

Am Kopf der Propan-Waschkolonne werden 115,96 kg/h mit einer Aufgabetemperatur von 30°C (P = 10,50 bar) technisches Tetradecan der Fa. Haltermann, DE, (wie in II. beschrieben) aufgegeben.

Am Kopf der Waschkolonne entweicht "Propan-gewaschenes" Produktgas A (Abgas), das bei einem Druck von 10,50 bar und einer Temperatur von 30°C sowie einer Menge von 9,06 Nm$^3$/h folgende Gehalte aufweist:

| Propan | 0,02 Vol.-%, |
|---|---|
| Propylen | 0,01 Vol.-%, |
| Ethan | 0,22 Vol.-%, |
| Methan | 1,04 Vol.-%, |
| $H_2$ | 5,59 Vol.-%, |
| $O_2$ | 0 Vol.-%, |
| $N_2$ | 90,60 Vol.-%, |
| $H_2O$ | 0,39 Vol.-%, |
| CO | 0 Vol.-%, und |
| $CO_2$ | 1,12 Vol.-%. |

Nach wie bereits beschriebenem indirektem Wärmeaustausch mit aus der Reaktionszone A herausgeführtem Produktgas A sowie mehrstufiger Entspannung auf 1,23 bar in einer mehrstufigen Expansionsturbine wird das Abgas der Rückstandsverbrennung zugeführt. Bevor das Abgas entspannt wird, kann es großtechnisch zweckmäßig sein, den darin enthaltenen molekularen Wasserstoff abzutrennen. Dies kann z. B. dadurch erfolgen, dass man das Abgas einer wie bereits beschriebenen Membranabtrennung unterwirft. Der so abgetrennte molekulare Wasserstoff kann vollständig oder teilweise in die Reaktionszone A rückgeführt werden (zweckmäßig als Bestandteil des Reaktionsgas A-Beschickungsgasgemisches). Alternativ kann er z. B. in Brennstoffzellen verbrannt werden.

Dem Sumpf der "Propan-Waschkolonne", die von außen weder gekühlt noch beheizt wird, werden 121,93 kg/h an Absorbat entnommen, das einen Druck von 10,5 bar und eine Temperatur von 40°C aufweist. Es enthält 3,14 Gew.-% Propan, 1,61 Gew.-% Propylen, 0,01 Gew.-% Ethan und 0,09 Gew.-% $CO_2$. Bevor das Absorbat zum Kopf der nachfolgenden Desorptionskolonne geführt wird, wird es auf einen Druck von 1,98 bar entspannt (z. B. in einer inversen Pumpe oder mittels eines Ventils; die im Fall der inversen Pumpe dabei freigesetzte mechanische Energie wird zweckmäßig zur Rückverdichtung von in der Desorptionskolonne von Propan befreitem Absorptionsmittel (dem flüssigen Ablauf der Desorptionskolonne) mitverwendet). Das dabei erzeugte zweiphasige Gemisch wird am Kopf der Desorptionskolonne in selbige geführt.

In die Desorptionskolonne (ebenfalls eine Füllkörperkolonne) werden als Stripgas von unten im Gegenstrom zum zum vom Desorptionskolonnenkopf ablaufenden Absorbat auf einen Druck von 3,00 bar verdichtete 7,88 $Nm^3$/h Luft (diese hat sich dabei auf 175°C erwärmt) geführt.

Der flüssige Ablauf der Desorptionslolonne (115,94 kg/h) wird mittels einer Pumpe wieder auf 10,50 bar komprimiert und nach indirekter Kühlung mit Kühlwasser mit einer Temperatur von 30°C zur Propan-Absorption auf den Kopf der Propan-Waschkolonne (Absorptionskolonne) rückgeführt.

Am Kopf der Desorptionskolonne entweichen 10,97 $Nm^3$/h Gasgemisch mit einer Temperatur von 33°C und einem Druck von 1,98 bar sowie den folgenden Gehalten:

| Propan | 17,77 Vol.-%, |
|---|---|
| Propylen | 9,53 Vol.-%, |
| Ethan | 0,11 Vol.-%, |
| $O_2$ | 14,54 Vol.-%, |
| $N_2$ | 54,91 Vol.-%, |
| $H_2O$ | 1,59 Vol.-%, |
| CO | 0 Vol.-%, und |
| $CO_2$ | 0,55 Vol.-% |

Durch indirekten Wärmeaustausch mit Wasserdampf wird die Temperatur des Gasgemischs auf 140°C erhöht (der Arbeitsdruck verringert sich dabei auf 1,88 bar).

Mit diesem Gasgemisch wird als Reaktionsgas B-Ausgangsgemisch (Beschickungsgasgemisch) eine Reaktionszone B beschickt, die wie diejenige aus II. aufgebaut ist.

Die Propylenbelastung der Katalysatorbeschickung der ersten Oxidationsstufe wird zu 130 Nl/l gewählt. Die Salzschmelzen (53 Gew.-% $KNO_3$, 40 Gew.-% $NaNO_2$, 7 Gew.-% $NaNO_3$) weisen folgende Eintrittstemperaturen auf:

$$T_A = 323°C \quad T_B = 327°C$$
$$T_C = 262°C \quad T_D = 265°C$$

Das Reaktionsgas B verlässt den Zwischenkühler in einer Menge von 10,99 Nm$^3$/h mit einer Temperatur von 260°C, einem Druck von 1,67 bar und folgenden Gehalten:

| | |
|---|---|
| Acrolein | 7,98 Vol.-%, |
| Acrylsäure | 0,53 Vol.-%, |
| Propan | 17,74 Vol.-%, |
| Propylen | 0,56 Vol.-%, |
| Ethan | 0,1 Vol.-%, |
| $O_2$ | 3,92 Vol.-%, |
| $N_2$ | 54,82 Vol.-%, |
| $H_2O$ | 11,44 Vol.-%, |
| CO | 0,41 Vol.-%, und |
| $CO_2$ | 1,49 Vol.-%. |

Ihm werden 2,21 Nm$^3$/h in einem Radialverdichter auf 3 bar verdichtete Luft (diese erwärmt sich dabei auf 175°C) zudosiert (eingedrosselt), so dass die Eintrittstemperatur des Reaktionsgases in die zweite Oxidationsstufe 251 °C bei einem Eingangsdruck von 1,67 bar beträgt (Acroleinbelastung =110 Nl/l•h).

Das Produktgasgemisch der zweiten Oxidationsstufe (das Produktgas B) weist eine Temperatur von 270°C und einen Druck von 1,55 bar sowie die folgenden Gehalte auf:

| | |
|---|---|
| Acrolein | 0,03 Vol.-%, |
| Acrylsäure | 6,94 Vol.-%, |
| Essigsäure | 0,21 Vol.-%, |
| Propan | 15,25 Vol.-%, |
| Propylen | 0,48 Vol.-%, |
| Ethan | 0,09 Vol.-%, |
| $O_2$ | 2,95 Vol.-%, |
| $N_2$ | 60,31 Vol.-%, |
| $H_2O$ | 10,49 Vol.-%, |
| CO | 0,55 Vol.-%, und |
| $CO_2$ | 1,69 Vol.-%. |

Das Produktgas B (12,79 Nm$^3$/h) wird wie in der WO 2004/035514 beschrieben, in einer Bodenkolonne fraktionierend kondensiert.

Der Rückstandsverbrennung werden 13,8 g/h Schwersieder (Polyacrylsäuren (Michael-Addukte) etc.) zugeführt.

Vom zweiten Fangboden oberhalb der Zufuhr des Produktgases B in die Bodenkolonne werden 2,82 kg/h einer kondensierten rohen Acrylsäure entnommen, die eine Temperatur von 15°C und 96,99 Gew.-% Acrylsäure aufweist. Diese wird wie in der WO 2004/035514 beschrieben, nach Zugabe einer geringen Menge Wasser suspensionskristallisiert, das Suspensionskristallisat in hydraulischen Waschkolonnen von der Mutterlauge getrennt und die Mutterlauge wie in der WO 2004/035514 beschrieben, in die Kondensationskolonne rückgeführt. Die Reinheit des gewaschenen Suspensionskristallisats beträgt > 99,87 Gew.-% Acrylsäure und eignet sich unmittelbar zur Herstellung von Wasser "superabsorbierenden" Polymerisaten zur Verwendung im Hygienebereich.

Die vom dritten Fangboden oberhalb der Zufuhr des Produktgases B in die Kondensationskolonne entnommene, nicht in die Kondensationskolonne rückgeführte Menge an Sauerwasserkondensat beträgt 1,06 kg/h und weist eine Temperatur von 33°C auf. Sie wird ebenfalls der Rückstandsverbrennung zugeführt.

Am Kopf der Kondensationskolonne verlassen 10,52 Nm$^3$/h an Restgas die Kondensationskolonne mit einer Temperatur von 33°C und einem Druck von 1,20 bar und folgenden Gehalten:

| | |
|---|---|
| Propan | 18,36 Vol.-%, |
| Propylen | 0,49 Vol.-%, |
| $O_2$ | 3,58 Vol.-%, |
| $N_2$ | 73,32 Vol.-%, |
| $H_2O$ | 1,73 Vol.-%, |

(fortgesetzt)

| CO | 0 Vol.-%, und |
| CO$_2$ | 1,51 Vol.-%. |

Das Restgas wird in seiner Gesamtmenge auf 3 bar verdichtet (dabei erwärmt es sich auf 135°C) und wie beschrieben nach indirektem Wärmeaustausch mit aus der Reaktionszone A herausgeführtem Produktgas A als Restgaskreisgas in die Reaktionszone A rückgeführt (als Treibstrahl für die zur Dehydrierkreisgasführung eingesetzte Strahlpumpe).

**Patentansprüche**

1.  Verfahren zur Herstellung von Acrolein, oder Acrylsäure, oder deren Gemisch aus Propan, bei dem man

    A)

    - einer ersten Reaktionszone A unter Ausbildung eines Reaktionsgases A wenigstens zwei gasförmige, Propan enthaltende Zufuhrströme zuführt, von denen wenigstens einer Frischpropan enthält;
    - in der Reaktionszone A das Reaktionsgas A durch wenigstens ein Katalysatorbett führt, in welchem durch partielle heterogen katalysierte Dehydrierung des Propans molekularer Wasserstoff und Propylen gebildet werden;
    - der Reaktionszone A molekularen Sauerstoff zuführt, der in der Reaktionszone A wenigstens eine Teilmenge von in Reaktionsgas A enthaltenem molekularem Wasserstoff zu Wasserdampf oxidiert, und
    - der Reaktionszone A Produktgas A entnimmt, das Propylen, Propan und Wasserdampf enthält;

    B) gegebenenfalls in einer ersten Trennzone I in Produktgas A enthaltenen Wasserdampf teilweise oder vollständig durch indirekte und/oder direkte Kühlung von Produktgas A unter Verbleib eines Produktgases A* kondensativ abtrennt;

    C) in einer Reaktionszone B Produktgas A oder Produktgas A* unter Zufuhr von molekularem Sauerstoff zur Beschickung wenigstens eines Oxidationsreaktors mit einem Propan, Propylen und molekularen Sauerstoff enthaltenden Reaktionsgas B verwendet und das in selbigem enthaltene Propylen einer heterogen katalysierten partiellen Gasphasenoxidation zu einem Acrolein, oder Acrylsäure, oder deren Gemisch als Zielprodukt, sowie nicht umgesetztes Propan enthaltenden Produktgas B unterwirft;

    D) aus der Reaktionszone B Produktgas B herausführt und in einer zweiten Trennzone II darin enthaltenes Zielprodukt unter Verbleib eines Propan enthaltenden Restgases abtrennt;

    E) gegebenenfalls eine die Zusammensetzung des Restgases aufweisende Teilmenge an Restgas als einen Propan enthaltenden Zufuhrstrom in die Reaktionszone A rückführt;

    F) in einer Trennzone 111 in nicht in die Reaktionszone A rückgeführtem Restgas, aus dem gegebenenfalls zuvor darin gegebenenfalls enthaltener Wasserdampf kondensativ und/oder gegebenenfalls darin enthaltener molekularer Wasserstoff über eine Trennmembran teilweise oder vollständig abgetrennt werden, enthaltenes Propan unter Ausbildung eines Propan enthaltenden Absorbats aus dem Restgas durch Absorption in ein organisches Lösungsmittel aufnimmt; und

    G) in einer Trennzone IV das Propan aus dem Absorbat abtrennt und als Propan enthaltenden Zufuhrstrom in die Reaktionszone A rückführt;

    **dadurch gekennzeichnet,**
    **dass** in der Reaktionszone A wenigstens soviel molekularer Wasserstoff zu Wasserdampf oxidiert wird, dass die in der Reaktionszone A zu Wasserdampf oxidierte Wasserstoffmenge wenigstens 20 mol-% der in der Reaktionszone A gebildeten Menge an molekularem Wasserstoff beträgt.

2.  Verfahren nach Anspruch 1, **dadurch gekennzeichnet, dass** wenigstens ein Teil der durch das Oxidieren von molekularem Wasserstoff in der Reaktionszone A erzeugten Wärmeenergie dazu verwendet wird, um der Reaktionszone A zugeführte gasförmige Zufuhrströme durch indirekten Wärmeaustausch mit Reaktionsgas A, oder Produktgas A, oder Reaktionsgas A und Produktgas A als Wärmeträger zu erwärmen.

3.  Verfahren nach Anspruch 1 oder 2, **dadurch gekennzeichnet, dass** die in der Reaktionszone A zu Wasserdampf oxidierte Wasserstoffmenge wenigstens 30 mol-% der in der Reaktionszone A gebildeten Menge an molekularem Wasserstoff beträgt.

**4.** Verfahren nach Anspruch 1 oder 2 **dadurch gekennzeichnet, dass** die in der Reaktionszone A zu Wasserdampf oxidierte Wasserstoffmenge wenigstens 40 mol- % der in der Reaktionszone A gebildeten Menge an molekularem Wasserstoff beträgt.

**5.** Verfahren nach Anspruch 1 oder 2, **dadurch gekennzeichnet, dass** die in der Reaktionszone A zu Wasserdampf oxidierte Wasserstoffmenge 20 bis 90 mol-% der in der Reaktionszone A gebildeten Menge an molekularem Wasserstoff beträgt.

**6.** Verfahren nach Anspruch 1 oder 2, **dadurch gekennzeichnet, dass** die in der Reaktionszone A zu Wasserdampf oxidierte Wasserstoffmenge 30 bis 70 mol-% der in der Reaktionszone A gebildeten Menge an molekularem Wasserstoff beträgt.

**7.** Verfahren nach einem der Ansprüche 1 bis 6, **dadurch gekennzeichnet, dass** das Reaktionsgas B molekularen Wasserstoff enthält.

**8.** Verfahren nach Anspruch 1 oder 2, **dadurch gekennzeichnet, dass** das Reaktionsgas B keinen molekularen Wasserstoff enthält.

**9.** Verfahren nach einem der Ansprüche 1 bis 8, **dadurch gekennzeichnet, dass** das der Reaktionszone A entnommene Produktgas A als solches zur Beschickung des wenigstens einen Oxidationsreaktors in der Reaktionszone B verwendet wird.

**10.** Verfahren nach einem der Ansprüche 1 bis 8, **dadurch gekennzeichnet, dass** in der Trennzone I wenigstens 5 mol-% des im Produktgas A enthaltenen Wasserdampfes kondensativ abgetrennt werden.

**11.** Verfahren nach einem der Ansprüche 1 bis 8, **dadurch gekennzeichnet, dass** in der Trennzone I wenigstens 25 mol-% des im Produktgas A enthaltenen Wasserdampfes kondensativ abgetrennt werden.

**12.** Verfahren nach einem der Ansprüche 1 bis 8, **dadurch gekennzeichnet, dass** in der Trennzone I wenigstens 50 mol-% des im Produktgas A enthaltenen Wasserdampfes kondensativ abgetrennt werden.

**13.** Verfahren nach einem der Ansprüche 1 bis 8, **dadurch gekennzeichnet, dass** in der Trennzone I 5 bis 98 mol-% des im Produktgas A enthaltenen Wasserdampfes kondensativ abgetrennt werden.

**14.** Verfahren nach einem der Ansprüche 1 bis 13, **dadurch gekennzeichnet, dass** der Arbeitsdruck in der Reaktionszone A > 1 bis 5 bar beträgt.

**15.** Verfahren nach einem der Ansprüche 1 bis 14, **dadurch gekennzeichnet, dass** für die Arbeitsdrücke P in den verschiedenen Zonen des Verfahrens, jeweils bestimmt am Eingang in die jeweilige Zone, nachfolgende Beziehungen gelten:

$$P_{Reaktionszone\ A} > P_{Trennzone\ I} > P_{Reaktionszone\ B} > P_{Trennzone\ II} < P_{Trennzone\ III} > P_{Trennzone\ IV} > P_{Reaktionszone\ A}.$$

**16.** Verfahren nach einem der Ansprüche 1 bis 15, **dadurch gekennzeichnet, dass** man in der Reaktionszone A spätestens dann molekularen Wasserstoff mit molekularem Sauerstoff verbrennt, nachdem 90 mol-% des in der Reaktionszone A insgesamt gebildeten molekularen Wasserstoff gebildet worden sind.

**17.** Verfahren nach einem der Ansprüche 1 bis 15, **dadurch gekennzeichnet, dass** man in der Reaktionszone A spätestens dann molekularen Wasserstoff mit molekularem Sauerstoff verbrennt, nachdem 75 mol-% des in der Reaktionszone A insgesamt gebildeten molekularen Wasserstoff gebildet worden sind.

**18.** Verfahren nach einem der Ansprüche 1 bis 15, **dadurch gekennzeichnet, dass** man in der Reaktionszone A spätestens dann molekularen Wasserstoff mit molekularem Sauerstoff verbrennt, nachdem 65 mol-% des in der Reaktionszone A insgesamt gebildeten molekularen Wasserstoff gebildet worden sind.

**19.** Verfahren nach einem der Ansprüche 1 bis 18, **dadurch gekennzeichnet, dass** in einer Trennzone I in Produktgas A enthaltener Wasserdampf wenigstens teilweise abgetrennt wird und diese Abtrennung durch indirekte und direkte Kühlung von Produktgas A bewirkt wird.

**20.** Verfahren nach einem der Ansprüche 1 bis 19, **dadurch gekennzeichnet, dass** man eine die Zusammensetzung des Restgases aufweisende Teilmenge an Restgas als einen Propan enthaltenden Zufuhrstrom in die Reaktionszone A rückführt, und das Restgas molekularen Sauerstoff enthält.

**21.** Verfahren nach einem der Ansprüche 1 bis 20, **dadurch gekennzeichnet, dass** der der Reaktionszone A zugeführte molekulare Sauerstoff auch als Bestandteil von Luft zugeführt wird.

**22.** Verfahren nach einem der Ansprüche 1 bis 20, **dadurch gekennzeichnet, dass** der der Reaktionszone A zugeführte molekulare Sauerstoff auch als Bestandteil eines Gases zugeführt wird, das nicht mehr als 50 Vol.-% an von molekularem Sauerstoff verschiedenen Bestandteile enthält.

**23.** Verfahren nach einem der Ansprüche 1 bis 22, **dadurch gekennzeichnet, dass** man die Abtrennung des Propans aus dem Absorbat in der Trennzone IV durch Strippen mit einem Wasserdampf enthaltenden Gas durchführt und das mit Propan beladene Stripgas als Propan enthaltenden Zufuhrstrom in die Reaktionszone A rückführt.

**24.** Verfahren nach einem der Ansprüche 1 bis 23, **dadurch gekennzeichnet, dass** man die Reaktionszone A adiabat gestaltet.

**25.** Verfahren nach einem der Ansprüch1 bis 24, **dadurch gekennzeichnet, dass**, bezogen auf einen einmaligen Durchgang durch die Reaktionszone A, 10 bis 40 mol-% des der Reaktionszone A insgesamt zugeführten Propan in der Reaktionszone A dehydrierend umgesetzt werden.

**26.** Verfahren nach einem der Ansprüche 1 bis 25, **dadurch gekennzeichnet, dass** die Reaktionszone A wenigstens einen Hordenreaktor umfasst.

**27.** Verfahren nach einem der Ansprüche 1 bis 26, **dadurch gekennzeichnet, dass** das sich in der Reaktionszone A ausbildende Reaktionsgas A folgende Gehalte aufweist:

| | |
|---|---|
| 50 bis 80 Vol.-% | Propan, |
| 0,1 bis 20 Vol-% | Propylen, |
| 0 bis 10 Vol.-% | $H_2$, |
| 0 bis 20 Vol.-% | $N_2$, und |
| 5 bis 15 Vol.-% | $H_2O$. |

**28.** Verfahren nach einem der Ansprüche 1 bis 26, **dadurch gekennzeichnet, dass** das sich in der Reaktionszone A ausbildende Reaktionsgas A folgende Gehalte aufweist:

| | |
|---|---|
| 55 bis 80 Vol.-% | Propan, |
| 0,1 bis 20 Vol.-% | Propylen, |
| 2 bis 10 Vol.-% | $H_2$, |
| 1 bis 5 Vol.-% | O, |
| 0 bis 20 Vol.-% | $N_2$, und |
| 5 bis 15 Vol.-% | $H_2O$. |

**29.** Verfahren nach einem der Ansprüche 1 bis 28, **dadurch gekennzeichnet, dass** das der Reaktionszone A entnommene Produktgas A folgende Gehalte aufweist:

| | |
|---|---|
| 30 bis 50 Vol.-% | Propan, |
| 15 bis 30 Vol.-% | Propylen, |

(fortgesetzt)

| | |
|---|---|
| 0 bis 10 Vol.-% | $H_2$, |
| 10 bis 25 Vol.-% | $H_2O$, |
| 0 bis 1 Vol.-% | O, und |
| 0 bis 35 Vol.-% | $N_2$. |

**30.** Verfahren nach einem der Ansprüche 1 bis 29, **dadurch gekennzeichnet, dass** das Produktgas A durch indirekten Wärmeaustausch mit dem in der Trennzone IV abgetrennten und in die Reaktionszone A rückgeführten Propan enthaltenden Zufuhrstrom abgekühlt wird.

**31.** Verfahren nach einem der Ansprüche 1 bis 30, **dadurch gekennzeichnet, dass** die heterogen katalysierte partielle Gasphasenoxidation eine zweistufige Partialoxidation von Propylen zu Acrylsäure ist.

**32.** Verfahren nach Anspruch 31, **dadurch gekennzeichnet, dass** das der zweiten Oxidationsstufe zugeführte Reaktionsgas folgende Gehalte aufweist:

| | |
|---|---|
| 3 bis 25 Vol.-% | Acrolein, |
| 5 bis 65 Vol.-% | molekularer Sauerstoff, |
| 6 bis 70 Vol.-% | Propan, |
| 0 bis 20 Vol.-% | $H_2$, |
| 8 bis 65 Vol.-% | $H_2O$, und |
| 0 bis 70 Vol.-% | $N_2$. |

**33.** Verfahren nach einem der Ansprüche 1 bis 32, **dadurch gekennzeichnet, dass** das Restgas folgende Gehalte aufweist:

| | |
|---|---|
| 1 bis 20 Vol.-% | $H_2O$ |
| 0 bis 80 Vol.-% | $N_2$, |
| 10 bis 90 Vol.-% | Propan, |
| 0 bis 20 Vol.-% | $H_2$, |
| 0 bis 10 Vol.-% | $O_2$, |
| 1 bis 20 Vol.-% | $CO_2$, und |
| $\geq$ 0 bis 5 Vol.-% | CO. |

**34.** Verfahren nach einem der Ansprüche 1 bis 33, **dadurch gekennzeichnet, dass** der in der Trennzone IV abgetrennte und in die Reaktionszone A rückgeführte Propan enthaltende Zufuhrstrom folgende Gehalte aufweist:

| | |
|---|---|
| 80 bis 99,99 mol-% | Propan, |
| 0 bis 5 mol-% | Propylen, und |
| 0 bis 20 mol-% | $H_2O$ |

**35.** Verfahren nach einem der Ansprüche 1 bis 34, **dadurch gekennzeichnet, dass** eine die Zusammensetzung des Restgases aufweisende Teilmenge an Restgas als ein Propan enthaltender Zufuhrstrom in die Reaktionszone A rückgeführt wird, und diese Teilmenge an Restgas, bezogen auf die Gesamtmenge an Restgas, bis zu 10 Gew.-% beträgt.

**36.** Verfahren nach einem der Ansprüche 1 bis 35, **dadurch gekennzeichnet, dass** der der Reaktionszone B zugeführte molekulare Sauerstoff auch als Bestandteil eines Gases zugeführt wird, das nicht mehr als 50 Vol.-% an von molekularem Sauerstoff verschiedenen Bestandteilen enthält.

**37.** Verfahren nach einem der Ansprüche 1 bis 35, **dadurch gekennzeichnet, dass** der der Reaktionszone B zugeführte molekulare Sauerstoff auch als Bestandteil von Luft zugeführt wird.

**Claims**

1. A process for preparing acrolein or acrylic acid or a mixture thereof from propane, in which

    A)

      - at least two gaseous propane-comprising feed streams, of which at least one comprises fresh propane, are fed to a first reaction zone A to form a reaction gas A;
      - in reaction zone A, reaction gas A is conducted through at least one catalyst bed in which partial heterogeneously catalyzed dehydrogenation of the propane forms molecular hydrogen and propylene;
      - molecular oxygen is fed to reaction zone A and, in reaction zone A, oxidizes at least a portion of molecular hydrogen present in reaction gas A to steam, and
      - product gas A which comprises propylene, propane and steam is withdrawn from reaction zone A;

    B) if appropriate, in a first separation zone I, steam present in product gas A is removed by condensation partly or fully by indirect and/or direct cooling of product gas A to leave a product gas A*;

    C) in a reaction zone B, product gas A or product gas A* is used, with feeding of molecular oxygen, to charge at least one oxidation reactor with a reaction gas B comprising propane, propylene and molecular oxygen, and the propylene present therein is subjected to a heterogeneously catalyzed partial gas phase oxidation to give acrolein or acrylic acid or a mixture thereof as the target product, and also product gas B comprising unconverted propane;

    D) product gas B is conducted out of reaction zone B and, in a second separation zone II, target product present therein is removed to leave a propane-comprising residual gas;

    E) if appropriate, a portion of residual gas having the composition of the residual gas is recycled as a propane-comprising feed stream into reaction zone A;

    F) in a separation zone III, propane present in residual gas which has not been recycled into reaction zone A and from which, if appropriate, any steam present therein has been removed beforehand partly or fully by condensation and/or any molecular hydrogen present therein has been removed beforehand partly or fully by means of a separating membrane is absorbed into an organic solvent by absorption from the residual gas to form a propane-comprising absorbate; and

    G) in a separation zone IV, the propane is removed from the absorbate and recycled into reaction zone A as a propane-comprising feed stream;

    which comprises
    oxidizing at least sufficient molecular hydrogen to steam in reaction zone A that the amount of hydrogen oxidized to steam in reaction zone A is at least 20 mol% of the amount of molecular hydrogen formed in reaction zone A.

2. The process according to claim 1, wherein at least some of the heat energy generated by the oxidation of molecular hydrogen in reaction zone A is used to heat gaseous feed streams fed to reaction zone A by indirect heat exchange with reaction gas A or product gas A or reaction gas A and product gas A as heat carriers.

3. The process according to claim 1 or 2, wherein the amount of hydrogen oxidized to steam in reaction zone A is at least 30 mol% of the amount of molecular hydrogen formed in reaction zone A.

4. The process according to claim 1 or 2, wherein the amount of hydrogen oxidized to steam in reaction zone A is at least 40 mol% of the amount of molecular hydrogen formed in reaction zone A.

5. The process according to claim 1 or 2, wherein the amount of hydrogen oxidized to steam in reaction zone A is from 20 to 90 mol% of the amount of molecular hydrogen formed in reaction zone A.

6. The process according to claim 1 or 2, wherein the amount of hydrogen oxidized to steam in reaction zone A is from 30 to 70 mol% of the amount of molecular hydrogen formed in reaction zone A.

7. The process according to any of claims 1 to 6, wherein reaction gas B comprises molecular hydrogen.

8. The process according to claim 1 or 2, wherein reaction gas B does not comprise any molecular hydrogen.

9. The process according to any of claims 1 to 8, wherein the product gas A withdrawn from reaction zone A is used as such to charge the at least one oxidation reactor in reaction zone B.

10. The process according to any of claims 1 to 8, wherein at least 5 mol% of the steam present in product gas B is removed by condensation in separation zone I.

11. The process according to any of claims 1 to 8, wherein at least 25 mol% of the steam present in product gas B is removed by condensation in separation zone I.

12. The process according to any of claims 1 to 8, wherein at least 50 mol% of the steam present in product gas B is removed by condensation in separation zone I.

13. The process according to any of claims 1 to 8, wherein from 5 to 98 mol% of the steam present in product gas B is removed by condensation in separation zone I.

14. The process according to any of claims 1 to 13, wherein the working pressure in reaction zone A is from > 1 to 5 bar.

15. The process according to any of claims 1 to 14, wherein the following relationships apply to the working pressures P in the different zones of the process, determined in each case at the entrance into the particular zone:

$$P_{reaction\ zone\ A} > P_{separation\ zone\ I} > P_{reaction\ zone\ B} > P_{separation\ zone\ II} < P_{separation\ zone\ III} > P_{separation\ zone\ IV} > P_{reaction\ zone\ A}.$$

16. The process according to any of claims 1 to 15, wherein molecular hydrogen is combusted with molecular oxygen in reaction zone A not later than when 90 mol% of the total amount of molecular hydrogen formed in reaction zone A has been formed.

17. The process according to any of claims 1 to 15, wherein molecular hydrogen is combusted with molecular oxygen in reaction zone A not later than when 75 mol% of the total amount of molecular hydrogen formed in reaction zone A has been formed.

18. The process according to any of claims 1 to 15, wherein molecular hydrogen is combusted with molecular oxygen in reaction zone A not later than when 65 mol% of the total amount of molecular hydrogen formed in reaction zone A has been formed.

19. The process according to any of claims 1 to 18, wherein steam present in product gas A in a separation zone I is removed at least partly and this removal is brought about by indirect and direct cooling of product gas A.

20. The process according to any of claims 1 to 19, wherein a portion of residual gas having the composition of the residual gas is recycled as a propane-comprising feed stream into reaction zone A, and the residual gas comprises molecular oxygen.

21. The process according to any of claims 1 to 20, wherein the molecular oxygen fed to reaction zone A is also fed as a constituent of air.

22. The process according to any of claims 1 to 20, wherein the molecular oxygen fed to reaction zone A is also fed as a constituent of a gas which comprises not more than 50% by volume of constituents other than molecular oxygen.

23. The process according to any of claims 1 to 22, wherein the removal of the propane from the absorbate in separation zone IV is performed by stripping with a steam-comprising gas and the propane-laden stripping gas is recycled as a propane-comprising feed stream into reaction zone A.

24. The process according to any of claims 1 to 23, wherein reaction zone A is configured adiabatically.

**25.** The process according to any of claims 1 to 24, wherein, based on a single pass through reaction zone A, from 10 to 40 mol% of the total amount of propane fed to reaction zone A is converted with dehydrogenation in reaction zone A.

**26.** The process according to any of claims 1 to 25, wherein reaction zone A comprises at least one tray reactor.

**27.** The process according to any of claims 1 to 26, wherein the reaction gas A which forms in reaction zone A has the following contents:

| | |
|---|---|
| from 50 to 80% by volume of | propane, |
| from 0.1 to 20% by volume of | propylene, |
| from 0 to 10% by volume of | $H_2$, |
| from 0 to 20% by volume of | $N_2$, and |
| from 5 to 15% by volume of | $H_2O$. |

**28.** The process according to any of claims 1 to 26, wherein the reaction gas A which forms in reaction zone A has the following contents:

| | |
|---|---|
| from 55 to 80% by volume of | propane, |
| from 0.1 to 20% by volume of | propylene, |
| from 2 to 10% by volume of | $H_2$, |
| from 1 to 5% by volume of | O, |
| from 0 to 20% by volume of | $N_2$, and |
| from 5 to 15% by volume of | $H_2O$ |

**29.** The process according to any of claims 1 to 28, wherein the product gas A withdrawn from reaction zone A has the following contents:

| | |
|---|---|
| from 30 to 50% by volume of | propane, |
| from 15 to 30% by volume of | propylene, |
| from 0 to 10% by volume of | $H_2$, |
| from 10 to 25% by volume of | $H_2O$ |
| from 0 to 1% by volume of | $O_2$, and |
| from 0 to 35% by volume of | $N_2$. |

**30.** The process according to any of claims 1 to 29, wherein product gas A is cooled by indirect heat exchange with the propane-comprising feed stream which has been removed in separation zone IV and recycled into reaction zone A.

**31.** The process according to any of claims 1 to 30, wherein the heterogeneously catalyzed partial gas phase oxidation is a two-stage partial oxidation of propylene to acrylic acid.

**32.** The process according to claim 31, wherein the reaction gas fed to the second oxidation stage has the following contents:

| | |
|---|---|
| from 3 to 25% by volume of | acrolein, |
| from 5 to 65% by volume of | molecular oxygen, |
| from 6 to 70% by volume of | propane, |
| from 0 to 20% by volume of | $H_2$, |
| from 8 to 65% by volume of | $H_2O$, and |
| from 0 to 70% by volume of | $N_2$. |

**33.** The process according to any of claims 1 to 32, wherein the residual gas has the following contents:

| | |
|---|---|
| from 1 to 20% by volume of | $H_2O$ |
| from 0 to 80% by volume of | $N_2$, |
| from 10 to 90% by volume of | propane, |
| from 0 to 20% by volume of | $H_2$, |
| from 0 to 10% by volume of | $O_2$, |
| from 1 to 20% by volume of | $CO_2$, and |
| from $\geq$ 0 to 5% by volume of | CO. |

**34.** The process according to any of claims 1 to 33, wherein the propane-comprising feed stream which has been removed in separation zone IV and recycled into reaction zone A has the following contents:

| | |
|---|---|
| from 80 to 99.99 mol% of | propane, |
| from 0 to 5 mol% of | propylene, and |
| from 0 to 20 mol% of | $H_2O$. |

**35.** The process according to any of claims 1 to 34, wherein a portion of residual gas having the composition of the residual gas is recycled as a propane-comprising feed stream into reaction zone A, and this portion of residual gas, based on the total amount of residual gas, is up to 10% by weight.

**36.** The process according to any of claims 1 to 35, wherein the molecular oxygen fed to reaction zone B is also fed as a constituent of a gas which does not comprise more than 50% by volume of constituents other than molecular oxygen.

**37.** The process according to any of claims 1 to 35, wherein the molecular oxygen fed to reaction zone B is also fed as a constituent of air.

**Revendications**

**1.** Procédé de fabrication d'acroléine ou d'acide acrylique ou leur mélange à partir de propane, selon lequel

A)

- au moins deux courants d'alimentation gazeux contenant du propane, parmi lesquels au moins un contient du propane frais, sont introduits dans une première zone de réaction A avec formation d'un gaz de réaction A ;
- dans la zone de réaction A, le gaz de réaction A traverse au moins un lit catalytique, dans lequel de l'hydrogène moléculaire et du propylène sont formés par déshydrogénation partielle sous catalyse hétérogène du propane ;
- de l'oxygène moléculaire est introduit dans la zone de réaction A, qui oxyde dans la zone de réaction A au moins une partie de l'hydrogène moléculaire contenu dans le gaz de réaction A en vapeur d'eau, et
- un gaz de produits A est soutiré de la zone de réaction A, qui contient du propylène, du propane et de la vapeur d'eau ;

B) dans une première zone de séparation I, la vapeur d'eau contenue dans le gaz de produits A est éventuellement séparée par condensation en partie ou en totalité par refroidissement indirect et/ou direct du gaz de produits A, en laissant un gaz de produits A* ;
C) dans une zone de réaction B, le gaz de produits A ou le gaz de produits A* est utilisé avec introduction d'oxygène moléculaire pour l'alimentation d'au moins un réacteur d'oxydation avec un gaz de réaction B contenant du propane, du propylène et de l'oxygène moléculaire, et le propylène contenu dans celui-ci est soumis à une oxydation partielle en phase gazeuse sous catalyse hétérogène en un gaz de produits B contenant de l'acroléine ou de l'acide acrylique ou leur mélange en tant que produit cible, ainsi que du propane non réagi ;
D) le gaz de produits B est déchargé de la zone de réaction B et le produit cible qu'il contient est séparé dans une deuxième zone de séparation II en laissant un gaz résiduel contenant du propane ;

E) une partie du gaz résiduel présentant la composition du gaz résiduel est éventuellement recyclée en tant que courant d'alimentation contenant du propane dans la zone de réaction A ;

F) dans une zone de séparation III, le propane contenu dans le gaz résiduel non recyclé dans la zone de réaction A, duquel sont éventuellement séparés au préalable en partie ou en totalité la vapeur d'eau éventuellement contenue dans celui-ci par condensation et/ou l'hydrogène moléculaire éventuellement contenu dans celui-ci par une membrane de séparation, est absorbé à partir du gaz résiduel par absorption dans un solvant organique pour former un absorbat contenant du propane ; et

G) dans une zone de séparation IV, le propane est séparé de l'absorbat et recyclé en tant que courant d'alimentation contenant du propane dans la zone de réaction A ;

**caractérisé en ce que**

dans la zone de réaction A, au moins suffisamment d'hydrogène moléculaire est oxydé en vapeur d'eau pour que la quantité d'hydrogène oxydée en vapeur d'eau dans la zone de réaction A soit d'au moins 20 % en moles de la quantité d'hydrogène moléculaire formée dans la zone de réaction A.

2. Procédé selon la revendication 1, **caractérisé en ce qu'**au moins une partie de l'énergie thermique générée par l'oxydation d'hydrogène moléculaire dans la zone de réaction A est utilisée pour chauffer les courants d'alimentation gazeux introduits dans la zone de réaction A par échange de chaleur indirect avec le gaz de réaction A ou le gaz de produits A ou le gaz de réaction A et le gaz de produits A en tant que caloporteur.

3. Procédé selon la revendication 1 ou 2, **caractérisé en ce que** la quantité d'hydrogène oxydée en vapeur d'eau dans la zone de réaction A est d'au moins 30 % en moles de la quantité d'hydrogène moléculaire formée dans la zone de réaction A.

4. Procédé selon la revendication 1 ou 2, **caractérisé en ce que** la quantité d'hydrogène oxydée en vapeur d'eau dans la zone de réaction A est d'au moins 40 % en moles de la quantité d'hydrogène moléculaire formée dans la zone de réaction A.

5. Procédé selon la revendication 1 ou 2, **caractérisé en ce que** la quantité d'hydrogène oxydée en vapeur d'eau dans la zone de réaction A est de 20 à 90 % en moles de la quantité d'hydrogène moléculaire formée dans la zone de réaction A.

6. Procédé selon la revendication 1 ou 2, **caractérisé en ce que** la quantité d'hydrogène oxydée en vapeur d'eau dans la zone de réaction A est de 30 à 70 % en moles de la quantité d'hydrogène moléculaire formée dans la zone de réaction A.

7. Procédé selon l'une quelconque des revendications 1 à 6, **caractérisé en ce que** le gaz de réaction B contient de l'hydrogène moléculaire.

8. Procédé selon la revendication 1 ou 2, **caractérisé en ce que** le gaz de réaction B ne contient pas d'hydrogène moléculaire.

9. Procédé selon l'une quelconque des revendications 1 à 8, **caractérisé en ce que** le gaz de produits A soutiré de la zone de réaction A est utilisé en tant que tel pour l'alimentation dudit au moins un réacteur d'oxydation dans la zone de réaction B.

10. Procédé selon l'une quelconque des revendications 1 à 8, **caractérisé en ce qu'**au moins 5 % en moles de la vapeur d'eau contenue dans le gaz de produits A est séparée par condensation dans la zone de séparation I.

11. Procédé selon l'une quelconque des revendications 1 à 8, **caractérisé en ce qu'**au moins 25 % en moles de la vapeur d'eau contenue dans le gaz de produits A est séparée par condensation dans la zone de séparation I.

12. Procédé selon l'une quelconque des revendications 1 à 8, **caractérisé en ce qu'**au moins 50 % en moles de la vapeur d'eau contenue dans le gaz de produits A est séparée par condensation dans la zone de séparation I.

13. Procédé selon l'une quelconque des revendications 1 à 8, **caractérisé en ce que** 5 à 98 % en moles de la vapeur d'eau contenue dans le gaz de produits A est séparée par condensation dans la zone de séparation I.

**14.** Procédé selon l'une quelconque des revendications 1 à 13, **caractérisé en ce que** la pression de travail dans la zone de réaction A est de > 1 à 5 bar.

**15.** Procédé selon l'une quelconque des revendications 1 à 14, **caractérisé en ce que**, pour les pressions de travail P dans les différentes zones du procédé, à chaque fois déterminées à l'entrée de la zone en question, les relations suivantes s'appliquent :

$$P_{zone\ de\ réaction\ A} > P_{zone\ de\ séparation\ I} > P_{zone\ de\ réaction\ B} > P_{zone\ de\ séparation\ II} < P_{zone\ de\ séparation\ III} > P_{zone\ de\ séparation\ IV} > P_{zone\ de\ réaction\ A}.$$

**16.** Procédé selon l'une quelconque des revendications 1 à 15, **caractérisé en ce que**, dans la zone de réaction A, l'hydrogène moléculaire est brûlé avec de l'oxygène moléculaire au plus tard après que 90 % en moles de l'hydrogène moléculaire formé au total dans la zone de réaction A ait été formé.

**17.** Procédé selon l'une quelconque des revendications 1 à 15, **caractérisé en ce que**, dans la zone de réaction A, l'hydrogène moléculaire est brûlé avec de l'oxygène moléculaire au plus tard après que 75 % en moles de l'hydrogène moléculaire formé au total dans la zone de réaction A ait été formé.

**18.** Procédé selon l'une quelconque des revendications 1 à 15, **caractérisé en ce que**, dans la zone de réaction A, l'hydrogène moléculaire est brûlé avec de l'oxygène moléculaire au plus tard après que 65 % en moles de l'hydrogène moléculaire formé au total dans la zone de réaction A ait été formé.

**19.** Procédé selon l'une quelconque des revendications 1 à 18, **caractérisé en ce que** la vapeur d'eau contenue dans le gaz de produits A est séparée au moins en partie dans une zone de séparation I et cette séparation est réalisée par refroidissement indirect et direct du gaz de produits A.

**20.** Procédé selon l'une quelconque des revendications 1 à 19, **caractérisé en ce qu'**une partie du gaz résiduel présentant la composition du gaz résiduel est recyclée dans la zone de réaction A en tant que courant d'alimentation contenant du propane, et le gaz résiduel contient de l'oxygène moléculaire.

**21.** Procédé selon l'une quelconque des revendications 1 à 20, **caractérisé en ce que** l'oxygène moléculaire introduit dans la zone de réaction A est également introduit en tant que constituant de l'air.

**22.** Procédé selon l'une quelconque des revendications 1 à 20, **caractérisé en ce que** l'oxygène moléculaire introduit dans la zone de réaction A est également introduit en tant que constituant d'un gaz qui ne contient pas plus de 50 % en volume de constituants différents de l'oxygène moléculaire.

**23.** Procédé selon l'une quelconque des revendications 1 à 22, **caractérisé en ce que** la séparation du propane à partir de l'absorbat dans la zone de séparation IV est réalisée par extraction avec un gaz contenant de la vapeur d'eau et le gaz d'extraction chargé avec du propane est recyclé dans la zone de réaction A en tant que courant d'alimentation contenant du propane.

**24.** Procédé selon l'une quelconque des revendications 1 à 23, **caractérisé en ce que** la zone de réaction A est configurée sous forme adiabatique.

**25.** Procédé selon l'une quelconque des revendications 1 à 24, **caractérisé en ce que**, par rapport à un passage unique dans la zone de réaction A, 10 à 40 % en moles du propane introduit au total dans la zone de réaction A est transformé par déshydrogénation dans la zone de réaction A.

**26.** Procédé selon l'une quelconque des revendications 1 à 25, **caractérisé en ce que** la zone de réaction A comprend au moins un réacteur à étages.

**27.** Procédé selon l'une quelconque des revendications 1 à 26, **caractérisé en ce que** le gaz de réaction A formé dans la zone de réaction A présente les teneurs suivantes :

50 à 80 % en volume de propane,
0,1 à 20 % en volume de propylène,
0 à 10 % en volume d'$H_2$,
0 à 20 % en volume de $N_2$ et
5 à 15 % en volume d'$H_2O$.

28. Procédé selon l'une quelconque des revendications 1 à 26, **caractérisé en ce que** le gaz de réaction A formé dans la zone de réaction A présente les teneurs suivantes :

55 à 80 % en volume de propane,
0,1 à 20 % en volume de propylène,
2 à 10 % en volume d'$H_2$,
1 à 5 % en volume d'$O_2$,
0 à 20 % en volume de $N_2$ et
5 à 15 % en volume d'$H_2O$.

29. Procédé selon l'une quelconque des revendications 1 à 28, **caractérisé en ce que** le gaz de produits A soutiré de la zone de réaction A présente les teneurs suivantes :

30 à 50 % en volume de propane,
15 à 30 % en volume de propylène,
0 à 10 % en volume d'$H_2$,
10 à 25 % en volume d'$H_2O$,
0 à 1 % en volume d'$O_2$ et
0 à 35 % en volume de $N_2$.

30. Procédé selon l'une quelconque des revendications 1 à 29, **caractérisé en ce que** le gaz de produits A est refroidi par échange de chaleur indirect avec le courant d'alimentation contenant du propane séparé dans la zone de séparation IV et recyclé dans la zone de réaction A.

31. Procédé selon l'une quelconque des revendications 1 à 30, **caractérisé en ce que** l'oxydation partielle en phase gazeuse sous catalyse hétérogène est une oxydation partielle à deux étapes de propylène en acide acrylique.

32. Procédé selon la revendication 31, **caractérisé en ce que** le gaz de réaction introduit dans la deuxième étape d'oxydation présente les teneurs suivantes :

3 à 25 % en volume d'acroléine,
5 à 65 % en volume d'oxygène moléculaire,
6 à 70 % en volume de propane,
0 à 20 % en volume d'$H_2$,
8 à 65 % en volume d'$H_2O$ et
0 à 70 % en volume de $N_2$.

33. Procédé selon l'une quelconque des revendications 1 à 32, **caractérisé en ce que** le gaz résiduel présente les teneurs suivantes :

1 à 20 % en volume d'$H_2O$,
0 à 80 % en volume de $N_2$,
10 à 90 % en volume de propane,
0 à 20 % en volume d'$H_2$,
0 à 10 % en volume d'$O_2$,
1 à 20 % en volume de $CO_2$ et
$\geq$ 0 à 5 % en volume de CO.

34. Procédé selon l'une quelconque des revendications 1 à 33, **caractérisé en ce que** le courant d'alimentation contenant du propane séparé dans la zone de séparation IV et recyclé dans la zone de réaction A présente les teneurs suivantes :

80 à 99,99 % en moles de propane,
0 à 5 % en moles de propylène et
0 à 20 % en moles d'$H_2O$.

**35.** Procédé selon l'une quelconque des revendications 1 à 34, **caractérisé en ce qu'**une partie du gaz résiduel présentant la composition du gaz résiduel est recyclée dans la zone de réaction A en tant que courant d'alimentation contenant du propane, et cette partie du gaz résiduel est de jusqu'à 10 % en poids, par rapport à la quantité totale de gaz résiduel.

**36.** Procédé selon l'une quelconque des revendications 1 à 35, **caractérisé en ce que** l'oxygène moléculaire introduit dans la zone de réaction B est également introduit en tant que constituant d'un gaz qui ne contient pas plus de 50 % en volume de constituants différents de l'oxygène moléculaire.

**37.** Procédé selon l'une quelconque des revendications 1 à 35, **caractérisé en ce que** l'oxygène moléculaire introduit dans la zone de réaction B est également introduit en tant que constituant de l'air.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6166263 A
- US 6187963 A
- EP 1617931 A
- WO 0409041 A
- DE 3313573 A
- EP 117146 A
- US 3161670 A
- DE 102004032129 A
- EP 731077 A
- DE 102005049699 A
- DE 102005052923 A
- WO 0196271 A
- WO 03011804 A
- WO 03076370 A
- WO 0196270 A
- DE 102005009891 A
- DE 102005013039 A
- DE 102005022798 A
- DE 102005009885 A
- DE 102005010111 A
- DE 10245585 A
- DE 10316039 A
- DE 102005056377
- DE 102005049699
- DE 102006015235
- DE 102005061626
- DE 102005013039
- DE 10246119 A
- WO 04007405 A
- US 316260 A
- DE 102006017623
- DE 102005057197
- DE 10211275 A
- DE 10131297 A
- WO 9946039 A
- US 4788371 A
- EP 705136 A
- WO 9929420 A
- US 4220091 A
- US 5430220 A
- US 5877369 A
- DE 19937196 A
- DE 19937105 A
- US 3670044 A
- US 6566573 A
- WO 9429021 A
- DE 19937107 A

- US 4886928 A
- US 5430209 A
- US 5530171 A
- US 5527979 A
- US 5563314 A
- DE 10200501339 A
- DE 10353014 A
- EP 700714 A
- EP 700893 A
- WO 04085369 A
- WO 0485363 A
- DE 10313212 A
- EP 1159248 A
- EP 1159246 A
- EP 1159247 A
- DE 19948248 A
- DE 10101695 A
- WO 04085368 A
- DE 102004021764
- WO 04085362 A
- WO 04085370 A
- WO 04085365 A
- WO 04085367 A
- EP 990636 A
- EP 1007007 A
- EP 1106598 A
- EP 253409 A
- DE 4431957 A
- DE 102004025445 A
- DE 4431949 A
- DE 10325488 A
- DE 10325487 A
- DE 10353954 A
- DE 10344149 A
- DE 10351269 A
- DE 10350812 A
- DE 10350822 A
- DE 19955176 A
- DE 19948523 A
- DE 19955168 A
- DE 10046957 A
- DE 10063162 A
- DE 3338380 C
- DE 19902562 A
- EP 15565 A
- DE 2380765 C
- EP 807465 A
- EP 279374 A
- DE 3300044 A

- EP 575897 A
- US 4438217 A
- DE 19855913 A
- WO 9824746 A
- DE 19746210 A
- JP 3294239 A
- EP 293224 A
- DE 4023239 A
- DE 102005037678 A
- DE 2909671 A
- EP 293859 A
- EP 714700 A
- DE 10046928 A
- DE 19815281 A
- DE 4335973 A
- EP 668104 A
- DE 19736105 A
- DE 19740493 A
- DE 19528646 A
- DE 19910506 A
- EP 1159244 A
- WO 04085363 A
- DE 19910508 A
- DE 10121592 A
- EP 911313 A
- EP 979813 A
- DE 2830765 A
- DE 19837517 A
- DE 19837519 A
- DE 10213998 A
- DE 2263496 A
- US 3433840 A
- EP 1388533 A
- EP 1388532 A
- DE 10235847 A
- EP 792867 A
- WO 9801415 A
- EP 1015411 A
- EP 1015410 A
- WO 9950219 A
- WO 0053560 A
- WO 0209839 A
- WO 03041833 A
- DE 10223058 A
- DE 10243625 A
- DE 10336386 A
- EP 854129 A
- US 4317926 A
- DE 19837520 A
- DE 19606877 A
- DE 190501325 A
- DE 10247240 A
- DE 19740253 A
- EP 695736 A
- EP 982287 A
- EP 1041062 A
- DE 4308087 A
- DE 4335172 A
- DE 4436243 A
- DE 19924532 A
- DE 10332758 A
- DE 19924533 A
- EP 982289 A
- DE 10115277 A
- DE 19740252 A
- DE 19627847 A
- EP 920408 A
- EP 1068174 A
- EP 1066239 A
- EP 1066240 A
- WO 0053561 A
- DE 10053086 A
- EP 982288 A
- WO 04063138 A
- WO 0435514 A
- WO 0177056 A
- WO 03041832 A
- WO 02055469 A
- WO 03078378 A
- DE 10219879 A
- WO 2004035514 A

**In der Beschreibung aufgeführte Nicht-Patentliteratur**

- **WALTER J. MOORE.** Physikalische Chemie. WDEG Verlag, 1973, 171